(19) Europäisches Patentamt
European Patent Office
Office européen des brevets

(11) Veröffentlichungsnummer: 0 035 244
B1

(12) # EUROPÄISCHE PATENTSCHRIFT

(45) Veröffentlichungstag der Patentschrift: 08.05.85

(21) Anmeldenummer: 81101406.7

(22) Anmeldetag: 26.02.81

(51) Int. Cl.⁴: **C 07 D 471/04,** C 07 D 209/80, A 61 K 31/47, A 61 K 31/475 // (C07D471/04, 221:00, 209:00)

(54) **Cycloalka(4,5)pyrrolo(2,3-g)isochinoline, Verfahren und Zwischenprodukte für ihre Herstellung, und sie enthaltende Arzneimittel.**

(30) Priorität: 28.02.80 US 125604
15.12.80 US 216116

(43) Veröffentlichungstag der Anmeldung: 09.09.81 Patentblatt 81/36

(45) Bekanntmachung des Hinweises auf die Patenterteilung: 08.05.85 Patentblatt 85/19

(84) Benannte Vertragsstaaten: AT BE CH DE FR GB IT LI LU NL SE

(56) Entgegenhaltungen:
EP - A - 0 010 661

CHEMICAL ABSTRACTS, Band 86, No. 25, 20. Juni 1977, Columbus, Ohio, USA TACHIKAWA "Pyridoindol derivatives" Seite 611, Spalte 2, Zusammenfassung No. 189902k

(73) Patentinhaber: F. HOFFMANN-LA ROCHE & CO. Aktiengesellschaft, CH-4002 Basel (CH)

(72) Erfinder: Berger, Leo, 7 The Parkway, Montclair, N.J. (US)
Erfinder: Olson, Gary Lee, 431 Everson Place, Westfield, N.J. (US)

(74) Vertreter: Lederer, Franz, Dr. et al, Patentanwälte Dr. Franz Lederer Reiner F. Meyer Lucile-Grahn-Strasse 22, D-8000 München 80 (DE)

EP 0 035 244 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Cycloalka[4,5]pyrrolo[2,3-g]isochinoline der allgemeinen Formel

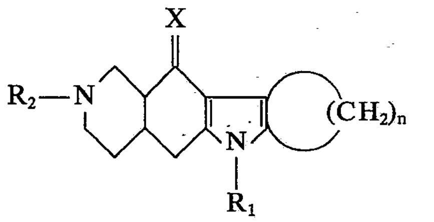

(A)

worin

$R_1$ Wasserstoff, niederes Alkyl, niederes Alkanoyl, Arylcarbonyl oder Aryl-niederes Alkyl,
$R_2$ Wasserstoff, niederes Alkyl, niederes Hydroxyalkyl, Aryl-niederes Hydroxyalkyl, niederes Alkoxy-niederes Alkyl, niederes Alkanoyloxy-niederes Alkyl, Arylcarbonyloxy-niederes Alkyl, niederes Alkanoyl-niederes Alkyl, Arylcarbonyl-niederes Alkyl, Aryl-niederes Alkyl, niederes Alkenyl, $(C_{3-6})$-Cycloalkyl-niederes Alkyl, niederes Alkinyl, Thienyl-niederes Alkyl, Furyl-niederes Alkyl, Arylcarboxamido-niederes Alkyl, Aryl-niederes Alkenyl, niederes Alkenyloxy-niederes Alkyl, Aryloxy-niederes Alkyl, Aryl-niederes Alkyloxy-niederes Alkyl oder Aryl-N-imidazolonyl-niederes Alkyl,
X ein Sauerstoff- oder Schwefelatom und
n die Zahl 3, 4, 5 oder 6

bedeuten,
optische und geometrische Isomeren dieser Verbindungen und pharmazeutisch annehmbare Säureadditionssalze davon.

Der Ausdruck »niederes Alkyl«, wie er in der vorliegenden Beschreibung verwendet wird, bezeichnet einen geradkettigen oder verzweigten gesättigten Kohlenwasserstoffrest mit 1—7 Kohlenstoffatomen; z. B. Methyl, Äthyl, Propyl, Isopropyl, Butyl, tert.Butyl, Neopentyl, Pentyl, Heptyl und dergleichen. Der Ausdruck »$(C_{3-6})$-Cycloalkyl« bezeichnet einen cyclischen Alkylrest mit 3—6 Kohlenstoffatomen, wie z. B. Cyclopropyl, Cyclohexyl und dergleichen. Der Ausdruck »niederes Alkoxy« bezeichnet eine über ein Sauerstoffatom gebundene niedere Alkylgruppe, in der die niedere Alkylgruppe wie zuvor beschrieben ist; z. B. Methoxy, Äthoxy, Propoxy, Pentoxy und dergleichen. Der Ausdruck »niederes Alkenyl« bezeichnet einen geradkettigen oder verzweigten Alkenylrest mit 2—7 Kohlenstoffatomen; z. B. Vinyl, Allyl und dergleichen. Der Ausdruck »niederes Alkenyloxy« bezeichnet eine über ein Sauerstoffatom gebundene niedere Alkenylgruppe, in der die niedere Alkenylgruppe wie zuvor beschrieben ist; z. B. Äthenyloxy und dergleichen. Der Ausdruck »niederes Alkinyl« bezeichnet einen geradkettigen oder verzweigten Alkinylrest mit 2—7 Kohlenstoffatomen; z. B. Äthinyl, Propargyl, Methylbutinyl und dergleichen. Der Ausdruck »Halogen« oder »Halo« bezeichnet die vier Formen Fluor, Chlor, Brom und Jod. Der Ausdruck »Aryl« bedeutet Phenyl oder durch Halogen, Trifluormethyl, niederes Alkyl, niederes Alkoxy, Nitro, Amino, niederes Alkylamino oder di-niederes Alkylamino einfach oder mehrfach substituiertes Phenyl. Der Ausdruck »Aryl-niederes Alkyl« bezeichnet vorzugsweise Benzyl und dergleichen. Der Ausdruck »Aryl-niederes Alkenyl« bedeutet vorzugsweise 3-Phenyl-2-propenyl und dergleichen. Der Ausdruck »Aryl-niederes Alkyloxy« bezeichnet über ein Sauerstoffatom gebundene Aryl-niedere Alkylgruppen, wie z. B. Benzyloxy und dergleichen. Der Ausdruck »Aryloxy« bezeichnet über ein Sauerstoffatom gebundene Arylgruppen, worin die Arylgruppe wie zuvor beschrieben ist; z. B. Phenoxy und dergleichen. Der Ausdruck »niederes Alkanoyl« bedeutet eine von einer Alkancarbonsäure mit 1—7 Kohlenstoffatomen abgeleitete Gruppe, wie z. B. Formyl, Acetyl, Propionyl und dergleichen. Der Ausdruck »Arylcarbonyl« leitet sich von einer Arylcarbonsäure her und bedeutet eine Gruppe wie Benzoyl und dergleichen. Der Ausdruck »niederes Alkanoyloxy« leitet sich von einer Alkancarbonsäure mit 1—7 Kohlenstoffatomen ab, z. B. Formyloxy, Acetoxy, Propionyloxy und dergleichen. Der Ausdruck »Arylcarbonyloxy« leitet sich von einer Arylcarbonsäure ab und bedeutet eine Gruppe wie Benzoyloxy und dergleichen. Beispiele für »niederes Alkanoyl-niederes Alkyl« und »Arylcarbonyl-niederes Alkyl« sind 2-Oxopropyl, 4-(4-Fluorphenyl)-4-oxobutyl und dergleichen. Beispiele für »niederes Alkanoyloxy-niederes Alkyl« und »Arylcarbonyloxy-niederes Alkyl« sind 2-Acetoxyäthyl, 3-Benzoyloxypropyl und dergleichen. Beispiele für »niederes Hydroxyalkyl« sind Hydroxyäthyl, 2-Hydroxy-3,3-dimethylbutyl und dergleichen. Beispiele für »$(C_{3-6})$-Cycloalkyl-niederes Alkyl« sind Cyclopropylmethyl, Cyclobutylmethyl und dergleichen. Beispiele für »Arylcarboxamido-niederes Alkyl« sind Benzamidoäthyl und dergleichen. Beispiele für »Aryloxy-niederes Alkyl« sind 3-Phenoxypropyl und dergleichen. Beispiele für »Aryl-niederes Alkyloxy-niederes Alkyl« sind 2-Benzyloxyäthyl, 3-Benzyloxypropyl und dergleichen. Beispiele für »Aryl-N-imidazolonyl-niederes Alkyl« sind 2-(2,3-Dihydro-2-oxo-1 H-benzimidazol-1-yl)äthyl, 3-(2,3-Dihydro-2-oxo-1 H-benzimidazol-1-yl)propyl und dergleichen. Beispiele für »Aryl-niederes Hydroxyalkyl« sind 2-Hydroxy-2-phenyläthyl, 2-Hydroxy-2-(4-chlorphenyl)äthyl und dergleichen. Beispiele für »niederes Alkoxy-niederes Alkyl« sind 2-Äthoxyäthyl, 3-Methoxypropyl und

dergleichen. Beispiele für »niederes Alkenyloxy-niederes Alkyl« sind 2-Äthenyloxyäthyl und dergleichen.

Bevorzugte Verbindungen der allgemeinen Formel A sind solche, worin n die Zahl 3, 4, 5 oder 6, $R_1$ Wasserstoff, $R_2$ niederes Alkyl, niederes Hydroxyalkyl, Aryl-niederes Hydroxyalkyl, niederes Alkoxy-niederes Alkyl, Aryloxy-niederes Alkyl, niederes Alkanoyl-niederes Alkyl, Arylcarbonyl-niederes Alkyl oder Aryl-niederes Alkyl und X ein Sauerstoff- oder Schwefelatom bedeuten. Besonders bevorzugte Verbindungen der allgemeinen Formel A sind solche, worin n die Zahl 3 oder 4, $R_1$ Wasserstoff, $R_2$ niederes Alkyl, niederes Hydroxyalkyl, Aryl-niederes Hydroxyalkyl, niederes Alkoxy-niederes Alkyl, Aryloxy-niederes Alkyl, niederes Alkanoyl-niederes Alkyl, Arylcarbonyl-niederes Alkyl oder Aryl-niederes Alkyl und X ein Sauerstoffatom bedeuten.

Ganz besonders bevorzugte erfindungsgemäße Verbindungen der allgemeinen Formel A sind:

2-Methyl-1,2,3,4,4a,5,7,9,10a-decahydro-4a,10a-trans-6H-cyclopenta[4,5]pyrrolo-[2,3-g]isochinolin-10(10H)-on,
2-Methyl-1,2,3,4,4a,5,7,8,9,10a-decahydro-4a,10a-trans-6H-cyclopenta[4,5]pyrrolo-[2,3-g]isochinolin-10(10H)-on-hydrochlorid · 0,5 $H_2O$,
2-(2-Phenyläthyl)-1,2,3,4,4a,5,7,8,9,10a-decahydro-4a,10a-trans-6H-cyclopenta-[4,5]pyrrolo-[2,3-g]isochinolin-10(10H)-on,
2-[4-(4-Fluorphenyl)-4-oxobutyl]-1,2,3,4,4a,5,7,8,9,10a-decahydro-4a,10a-trans-6H-cyclopenta[4,5]pyrrolo[2,3-g]isochinolin-10(10H)-on,
(−)-2-Methyl-1,2,3,4,4a,5,7,8,9,10a-decahydro-4a,10a-trans-6H-cyclopenta[4,5]pyrrolo-[2,3-g]isochinolin-10(10H)-on,
2-Methyl-2,3,4,4a,5,7,8,9,10,11a-decahydro-4a,11a-trans-1H,6H-cyclohexa-[4,5]pyrrolo[2,3-g]isochinolin-11(11H)-on,
2-Methyl-2,3,4,4a,5,7,8,9,10,11a-decahydro-4a,11a-trans-1H,6H-cyclohexa-[4,5]pyrrolo[2,3-g]isochinolin-11(11H)-on · 0,75 $H_2O$,
2-(2-Phenyläthyl)-2,3,4,4a,5,7,8,9,10,11a-decahydro-4a,11a-trans-1H,6H-cyclohexa-[4,5]pyrrolo[2,3-g]isochinolin-11(11H)-on,
2-[4-(4-Fluorphenyl)-4-oxobutyl]-2,3,4,4a,5,7,8,9,10,11a-decahydro-4a,11a-trans-1H,6H-cyclohexa[4,5]pyrrolo[2,3-g]isochinolin-11(11H)-on und
2-Methyl-1,2,3,4,4a,5,7,8,9,10a-decahydro-4a,10a-trans-6H-cyclopenta[4,5]pyrrolo-[2,3-g]isochinolin-10(10H)-thion.

Beispiele für Verbindungen der allgemeinen Formel A, worin n die Zahl 3 bedeutet, d. h. Verbindungen der allgemeinen Formel

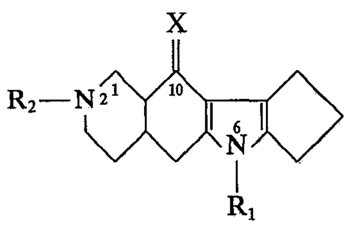

(A-1)

worin $R_1$, $R_2$ und X obige Bedeutung besitzen, sind:

2-Äthyl-1,2,3,4,4a,5,7,9,10a-decahydro-4a,10a-trans-6H-cyclopenta[4,5]pyrrolo-[2,3-g]isochinolin-10(10H)-on,
2-(2-Hydroxyäthyl)-1,2,3,4,4a,5,7,8,9,10a-decahydro-4a,10a-trans-6H-cyclopenta-[4,5]pyrrolo[2,3-g]isochinolin-10(10H)-on,
2-(2-Hydroxy-2-phenyläthyl)-1,2,3,4,4a,5,7,8,9,10a-decahydro-4a,10a-trans-6H-cyclopenta-[4,5]pyrrolo-[2,3-g]isochinolin-10(10H)-on,
2-(2-Äthoxyäthyl)-1,2,3,4,4a,5,7,8,9,10a-decahydro-4a,10a-trans-6H-cyclopenta-[4,5]pyrrolo[2,3-g]isochinolin-10(10H)-on,
2-(2-Acetoxyäthyl)-1,2,3,4,4a,5,7,8,9,10a-decahydro-4a,10a-trans-6H-cyclopenta-[4,5]pyrrolo[2,3-g]isochinolin-10(10H)-on,
2-[3-(4-Fluorphenyl)-3-oxopropyl]-1,2,3,4,4a,5,7,8,9,10a-decahydro-4a,10a-trans-6H-cyclopenta-[4,5]pyrrolo[2,3-g]isochinolin-10(10H)-on,
2-[2-(4-Methoxyphenyl)äthyl]-1,2,3,4,4a,5,7,8,9,10a-decahydro-4a,10a-trans-6H-cyclopenta[4,5]pyrrolo[2,3-g]isochinolin-10(10H)-on,
2-Allyl-1,2,3,4,4a,5,7,8,9,10a-decahydro-4a,10a-trans-6H-cyclopenta[4,5]pyrrolo-[2,3-g]isochinolin-10(10H)-on,
2-Cyclopropylmethyl-1,2,3,4,4a,5,7,8,9,10a-decahydro-4a,10a-trans-6H-cyclopenta-[4,5]pyrrolo[2,3-g]isochinolin-10(10H)-on,
2-Propargyl-1,2,3,4,4a,5,7,8,9,10a-decahydro-4a,10a-trans-6H-cyclopenta[4,5]pyrrolo-

[2,3-g]isochinolin-10(10H)-on,
2-[2-(2-Thienyl)äthyl]-1,2,3,4,4a,5,7,8,9,10a-decahydro-4a,10a-trans-6H-cyclopenta-[4,5]pyrrolo[2,3-g]isochinolin-10(10H)-on,
2-[2-(2-Furyl)äthyl]-1,2,3,4,4a,5,7,8,9,10a-decahydro-4a,10a-trans-6H-cyclopenta-[4,5]pyrrolo[2,3-g]isochinolin-10(10H)-on,
2-[2-(2,3-Dihydro-2-oxo-1H-benzimidazol-1-yl)äthyl]-1,2,3,4,4a,5,7,8,9,10-decahydro-4a,10a-trans-6H-cyclopenta[4,5]pyrrolo[2,3-g]isochinolin-10(10H)-on,
2-[2-(Benzyloxy)äthyl]-1,2,3,4,4a,5,7,8,9,10a-decahydro-4a,10a-trans-6H-cyclopenta-[4,5]pyrrolo[2,3-g[isochinolin-10(10H)-on,
2-(3-Phenyl-2-propenyl)-1,2,3,4,4a,5,7,8,9,10a-decahydro-4a,10a-trans-6H-cyclopenta-[4,5]pyrrolo[2,3-g]isochinolin-10(10H)-on,
2-[2-(4-Fluorbenzamido)äthyl]-1,2,3,4,4a,5,7,8,9,10a-decahydro-4a,10a-trans-6H-cyclopenta[4,5]pyrrolo[2,3-g]isochinolin-10(10H)-on,
2-[2-(Äthenyloxy)äthyl]-1,2,3,4,4a,5,7,8,9,10a-decahydro-4a,10a-trans-6H-cyclopenta-[4,5]pyrrolo[2,3-g]isochinolin-10(10H)-on,
2-Benzyl-1,2,3,4,4a,5,7,8,9,10a-decahydro-4a,10a-trans-6H-cyclopenta[4,5]pyrrolo-[2,3-g]isochinolin-10(10H)-on,
1,2,3,4,4a,5,7,8,9,10a-Decahydro-4a,10a-trans-6H-cyclopenta[4,5]pyrrolo[2,3-g]-isochinolin-10(10H)-on,
6-Benzoyl-2-methyl-1,2,3,4,4a,5,7,8,9,10a-decahydro-4a,10a-trans-6H-cyclopenta-[4,5]pyrrolo[2,3-g]isochinolin-10(10H)-on,
2,6-Dimethyl-1,2,3,4,4a,5,7,8,9,10a-decahydro-4a,10a-trans-6H-cyclopenta[4,5]pyrrolo-[2,3-g]isochinolin-10(10H)-on,
6-Benzyl-2-methyl-1,2,3,4,4a,5,7,8,9,10a-decahydro-4a,10a-trans-6H-cyclopenta-[4,5]pyrrolo[2,3-g]isochinolin-10(10H)-on,
6-Methyl-2-[4-(4-fluorphenyl)-4-oxobutyl]-1,2,3,4,4a,5,7,8,9,10a-decahydro-4a,10a-trans-6H-cyclopenta[4,5]pyrrolo[2,3-g]isochinolin-10(10H)-on,
1,2,3,4,4a,5,7,8,9,10a-Decahydro-4a,10a-trans-6H-cyclopenta[4,5]pyrrolo-[2,3-g]isochinolin-10(10H)-thion,
2-(2-Hydroxy-3,3-dimethylbutyl)-1,2,3,4,4a,5,7,8,9,10a-decahydro-4a,10a-trans-6H-cyclopenta[4,5]pyrrolo[2,3-g]isochinolin-10(10H)-thion,
2-(2-Phenyläthyl)-1,2,3,4,4a,5,7,8,9,10a-decahydro-4a,10a-trans-6H-cyclopenta-[4,5]pyrrolo[2,3-g]isochinolin-10(10H)-thion und
2-[4-(4-Fluorphenyl)-4-oxobutyl]-1,2,3,4,4a,5,7,8,9,10a-decahydro-4a,10a-trans-6H-cyclopenta[4,5]pyrrolo[2,3-g]isochinolin-10(10H)-thion.

Beispiele für Verbindungen der allgemeinen Formel A, worin n die Zahl 4 bedeutet, d. h. Verbindungen der allgemeinen Formel

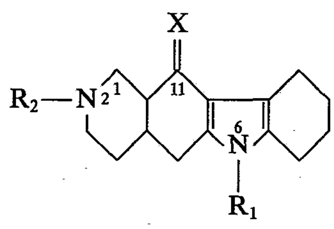

(A-2)

worin $R_1$, $R_2$ und X obige Bedeutung besitzen, sind:

2-(2-Hydroxy-3,3-dimethylbutyl)-2,3,4,4a,5,7,8,9,10,11a-decahydro-4a,11a-trans-1H,6H-cyclohexa[4,5]pyrrolo[2,3-g]isochinolin-11(11H)-on,
2-(2-Äthoxyäthyl)-2,3,4,4a,5,7,8,9,10,11a-decahydro-4a,11a-trans-1H,6H-cyclohexa-[4,5]pyrrolo[2,3-g]isochinolin-11(11H)-on,
2-Cyclobutylmethyl-2,3,4,4a,5,7,8,9,10,11a-decahydro-4a,11a-trans-1H,6H-cyclohexa-[4,5]pyrrolo[2,3-g]isochinolin-11(11H)-on,
2-[2-(2-Thienyl)äthyl]-2,3,4,4a,5,7,8,9,10,11a-decahydro-4a,11a-trans-1H,6H-cyclohexa-[4,5]pyrrolo[2,3-g]isochinolin-11(11H)-on,
2-[2-(2-Furyl)äthyl]-2,3,4,4a,5,7,8,9,10,11a-decahydro-4a,11a-trans-1H-6H-cyclohexa[4,5]pyrrolo[2,3-g]isochinolin-11(11H)-on,
2-[3-(4-Fluorphenyl)-3-oxopropyl]-2,3,4,4a,5,7,8,9,10,11a-decahydro-4a,11a-trans-1H,6H-cyclohexa[4,5]pyrrolo[2,3-g]isochinolin-11(11H)-on,
2-(2-Propenyl)-2,3,4,4a,5,7,8,9,10,11a-decahydro-4a,11a-trans-1H,6H-cyclohexa-[4,5]pyrrolo[2,3-g]isochinolin-11(11H)-on,
2-(3-Phenoxypropyl)-2,3,4,4a,5,7,8,9,10,11a-decahydro-4a,11a-trans-1H,6H-cyclohexa-[4,5]pyrrolo[2,3-g]isochinolin-11(11H)-on,

2-[3-(2,3-Dihydro-2-oxo-1 H-benzimidazol-1-yl)propyl]-2,3,4,4a,5,7,8,9,10,11a-decahydro-4a,11a-trans-1 H,6 H-cyclohexa[4,5]pyrrolo[2,3-g]isochinolin-11(11 H)-on,
2-Benzyl-2,3,4,4a,5,7,8,9,10,11a-decahydro-4a,11a-trans-1 H,6 H-cyclohexa[4,5]pyrrolo-[2,3-g]isochinolin-11(11 H)-on,
2,3,4,4a,5,7,8,9,10,11a-Decahydro-4a,11a-trans-1 H,6 H-cyclohexa[4,5]pyrrolo-[2,3-g]isochinolin-11(11 H)-on,
2,6-Dimethyl-2,3,4,4a,5,7,8,9,10,11a-decahydro-4a,11a-trans-1 H,6 H-cyclohexa-[4,5]pyrrolo[2,3-g]isochinolin-11(11 H)-on,
6-Benzoyl-2-[4-(4-fluorphenyl)-4-oxobutyl]-2,3,4,4a,5,7,8,9,10,11a-decahydro-4a,11a-trans-1 H,6 H-cyclohexa[4,5]pyrrolo[2,3-g]isochinolin-11(11 H)-on,
2-(2-Phenyläthyl)-2,3,4,4a,5,7,8,9,10,11a-decahydro-4a,11a-trans-1 H,6 H-cyclohexa-[4,5]pyrrolo[2,3-g]isochinolin-11(11 H)-thion,
2,3,4,4a,5,7,8,9,10,11a-Decahydro-4a,11a-trans-1 H,6 H-cyclohexa[4,5]pyrrolo-[2,3-g]isochinolin-11(11 H)-thion und
2-[4-(4-Fluorphenyl)-4-oxobutyl]-2,3,4,4a,5,7,8,9,10,11a-decahydro-4a,11a-trans-1 H,6 H-cyclohexa[4,5]pyrrolo[2,3-g]isochinolin-11(11 H)-thion.

Für Verbindungen der allgemeinen Formel A-2, worin X ein Sauerstoffatom bedeutet, kann auch eine andere Nomenklatur verwendet werden. Beispielsweise sind 2-Methyl-2,3,4,4a,5,7,8,9,10,11a-decahydro-4a,11a-trans-1 H,6 H-cyclohexa[4,5]pyrrolo[2,3-g]isochinolin-11(11 H)-on und 2-Methyl-2,3,4,4a,5,7,8,9,10,11a-decahydro-4a,11a-trans-pyrido[4,3-b]carbazol-11(1 H,6 H)-on ein und dieselbe Verbindung.

Beispiele für Verbindungen der allgemeinen Formel A, worin n die Zahl 5 bedeutet, d. h. Verbindungen der allgemeinen Formel

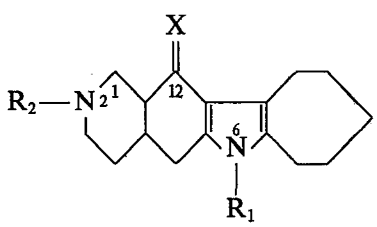

(A-3)

worin $R_1$, $R_2$ und X obige Bedeutung besitzen, sind:

2-Methyl-1,2,3,4,4a,5,7,8,9,10,11,12a-dodecahydro-4a,12a-trans-6 H-cyclohepta-[4,5]pyrrolo[2,3-g]isochinolin-12(12 H)-on,
2-[4-(4-Fluorphenyl)-4-oxobutyl]-1,2,3,4,4a,5,7,8,9,10,11,12a-dodecahydro-4a,12a-trans-6 H-cyclohepta[4,5]pyrrolo[2,3-g]isochinolin-12(12 H)-on,
2-(3-Phenoxypropyl)-1,2,3,4,4a,5,7,8,9,10,11,12a-dodecahydro-4a,12a-trans-6 H-cyclohepta[4,5]pyrrolo[2,3-g]isochinolin-12(12 H)-on,
2-(2-Phenyläthyl)-1,2,3,4,4a,5,7,8,9,10,11,12a-dodecahydro-4a,12a-trans-6 H-cyclohepta-[4,5]pyrrolo[2,3-g]isochinolin-12(12 H)-on,
2-[4-(4-Fluorphenyl)-4-oxobutyl]-1,2,3,4,4a,5,7,8,9,10,11,12a-dodecahydro-4a,12a-trans-6 H-cyclohepta[4,5]pyrrolo[2,3-g]isochinolin-12(12 H)-thion,
2-(3-Phenoxypropyl)-1,2,3,4,4a,5,7,8,9,10,11,12a-dodecahydro-4a,12a-trans-6 H-cyclohepta[4,5]pyrrolo[2,3-g]isochinolin-12(12 H)-thion und
2-(2-Phenyläthyl)-1,2,3,4,4a,5,7,8,9,10,11,12a-dodecahydro-4a,12a-trans-6 H-cyclohepta-[4,5]pyrrolo[2,3-g]isochinolin-12(12 H)-thion.

Beispiele für Verbindungen der allgemeinen Formel A, worin n die Zahl 6 bedeutet, d. h. Verbindungen der allgemeinen Formel

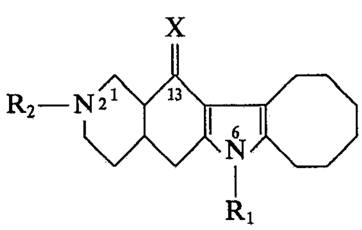

(A-4)

worin $R_1$, $R_2$ und X obige Bedeutung besitzen, sind:

2-[4-(4-Fluorphenyl)-4-oxobutyl]-2,3,4,4a,5,7,8,9,10,11,12,13a-dodecahydro-4a,13a-trans-1 H,6 H-cycloocta[4,5]pyrrolo[2,3-g]isochinolin-13(13 H)-on,

2-(2-Phenyläthyl)-2,3,4,4a,5,7,8,9,10,11,12,12a-dodecahydro-4a,13a-trans-1H,6H-cycloocta[4,5]pyrrolo[2,3-g]isochinolin-13(13H)-on,
2-(2-Hydroxy-3,3-dimethylpropyl)-2,3,4,4a,5,7,8,9,10,11,12,13a-dodecahydro-4a,13a-trans-1H,6H-cycloocta[4,5]pyrrolo[2,3-g]isochinolin-13(13H)-on,
2-Methyl-2,3,4,4a,5,7,8,9,10,11,12,13a-dodecahydro-4a,13a-trans-1H,6H-cycloocta-[4,5]pyrrolo[2,3-g]isochinolin-13(13H)-on,
2-(3-Phenoxypropyl)-2,3,4,4a,5,7,8,9,10,11,12,13a-dodecahydro-4a,13a-trans-1H,6H-cycloocta[4,5]pyrrolo[2,3-g]isochinolin-13(13H)-on,
2-[4-(4-Fluorphenyl)-4-oxobutyl]-2,3,4,4a,5,7,8,9,10,11,12,13a-dodecahydro-4a,13a-trans-1H,6H-cycloocta[4,5]pyrrolo[2,3-g]isochinolin-13(13H)-thion,
2-(2-Phenyläthyl)-2,3,4,4a,5,7,8,9,10,11,12,13a-dodecahydro-4a,13a-trans-1H,6H-cycloocta[4,5]pyrrolo[2,3-g]isochinolin-13(13H)-thion,
2-(2-Äthoxyäthyl)-2,3,4,4a,5,7,8,9,10,11,12,13a-dodecahydro-4a,13a-trans-1H,6H-cycloocta[4,5]pyrrolo[2,3-g]isochinolin-13(13H)-thion und
2-(3-Phenoxypropyl)-2,3,4,4a,5,7,8,9,10,11,12,13a-dodecahydro-4a,13a-trans-1H,6H-cycloocta[4,5]pyrrolo[2,3-g]isochinolin-13(13H)-thion.

Die erfindungsgemäßen Verbindungen, worin n die Zahl 3 bedeutet, können als 4a,10a-trans- oder als 4a,10a-cis-Isomere oder als Mischungen davon vorliegen; die 4a,10a-trans-Isomeren werden bevorzugt.

Die erfindungsgemäßen Verbindungen, worin n die Zahl 4 bedeutet, können als 4a,11a-trans- oder als 4a,11a-cis-Isomere oder als Mischungen davon vorliegen; die 4a,11a-trans-Isomeren werden bevorzugt.

Die erfindungsgemäßen Verbindungen, worin n die Zahl 5 bedeutet, können als 4a,12a-trans- oder als 4a,12a-cis-Isomere oder als Mischungen davon vorliegen; die 4a,12a-trans-Isomeren werden bevorzugt.

Die erfindungsgemäßen Verbindungen, worin n die Zahl 6 bedeutet, können als 4a,13a-trans- oder als 4a,13a-cis-Isomere oder als Mischungen davon vorliegen; die 4a,13a-trans-Isomeren werden bevorzugt.

Die Verbindungen der obigen allgemeinen Formel A, ihre optischen und geometrischen Isomeren und ihre pharmazeutisch annehmbaren Säureadditionssalze können erfindungsgemäß dadurch hergestellt werden, daß man

a) zur Herstellung einer Verbindung der allgemeinen Formel

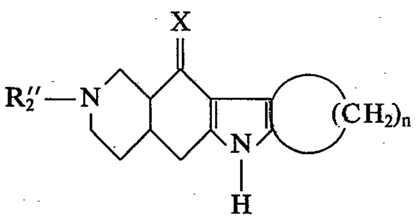

(Ia)

worin

$R_2''$ niederes Alkyl, niederes Alkoxy-niederes Alkyl oder $(C_{3-6})$-Cycloalkyl-niederes Alkyl bedeutet und
n obige Bedeutung besitzt,

eine Verbindung der allgemeinen Formel

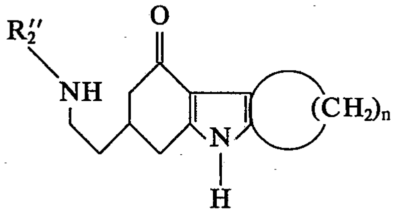

(IX)

worin $R_2''$ und n obige Bedeutung besitzen,

mit Formaldehyd behandelt, oder

b) zur Herstellung einer Verbindung der obigen allgemeinen Formel Ia, eine Verbindung der allgemeinen Formel

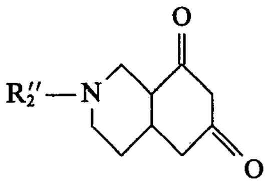

(XII)

worin $R_2''$ obige Bedeutung besitzt,

mit einer Verbindung der allgemeinen Formel

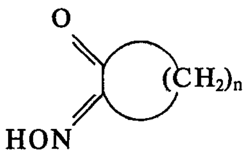

(VII)

in Gegenwart eines Reduktionsmittels oder mit einer Verbindung der allgemeinen Formel

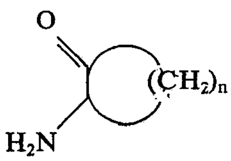

(VIII)

worin n obige Bedeutung besitzt,

oder einer Vorstufe von dieser, behandelt, oder

c) zur Herstellung einer Verbindung der allgemeinen Formel

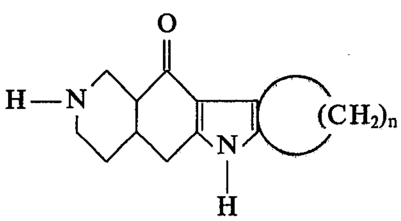

(Ib)

worin n obige Bedeutung besitzt,

eine Verbindung der obigen allgemeinen Formel Ia, worin $R_2''$ bedeutet, N-demethyliert, oder

d) zur Herstellung einer Verbindung der allgemeinen Formel

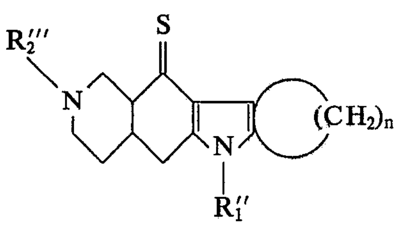

(IIe)

worin

$R_1''$ Wasserstoff, niederes Alkyl oder Aryl-niederes Alkyl und
$R_2'''$ Wasserstoff, niederes Alkyl, niederes Alkoxy-niederes Alkyl, Aryl-niederes Alkyl, niederes Alkenyl, $(C_{3-6})$-Cycloalkyl-niederes Alkyl, niederes Alkinyl, Thienyl-niederes Alkyl, Furyl-niederes Alkyl, niederes Alkenyloxy-niederes Alkyl, Aryl-niederes Alkenyl, Aryloxy-niederes Alkyl oder Aryl-niederes Alkyloxy-niederes Alkyl bedeuten und
n obige Bedeutung besitzt,

eine Verbindung der allgemeinen Formel

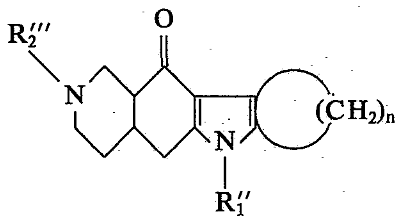

(Ie)

worin $R_1''$, $R_2'''$ und n obige Bedeutung besitzen,

mit Phosphorpentasulfid behandelt, oder

e) zur Herstellung einer Verbindung der allgemeinen Formel

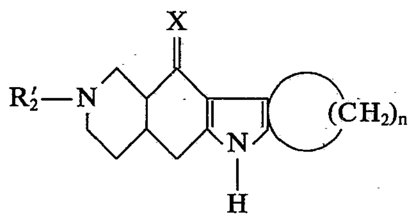

(Ad)

worin

$R_2'$ niederes Alkyl, niederes Hydroxyalkyl, Aryl-niederes Hydroxyalkyl, niederes Alkoxy-niederes Alkyl, niederes Alkanoyloxy-niederes Alkyl, Arylcarbonyloxy-niederes Alkyl, niederes Alkanoyl-niederes Alkyl, Arylcarbonyl-niederes Alkyl, Aryl-niederes Alkyl, niederes Alkenyl, $(C_{3-6})$-Cycloalkyl-niederes Alkyl, niederes Alkinyl, Thienyl-niederes Alkyl, Furyl-niederes Alkyl, Arylcarboxamido-niederes Alkyl, Aryl-niederes Alkenyl, niederes Alkenyloxy-niederes Alkyl, Aryloxy-niederes Alkyl, Aryl-niederes Alkyloxy-niederes Alkyl oder Aryl-N-imidazolonyl-niederes Alkyl

bedeutet und

X und n obige Bedeutung besitzen,

eine Verbindung der allgemeinen Formel

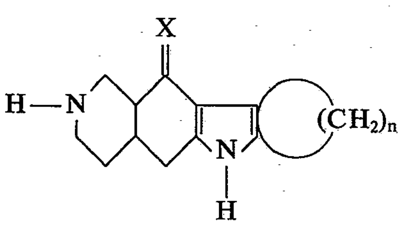

(Ab)

worin X und n obige Bedeutung besitzen,

am Isochinolin-Stickstoffatom substituiert, oder

f) zur Herstellung einer Verbindung der allgemeinen Formel

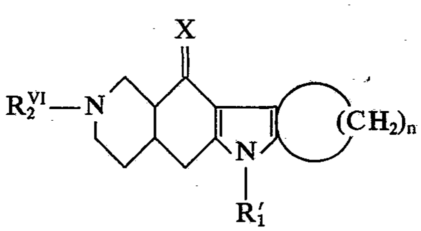

(A'c)

worin

$R_1'$ niederes Alkyl, niederes Alkanoyl, Arylcarbonyl oder Aryl-niederes Alkyl und

$R_2^{VI}$ niederes Alkyl, niederes Alkoxy-niederes Alkyl, niederes Alkanoyloxy-niederes Alkyl, Arylcarbonyloxy-niederes Alkyl, niederes Alkanoyl-niederes Alkyl, Arylcarbonyl-niederes Alkyl, Aryl-niederes Alkyl, niederes Alkenyl, $(C_{3-6})$-Cycloalkyl-niederes Alkyl, Thienyl-niederes Alkyl, niederes Alkinyl, Furyl-niederes Alkyl, Arylcarboxamido-niederes Alkyl, Aryl-niederes Alkenyl, niederes Alkenyloxy-niederes Alkyl, Aryloxy-niederes Alkyl, Aryl-niederes Alkyloxy-niederes Alkyl oder Aryl-N-imidazolonyl-niederes Alkyl bedeuten und
X und n obige Bedeutung besitzen,

eine Verbindung der allgemeinen Formel

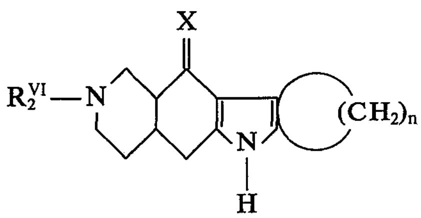

(Ad')

worin $R_2^{VI}$, X und n obige Bedeutung besitzen,

am Pyrrol-Stickstoffatom substituiert, oder

g) zur Herstellung einer Verbindung der allgemeinen Formel

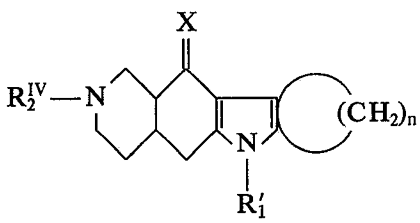

(Ac'')

worin

$R_2^{VI}$ niederes Hydroxyalkyl oder Aryl-niederes Hydroxyalkyl bedeuten und
$R_1'$, X und n obige Bedeutung besitzen,

in einer Verbindung der allgemeinen Formel

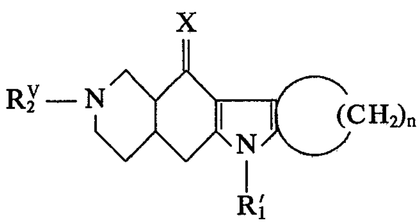

(XIV)

worin

$R_2^{V}$ eine an der Hydroxygruppe geschützte niedere Hydroxyalkyl- oder Aryl-niedere Hydroxyalkyl-Gruppe bedeutet und
$R_1'$, X und n obige Bedeutung besitzen,

die Schutzgruppe abspaltet, oder

h) zur Herstellung einer Verbindung der allgemeinen Formel

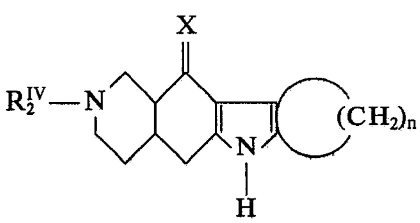

(Ad'')

worin

$R_2^{IV}$, X und n obige Bedeutung besitzen,

in einer Verbindung der allgemeinen Formel

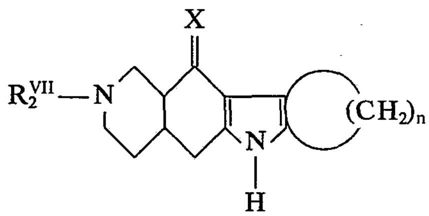

(Ad''')

worin

$R_2^{VII}$ niederes Alkanoyl-niederes Alkyl oder Arylcarbonyl-niederes Alkyl oder Arylcarbonyl-niederes Alkyl bedeutet und
X und n obige Bedeutung besitzen,

die niedere Alkanoyl-niedere Alkyl- oder Arylcarbonyl-niedere Alkyl-Gruppe reduziert, oder

i) zur Herstellung einer Verbindung der allgemeinen Formel

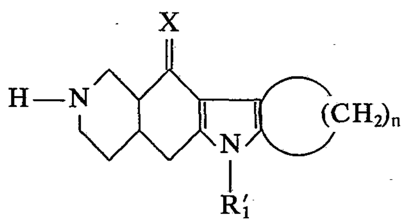

(Ah)

worin X, n und $R_1'$ obige Bedeutung besitzen,

in einer Verbindung der allgemeinen Formel

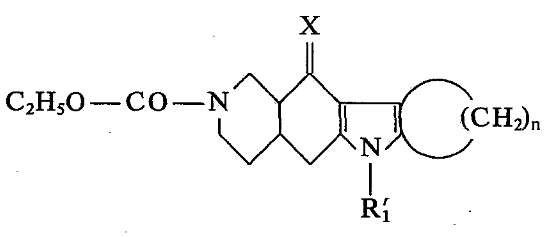

(XIII)

worin X, n und $R_1'$ obige Bedeutung besitzen,

die Äthoxycarbonylgruppe abspaltet, und

j) erwünschtenfalls ein erhaltenes Gemisch der cis- und trans-Isomeren zu einem Endverhältnis, das überwiegend das trans-Isomere aufweist, isomerisiert, und/oder
k) erwünschtenfalls das trans-Isomere aus dem erhaltenen Gemisch abtrennt, und/oder
l) erwünschtenfalls ein erhaltenes Racemat in die optischen Antipoden spaltet, und/oder
m) erwünschtenfalls eine erhaltene Verbindung oder ein pharmazeutisch nicht annehmbares Säureadditionssalz in ein pharmazeutisch annehmbares Säureadditionssalz überführt.

Insbesondere können die Verbindungen der obigen Formel A, ihre optischen und geometrischen Isomeren und ihre pharmazeutisch annehmbaren Säureadditionssalze sowie verschiedene Zwischenstufen wie nachfolgend im einzelnen veranschaulicht hergestellt werden.
So werden z. B. die Verbindungen der Formel A, worin X ein Sauerstoffatom bedeutet, durch die Formel

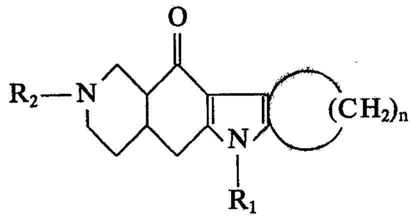

(I)

worin n, $R_1$ und $R_2$ obige Bedeutung besitzen,

charakterisiert und können hergestellt werden, wie in den Schemata I, II, III und IV aufgeführt und weiter beschrieben.

Formelschema I

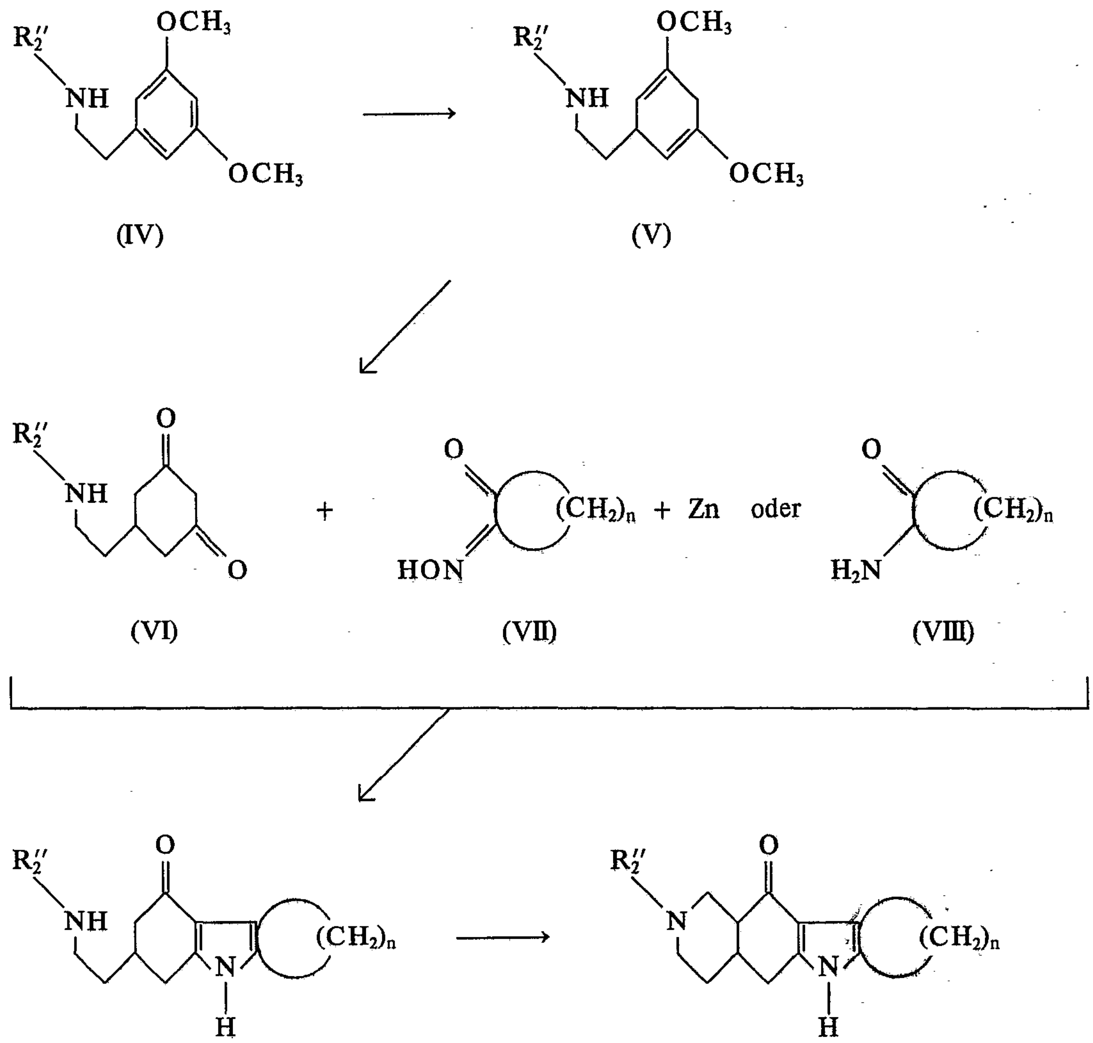

(IV) (V)

(VI) + (VII) + Zn oder (VIII)

(IX) (Ia)

worin

n obige Bedeutung besitzt und
$R_2''$ niederes Alkyl, niederes Alkoxy-niederes Alkyl oder $(C_{3-6})$-Cycloalkyl-niederes Alkyl bedeutet.

Gemäß Formelschema I können Verbindungen der Formel Ia aus bekannten Verbindungen der Formel IV, worin $R_2''$ niederes Alkyl, niederes Alkoxy-niederes Alkyl oder $(C_{3-6})$-Cycloalkyl-niederes Alkyl bedeutet, hergestellt werden. Die Birch-Reduktion eines Amins der Formel IV mit Lithium in tert. Butanol enthaltendem Ammoniak liefert das Dihydroamin der Formel V. Andere Abwandlungen der Birch-Reduktion können auch angewandt werden. So kann ein Amin der Formel IV mit einem Alkalimetall, wie Natrium, Lithium, Kalium oder Cäsium, in Ammoniak oder in einem Amin, wie Methylamin oder

Äthylamin, in Gegenwart eines niederen Alkanols, wie Äthanol, Butanol oder tert.-Butanol, umgesetzt werden. Die Reaktion erfolgt im allgemeinen beim Siedepunkt des Lösungsmittels oder darunter, z. B. von −78 bis 15°C. Verwendet man Ammoniak als Lösungsmittel, so erfolgt die Reaktion bei der Rückflußtemperatur. Gegebenenfalls können Lösungsvermittler, wie Diäthyläther oder Tetrahydrofuran, zugesetzt werden.

Die Hydrolyse eines Dihydroamins der Formel V erfolgt leicht nach üblichen Methoden der Hydrolyse von Enoläthern, z. B. mit wäßriger Säure. Beispiele für verwendbare Säuren sind Salzsäure, Bromwasserstoffsäure, Ameisensäure, Essigsäure, p-Toluolsulfonsäure und Perchlorsäure. Sie können in wäßrigen Lösungen oder gemischten Lösungsmitteln verwendet werden. Tetrahydrofuran, Benzol, Diäthyläther, Aceton, Toluol, Dioxan oder Acetonitril sind Beispiele für verwendbare Lösungsmittel. Beispielsweise führt die Hydrolyse eines Dihydroamins der Formel V, worin $R_4''$ Methyl bedeutet, in 2 N-Salzsäure bei Raumtemperatur oder darüber oder in wäßriger Essigsäure zwischen 40°C und der Rückflußtemperatur zu einem Diketon der Formel VI, worin $R_2''$ Methyl bedeutet.

Das Diketon der Formel VI kann in einer Knorr-Kondensation zu einem Methylaminoäthylketon der Formel IX kondensiert werden. Die Knorr-Kondensation ist ein wohlbekanntes Verfahren zur Herstellung von Pyrrolen, und das Verfahren kann bei jeder der bekannten Abwandlungen angewandt werden (zu beispielsweisen Bedingungen vgl. J. M. Patterson, Synthesis, 281, 1976 und die dort genannten Literaturstellen). So wird angenommen, daß z. B. die Reaktion eines Isonitrosoketons der Formel VII in Gegenwart eines Reduktionsmittels, z. B. mit Zink in wäßriger Essigsäure oder Salzsäure, über die Aminocarbonylverbindung der Formel VIII verläuft, die dann mit dem Diketon der Formel VI zum Methylaminoäthylketon der Formel IX kondensiert. Andererseits kann die Kondensation mit einer Aminocarbonylverbindung der Formel VIII oder einer Vorstufe, wie einem Aminoketon-hydrochlorid oder einem Ketalderivat eines Aminoketons, erfolgen. Die Verwendung einer Vorstufe eines Aminoketons wird bevorzugt, da solche Aminoketone leicht zu Selbstkondensation neigen. Sie werden am besten in Gegenwart eines Diketons der Formel VI in situ freigesetzt. Die Aminocarbonyl-Komponente reagiert sofort zu Verbindung der Formel IX. Das Diketon der Formel VI muß nicht notwendigerweise vor der Durchführung der Knorr-Kondensation isoliert werden, da die angewandten Reaktionsbedingungen zur Hydrolyse des Dihydroamins der Formel V zu dem Diketon der Formel VI ausreichen. Die Knorr-Kondensation wird am besten bei einem pH von etwa 2—6 durchgeführt. Bei einem pH wesentlich über 6 tritt erheblicher Ausbeutenverlust ein, und zwar aufgrund der Bildung von Selbstkondensationsprodukten der Aminocarbonylverbindung der Formel VIII.

Vorzugsweise kondensiert man ein Isonitrosoketon der Formel VII mit Zinkstaub in wäßriger Essigsäure mit einem Diketon der Formel VI, worin $R_2''$ Methyl bedeutet, wobei man das Methylaminoäthylketon der Formel IX, worin $R_2''$ Methyl bedeutet, erhält.

Die Knorr-Kondensation erfolgt vorzugsweise in einem Temperaturbereich von etwa Raumtemperatur bis Rückflußtemperatur. Die Isonitrosoketone der Formel VII sind bekannte Verbindungen oder können leicht durch Nitrosierung eines entsprechenden β-Ketoesters, beispielsweise mit Natriumnitrit, hergestellt werden (siehe z. B. T. A. Geissmann and M. J. Schlatter, J. Org. Chem. 11, 771 [1946]).

Beispiele für bei der Knorr-Kondensation verwendbare Isonitrosoketone sind folgende:

2-Isonitrosocyclopentanon,
2-Isonitrosocyclohexanon,
2-Isonitrosocycloheptanon und
2-Isonitrosocyclooctanon.

Beispiele für bei der Knorr-Kondensation verwendbare Aminocarbonyl-Vorstufen sind folgende:

2-Aminocyclohexanon-hydrochlorid,
2-Aminocyclopentanon-hydrochlorid,
2-Aminocycloheptanon-hydrochlorid und
2-Aminocyclooctanon-hydrochlorid.

Diese Verbindungen sind bekannt oder können durch Reduktion des entsprechenden Isonitrosoketons, beispielsweise durch katalytische Hydrierung in Gegenwart von Salzsäure, hergestellt werden.

Ein Amin der Formel IX kann über eine intramolekulare Mannich-Reaktion in eine Verbindung der Formel Ia übergeführt werden. Die Mannich-Reaktion wird gewöhnlich ausgehend von einem Keton und einem Dialkylaminsalz, z. B. Dimethylaminhydrochlorid und Formaldehyd (z. B. als wäßrige Lösung, als p-Formaldehyd oder als Trioxan) in einem alkoholischen Lösungsmittel, wie Äthanol, ausgehend, bei der Rückflußtemperatur durchgeführt. Bei der hier beschriebenen Ausführungsform wird ein Säureadditionssalz eines Methylaminoäthylketons der Formel IX mit Formaldehyd, zugesetzt in Form von p-Formaldehyd, Trioxan oder als wäßriger Formaldehyd in einem Lösungsmittel, umgesetzt. Beispielsweise kann ein hochsiedendes hydroxylhaltiges Lösungsmittel, wie Amylalkohol, Octanol, Äthylenglykol oder Diäthylenglykolmonoäthyläther, ein hochsiedendes polares aprotisches Lösungsmittel, wie Dimethylformamid, N-Methylpyrrolidinon oder Diäthylenglykol-dimethyläther, ein tiefsiedendes polares Lösungsmittel, wie Äthanol, Butanol oder 2-Propanol unter Druck, oder ein tiefsiedendes aprotisches Lösungsmittel unter Druck, wie Dioxan oder Tetrahydrofuran, bei einer Temperatur im Bereich von etwa 135 bis etwa 200°C verwendet werden, um die Cycloalka[4,5]pyrrolo[2,3-g]isochinoline der Formel Ia zu erhalten. Die Reaktion führt insbesondere bei Temperaturen unter 150°C zu einem Gemisch von cis- und trans-Isomeren, d. h. z. B. wenn $R_2''$ Methyl bedeutet, zu Verbindungen der Formeln

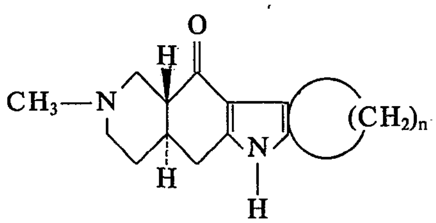

(If')

trans

und

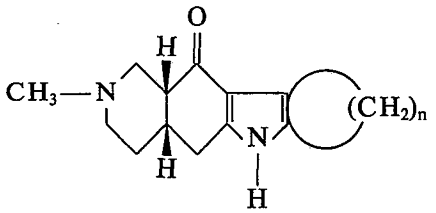

(Ig')

cis

Längeres Erwärmen des Reaktionsgemisches oder getrenntes Erwärmen des Isomerengemisches der Hydrochloride der Verbindungen If' und Ig', z. B. in Äthylenglykol 2 Stunden unter Rückfluß, kann zur Gleichgewichtseinstellung der cis- und trans-Isomeren zu einem Endverhältnis angewandt werden, das überwiegend das trans-Isomere enthält, das leicht durch Kristallisation oder durch chromatographische Trennung isoliert werden kann.

Wenn beispielsweise das Hydrochlorid eines Amins der Formel IX, worin $R_2''$ Methyl bedeutet, während 2 Stunden mit p-Formaldehyd in Butanol bei 180°C umgesetzt wird, erhält man als Produkt fast ausschließlich das trans-Isomere If'.

Formelschema II

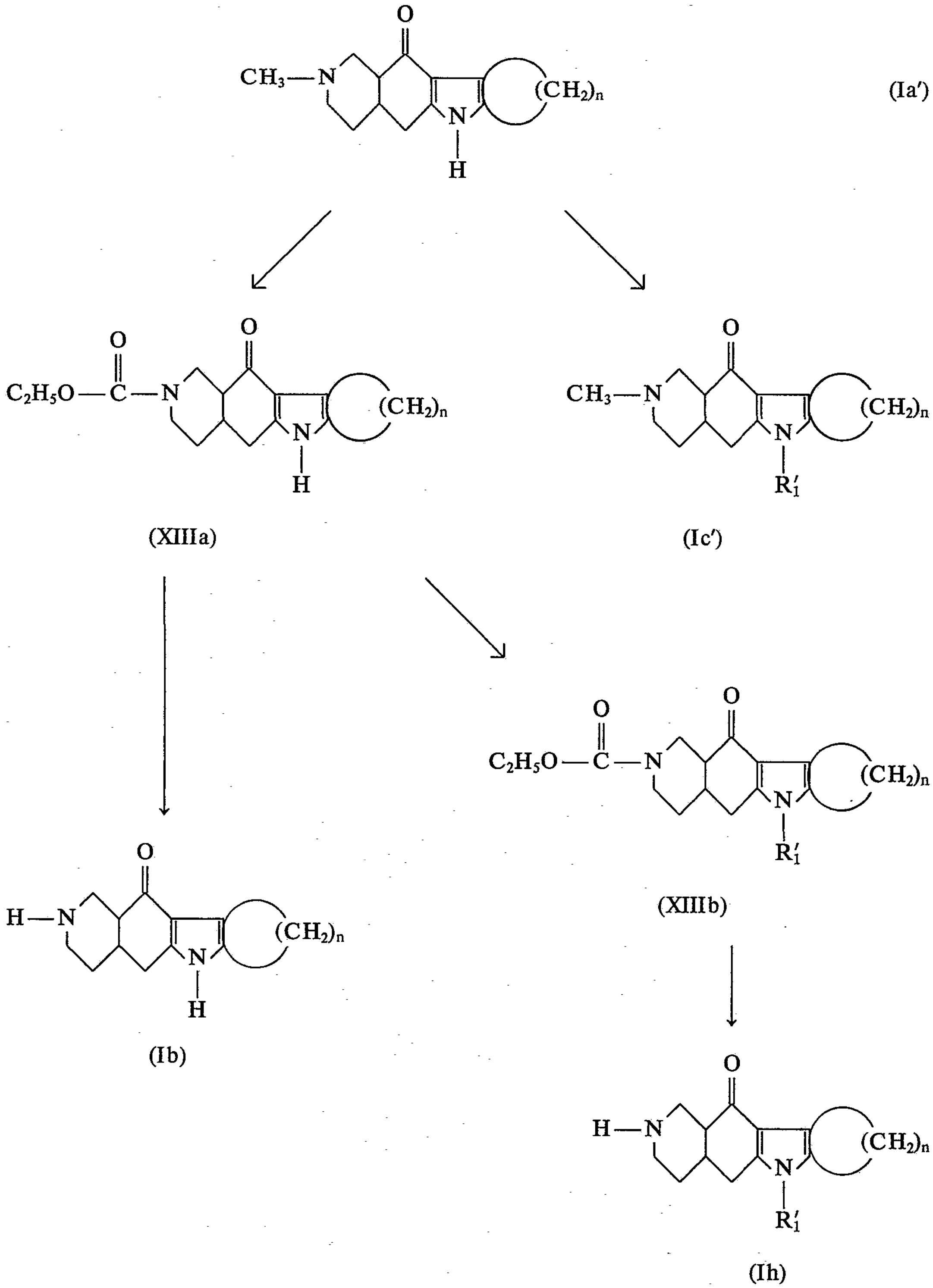

(Ia′)

(XIIIa)

(Ic′)

(Ib)

(XIIIb)

(Ih)

worin

n obige Bedeutung besitzt und
$R_1'$ niederes Alkyl, niederes Alkanoyl, Arylcarbonyl oder Aryl-niederes Alkyl bedeutet.

Gemäß Formelschema II können Verbindungen der Formel Ic′ durch Alkylierung bzw. Acylierung des Pyrrol-Stickstoffatoms in einer Verbindung der Formel Ia′ und in anderen N-2-Alkyl-derivaten hergestellt werden, indem man mit einer starken Base, wie z. B. Natriumamid, Kaliumhydrid, Natriummethylsulfinylcarbanion, Kalium-t-butoxid oder Butyllithium, oder mit einem Alkalimetall, das Pyrrolanion herstellt, und dieses mit einem Alkyl- oder Acylhalogenid in einem Lösungsmittel, wie Tetrahydrofuran, Dioxan, Äthyläther, Dimethylformamid oder Dimethylsulfoxid, abfängt. Beispielsweise erhält man

durch Behandeln einer Verbindung der Formel Ia', worin n die Zahl 3 bedeutet, mit Kalium-t-butoxid in Tetrahydrofuran und anschließend mit Methyljodid das 6-Methyl-derivat, d. h. eine Verbindung der Formel Ic', worin n die Zahl 3 und $R_1'$ Methyl bedeuten. In ähnlicher Weise erhält man durch Reaktion einer Verbindung der Formel Ia', worin n die Zahl 4 bedeutet, mit Butyllithium in Tetrahydrofuran bei −30°C und anschließend mit Benzoylchlorid, das 6-Benzoyl-derivat, d. h. eine Verbindung der Formel Ic', worin $R_1'$ Benzoyl und n die Zahl 4 bedeuten. In ähnlicher Weise erhält man durch Reaktion einer Verbindung der Formel Ia', worin n die Zahl 3 bedeutet, mit Natriummethylsulfinylcarbanion in Dimethylsulfoxid und anschließendes Abfangen des gebildeten Pyrrolanions mit Benzylchlorid, das 6-Benzyl-derivat, d. h. eine Verbindung der Formel Ic, worin $R_1'$ Benzyl und n die Zahl 3 bedeuten.

Die N-Demethylierung einer Verbindung der Formel Ia' kann nach für die N-Dealkylierung üblichen Standardverfahren erfolgen, wie die von Braun-Methode (H. A. Hageman, Org. Reactions, 7, 198 [1953]) oder über Säuren- oder Basen-Hydrolyse eines Urethanderivats, wie die von K. C. Rice (J. Org. Chem. 40, 1850 [1975]) aufgeführten. Eine Arbeitsweise zur Dealkylierung einer Verbindung der Formel Ia' verläuft über ein Urethan der Formel XIIIa und Säurehydrolyse zum sekundären Amin der Formel Ib. Beispielsweise erhält man aus einer Verbindung der Formel Ia', worin n die Zahl 4 bedeutet, durch Erhitzen in Dioxan mit überschüssigem Äthylchloroformat und Kaliumbicarbonat unter Rückfluß während 6 Stunden eine Verbindung der Formel XIII, worin n die Zahl 4 bedeutet. Hydrolyse der vorstehend erwähnten Verbindung mit 30%iger Natronlauge in Äthanol/Dioxan während 24 Stunden bei der Rückflußtemperatur liefert eine Verbindung der Formel Ib, worin n die Zahl 4 bedeutet.

Urethan-Derivate eignen sich auch als Ausgangsprodukte für die Herstellung von am Pyrrol-Ring substituierten Derivaten durch Alkylierung oder Acylierung am Pyrrol-Stickstoff und anschließende Spaltung des Urethans. Die Alkylierung bzw. Acylierung erfolgt nach den Methoden, welche für die Herstellung der Verbindungen der Formel I'c beschrieben wurden; Urethan-Derivate und Verfahren zu deren Spaltung sind, wie oben erwähnt, von K. C. Rice (ibid) beschrieben. Beispielsweise ergibt gemäß Formelschema II Behandlung des Urethans der Formel XIIIa, worin n die Zahl 4 bedeutet, mit Natriummethylsulfinyl-carbanion in Dimethylsulfoxid und anschließende Behandlung mit Benzylchlorid die Verbindung der Formel XIIIb, worin n die Zahl 4 und $R_1'$ Benzyl bedeuten. Hydrolyse mit Natriumhydroxid ergibt das 6-Benzylderivat, d. h. eine Verbindung der Formel Ih, worin $R_1'$ Benzyl und n die Zahl 4 bedeuten. In Fällen, in welchen $R_1'$ eine niedere Alkanoyl- oder Arylcarbonylgruppe ist, welche unter rigorosen alkalischen oder stark sauren Bedingungen hydrolysiert werden könnte, kann man zur Herstellung von Verbindungen der Formel Ih, worin $R_1'$ niederes Alkanoyl oder Arylcarbonyl bedeutet, eine Gruppe wie die 2,2,2-Trichloräthoxycarbonylgruppe, welche unter milden Bedingungen mit Zink in wäßriger Essigsäure gespalten werden kann, verwenden.

Formelschema III

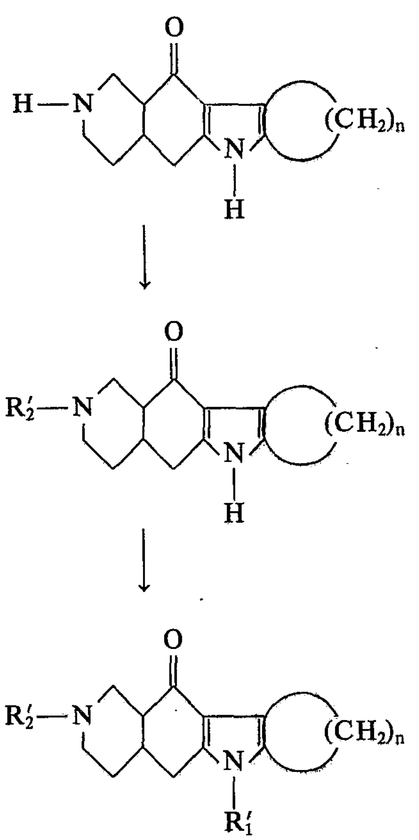

(Ib)

(Id)

(Ic)

worin

n obige Bedeutung besitzt und
$R_1'$ niederes Alkyl, niederes Alkanoyl, Arylcarbonyl oder Aryl-niederes Alkyl und
$R_2'$ niederes Alkyl, niederes Hydroxyalkyl, Aryl-niederes Hydroxyalkyl, niederes Alkoxy-niederes Alkyl, niederes Alkanoyloxy-niederes Alkyl, Arylcarbonyloxy-niederes Alkyl, niederes Alkanoyl-niederes Alkyl, Arylcarbonyl-niederes Alkyl, Aryl-niederes Alkyl, niederes Alkenyl, ($C_{3-6}$)-Cycloalkyl-niederes Alkyl, niederes Alkinyl, Thienyl-niederes Alkyl, Furyl-niederes Alkyl, Arylcarboxamido-niederes Alkyl, Aryl-niederes Alkenyl, niederes Alkenyloxy-niederes Alkyl, Aryloxy-niederes Alkyl, Aryl-niederes Alkyloxy-niederes Alkyl oder Aryl-N-imidazolonyl-niederes Alkyl bedeuten.

Gemäß Formelschema III können die Verbindungen der Formeln Id und Ic ausgehend von sekundären Aminen der Formel Ib (dem Ausgangsmaterial für die Herstellung zahlreicher von der Formel I umfaßter Derivate) durch Substitution am basischen Amin-Stickstoffatom (N-2) und/oder am Pyrrol-Stickstoffatom (N-6) hergestellt werden. Beispielsweise erhält man durch Behandeln einer Verbindung der Formel Ib mit einem Alkylhalogenid, wie Äthylbromid, einem Alkenylhalogenid, wie Allylbromid, einem Cycloalkylalkylhalogenid, wie Chlormethylcyclopropan, einem Aralkylhalogenid, wie Benzylbromid, oder einem Acylalkylhalogenid, wie γ-Chlor-p-fluorbityrophenon, in Gegenwart einer Base, wie beispielsweise Kaliumcarbonat, in Aceton, 2-Propanon oder Dimethylformamid entsprechend substituierte Verbindungen der Formel Id, worin $R_2'$ niederes Alkyl, niederes Alkenyl, ($C_{3-6}$)-Cycloalkyl-niederes Alkyl, Aryl-niederes Alkyl bzw. niederes Alkanoyl- oder Arylcarbonyl-niederes Alkyl bedeutet. Verwendet man reaktive Halogenide, so kann die Reaktion bei Raumtemperatur durchgeführt werden; verwendet man weniger reaktive Halogenide, so kann man bei der Rückflußtemperatur arbeiten. In manchen Fällen kann die Reaktionsgeschwindigkeit durch Zugabe eines Jodids, wie Lithiumjodid, zum Reaktionsgemisch gesteigert werden.

Die Reaktion einer Verbindung der Formel Ib mit Epoxyalkanen liefert die Hydroxyalkyl-substituierten Verbindungen der Formel Id. Die Behandlung mit einem substituierten Epoxyalkan liefert die in 2-Stellung substituierten 2-Hydroxyalkylanalogen einer Verbindung der Formel Ib; z. B. liefert die Reaktion einer Verbindung der Formel Ib mit Styroloxid eine Verbindung der Formel Id, worin $R_2'$ 2-Phenyl-2-hydroxyäthyl bedeutet. Die Reaktion erfolgt gewöhnlich in Gegenwart eines alkoholischen Lösungsmittels, wie Methanol, bei etwa Raumtemperatur bis zur Rückflußtemperatur des Gemisches. Die Epoxalkane sind entweder im Handel erhältlich oder werden durch Epoxydation der entsprechenden Olefine, oder durch Methylenierung eines Ketons mit einem Sulfoniummethylid oder Sulfoxoniummethylid, z. B. Dimethylsulfoniummethylid, hergestellt. So liefert z. B. die Behandlung von Benzaldehyd mit Dimethylsulfoniummethylid Styroloxid.

Enthält $R_2'$ in einer Verbindung der Formel Id keine funktionellen Gruppen, welche alkyliert bzw. acyliert werden können, so können in einigen Fällen die in Formelschema II aufgeführten Arbeitsweisen zur Herstellung von Verbindungen der Formel Ic' direkt zur Herstellung N-6-substituierter Analoga der in Formelschema III dargestellten Formel Ic verwendet werden. Alkylierungen können bei Verbindungen eintreten, worin $R_2'$ niederes Hydroxyalkyl oder Aryl-niederes Hydroxyalkyl bedeutet. Die Hydroxygruppen müssen mit einer gegenüber Basen stabilen Schutzgruppe, wie die Tetrahydropyranylgruppe, geschützt werden. Nach erfolgter N-6-Alkylierung wird die Schutzgruppe durch saure Hydrolyse abgespalten.

Andererseits können Verbindungen der Formel Id, worin $R_2'$ niederes Hydroxyalkyl oder Aryl-niederes Hydroxyalkyl bedeutet, hergestellt werden, indem man eine entsprechende Verbindung der Formel Id, worin $R_2'$ niederes Alkanoyl- oder Arylcarbonyl-niederes Alkyl bedeutet, reduziert. Im speziellen kann die vorstehend erwähnte Reduktion beispielsweise mit einem Alkalimetallborhydrid, wie Natriumborhydrid oder Lithiumborhydrid, beispielsweise bei Raumtemperatur in einem Lösungsmittel, wie z. B. einem Alkohol, wie Äthanol, oder dergleichen durchgeführt werden.

Bei den in den Formelschemata I, II und III beschriebenen Reaktionen können sowohl die trans-Isomeren der Formel

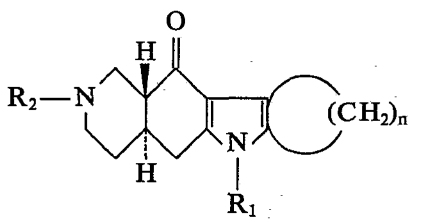

(If)

worin n, $R_1$ und $R_2$ obige Bedeutung besitzen,

als auch die cis-Isomeren der Formel

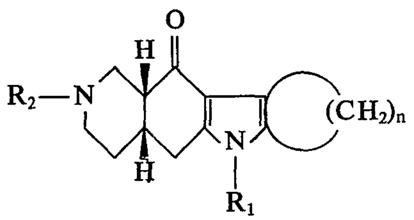

(Ig)

worin n, $R_1$ und $R_2$ obige Bedeutung besitzen,

der Verbindungen der Formel I entstehen, wobei das trans-Isomere überwiegt. Das reine trans-Isomere kann durch Chromatographie oder Kristallisation abgetrennt werden. Außerdem kann das Gemisch isomerisiert werden, wie für die Isomerisierung der trans- und cis-Isomeren der Oxoverbindungen der Formeln If' und Ig' beschrieben, oder durch Basen-katalysiertes Äquilibrieren, z. B. mit Natriumhydroxid in Äthanol.

Enthalten die Substituenten $R_1$ und $R_2$ in Verbindungen der Formeln I zusätzliche asymmetrische Zentren, so können Mischungen von Diastereomeren erhalten werden. Die Anzahl der möglichen Isomeren ist $2^n$, worin n die Anzahl der asymmetrischen Zentren in der Verbindung bedeutet. Bevorzugt werden die Enantiomeren und/oder Diastereoisomeren der Verbindungen der obigen Formel If.

Formelschema IV

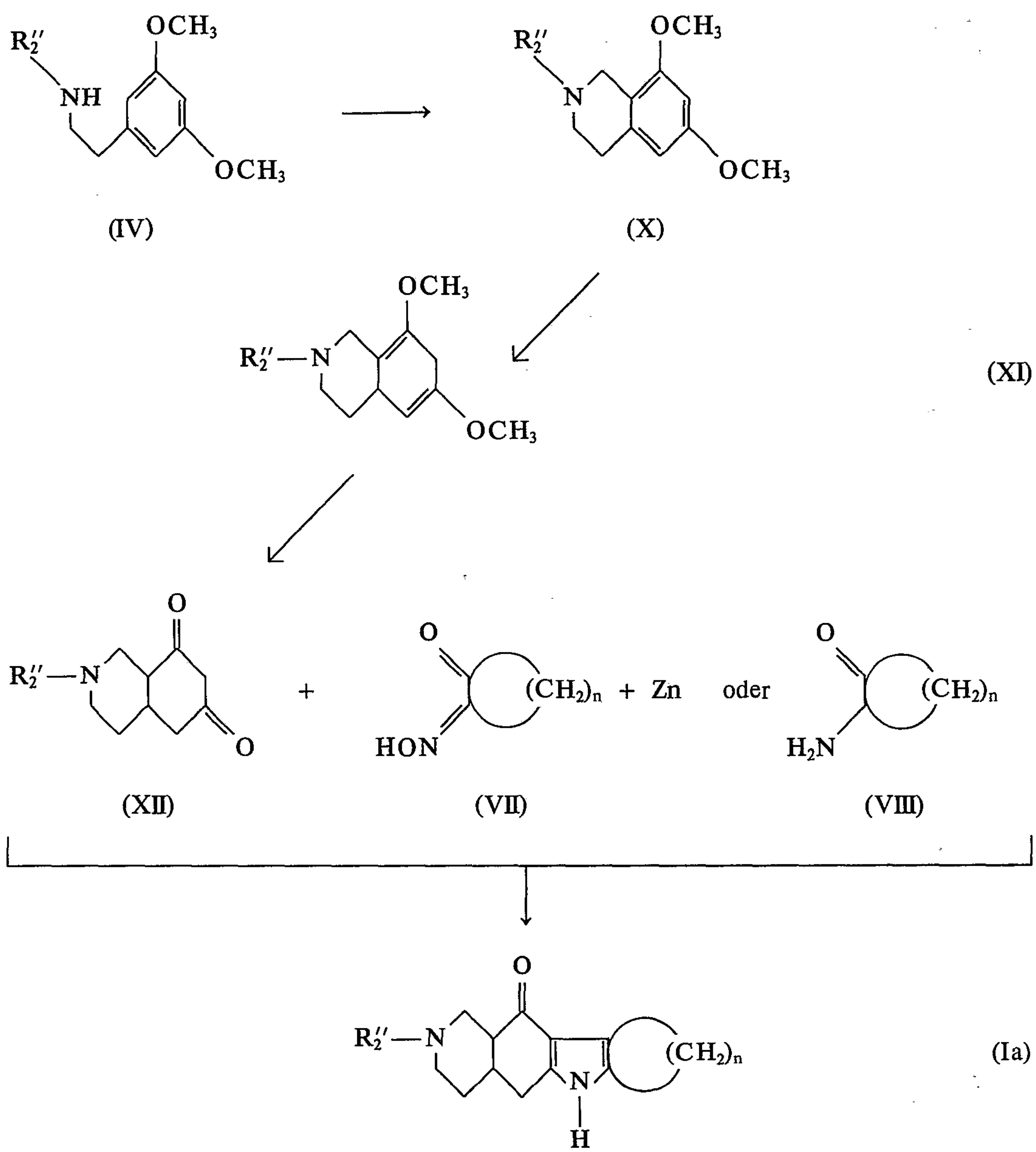

(IV) (X)

(XI)

(XII) + (VII) + Zn oder (VIII)

(Ia)

worin

n obige Bedeutung besitzt und
$R_2''$ niederes Alkyl, niederes Alkoxy-niederes Alkyl oder $(C_{3-6})$-Cycloalkyl-niederes Alkyl bedeutet.

Eine andere Synthese für die Verbindungen der Formel Ia ist in Formelschema IV beschrieben, wobei der Isochinolinring vor der Bildung des Pyrrolrings gebildet wird. Nach dem Formelschema IV wird das 3,5-Dimethoxyphenyl-äthylamin der Formel IV zusammen mit wäßrigem Formaldehyd unter Rückfluß zum Sieden erhitzt, um das Tetrahydroisochinolin der Formel X zu liefern. Die Birch-Reduktion des Tetrahydroisochinolins der Formel X mit Lithium in tert.-Butanol enthaltendem flüssigem Ammoniak unter praktisch gleichen Bedingungen, wie für die Birch-Reduktion der Verbindung der Formel IV beschrieben, liefert das Hexahydroisochinolin der Formel XI. Hydrolyse des rohen Hexahydroisochinolins der Formel XI unter praktisch gleichen Bedingungen, wie für die Hydrolyse des Dihydroamins der Formel V beschrieben, liefert das Diketon der Formel XII. Die Verbindung der Formel XII wird in einer Knorr-Kondensation, wie bei der Herstellung des Methylaminoäthylketons der Formel IX beschrieben, mit einem Isonitrosoketon der Formel VII oder mit einer Aminocarbonylverbindung der Formel VIII zu dem Cycloalka[4,5]pyrroloisochinolin der Formel Ia umgesetzt. Bevorzugt ist die Reaktionsfolge nach dem Formelschema IV ausgehend vom Amin der Formel IV, worin $R_2''$ Methyl bedeutet, was zum entsprechenden N-Methyl-cycloalka[4,5]pyrroloisochinolin der Formel Ia führt, und zwar als Gemisch des trans-Isomeren If′ und des cis-Isomeren der Formel Ig′.

Die weiter oben für die Isomerisierung von Mischungen von Cycloalka[4,5]pyrroloisochinolinen der Formeln If und Ig beschriebenen Methoden können verwendet werden, um vorwiegend das trans-Isomere einer Verbindung der Formel If′ zu erhalten.

Verbindungen der allgemeinen Formel A, worin X ein Schwefelatom bedeutet, entsprechend der allgemeinen Formel

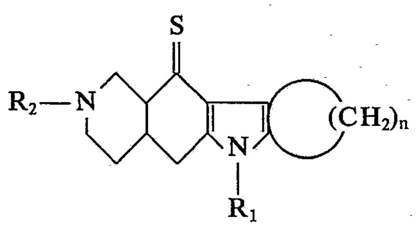

(II)

worin n, $R_1$ und $R_2$ obige Bedeutung besitzen,

und können gemäß den nachfolgenden Formelschemata V und VI und den folgenden Ausführungen hergestellt werden.

Formelschema V

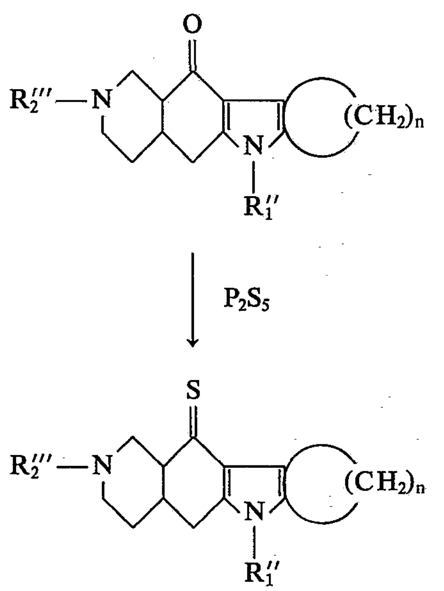


(Ie)

(IIe)

worin

n obige Bedeutung besitzt und
$R_1''$ Wasserstoff, niederes Alkyl oder Aryl-niederes Alkyl und
$R_2'''$ Wasserstoff, niederes Alkyl, niederes Alkoxy-niederes Alkyl, Aryl-niederes Alkyl, niederes Alkenyl, Aryloxy-niederes Alkyl, Thienyl-niederes Alkyl, Furyl-niederes Alkyl, niederes Alkinyl, Aryl-niederes Alkenyl, niederes Alkenyloxy niederes Alkyl, Aryl-niederes Alkyloxy-niederes Alkyl oder $(C_{3-6})$-Cycloalkyl-niederes Alkyl bedeuten.

Gemäß Formelschema V können Verbindungen der Formel IIe hergestellt werden, indem man Verbindungen der Formel Ie mit Phosphorpentasulfid in einem inerten organischen Lösungsmittel erhitzt. Bevorzugte Lösungsmittel sind Tetrahydrofuran, Benzol, Toluol oder Dioxan. Die Reaktion wird im allgemeinen bei der Rückflußtemperatur durchgeführt.

Weitere Verbindungen der Formel II können gemäß Formelschema VI hergestellt werden. Gemäß Formelschema VI kann eine Verbindung der Formel IIb zu einer Verbindung der Formel IId umgesetzt werden, und zwar in Analogie zu den Angaben in Formelschema III zur Herstellung entsprechender Oxoverbindungen der Formel Id.

Formelschema VI

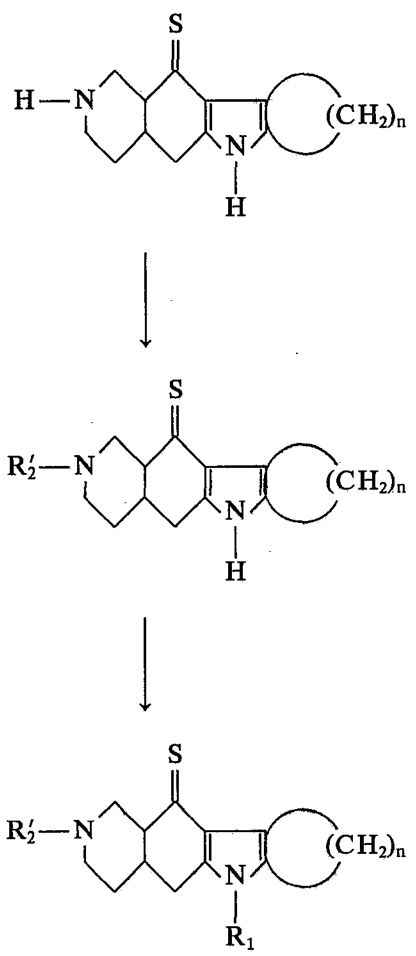

(IIb)

(IId)

(IIc)

worin n, $R_1'$ und $R_2'$ obige Bedeutung besitzen.

In Analogie zu den Angaben im Formelschema III zur Herstellung von Oxoverbindungen der Formel Ic erhält man Verbindungen der Formel IIc aus Verbindungen der Formel IId.

Weitere Verbindungen der Formel II erhält man in Analogie zu den in Formelschema II dargestellten Maßnahme zur Herstellung entsprechender Oxoverbindungen der Formel Ih.

Bei diesen Reaktionen können sowohl die trans-Isomeren der Formel

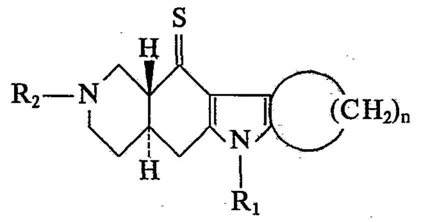

(IIf)

worin n, $R_1$ und $R_2$ obige Bedeutung besitzen,

als auch die cis-Isomeren der Formel

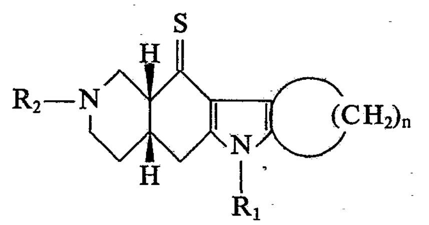

(IIg)

worin n, $R_1$ und $R_2$ obige Bedeutung besitzen,

der Verbindungen der Formel II entstehen, wobei das trans-Isomere überwiegt. Das reine trans-Isomere kann durch Chromatographie oder Kristallisation abgetrennt werden. Außerdem kann das Gemisch isomerisiert werden, wie für die Isomerisierung der trans- und cis-Isomeren der Oxoverbindungen der Formel If' und Ig' beschrieben.

Enthalten die Substituenten $R_1$ und $R_2$ in Verbindungen der Formel II, wie weiter oben für Verbindungen der Formel I beschrieben, zusätzliche asymmetrische Zentren, so erhält man eine Mischung von Diastereoisomeren. Bevorzugt werden die Enantiomeren und/oder die Diastereoisomeren von Verbindungen der obigen Formel IIf.

Die Verbindungen der Formel A bilden Säureadditionssalze mit anorganischen und organischen Säuren. So bilden sie pharmazeutisch annehmbare Säureadditionssalze sowohl mit pharmazeutisch annehmbaren organischen als auch anorganischen Säuren, z. B. mit Halogenwasserstoffsäuren, wie Salzsäure, Bromwasserstoffsäure und Jodwasserstoffsäure, mit anderen Mineralsäuren, wie Schwefelsäure, Salpetersäure, Phosphorsäure oder dergleichen, mit Alkyl- und Mono-arylsulfonsäuren, wie Äthansulfonsäure, p-Toluolsulfonsäure, Benzolsulfonsäure oder dergleichen, mit andern organischen Säuren, wie Essigsäure, Weinsäure, Maleinsäure, Citronensäure, Benzoesäure, Salicylsäure, Ascorbinsäure und dergleichen. Pharmazeutisch nicht annehmbare Säureadditionssalze von Verbindungen der Formel A können in pharmazeutisch annehmbare Säureadditionssalze über herkömmliche Austauschreaktionen überführt werden, wodurch das pharmazeutisch nicht annehmbare Anion durch ein pharmazeutisch annehmbares Anion ersetzt wird, oder andererseits durch Neutralisieren des pharmazeutisch nicht annehmbaren Säureadditionssalzes und anschließendes Umsetzen der so erhaltenen freien Base mit einem Reagens, das ein pharmazeutisch annehmbares Säureadditionssalz liefert. Die Säureadditionssalze können Hydrate bilden.

Die Verbindungen der allgemeinen Formeln IX und XIII sind neu und sind ebenfalls Gegenstand der vorliegenden Erfindung.

Die Verbindungen der Formel A und ihre pharmazeutisch annehmbaren Säureadditionssalze zeigen neuroleptische Aktivitäten. Dementsprechend sind die Verbindungen der Formel A als antipsychotische Mittel brauchbar, z. B. bei der Behandlung von Schizophrenie. Die Aktivität der Verbindungen der Formel A, die sie als antipsychotische Mittel brauchbar macht, kann nach bekannten Arbeitsweisen an Warmblütern demonstriert werden. Nach einer Methode werden z. B. trainierte Ratten in Versuchskammern gebracht, die mit einem Reaktionshebel, einem Stahldrahtboden zum Austeilen eines elektrischen Schocks und einen Lautsprecher für akustische Stimulation ausgestattet sind. Jeder Versuch besteht aus einem Warnton von 15 Sekunden (bedingter Stimulus), der für weitere 15 Sekunden seine Fortsetzung findet und dabei von einem elektrischen Schock begleitet wird (unbedingter Stimulus: 1,0 mA, 350 V Wechselspannung). Die Ratten können einen Versuch an jeder Stelle durch Niederdrücken des Reaktionshebels beenden. Eine Reaktion während des ersten 15-Sekunden-Warntons beendet den Versuch, bevor ein Schock ausgeteilt wird, und wird als Vermeidungsreaktion angesehen, während eine Reaktion, die während der Schockausteilung erfolgt, eine Fluchtreaktion ist. Die Versuche werden alle 2 Minuten während einer 1stündigen Testdauer vorgenommen (30 Versuche pro Testdauer).

Trainierte Ratten zeigen eine verläßliche Grundlinie des Vermeidungsverhaltens (0—3 ausbleibende Vermeidungsreaktionen pro Testdauer). Mindestens 3—4 Ratten erhalten zu geeigneten Vorbehandlungszeiten Verbindungen in jeder Dosierungsmenge über einen Dosierungsbereich verabreicht. Während Kontrollversuchen erhalten Ratten Trägermittel alleine. Jede Woche wechselt eine Kontroll- und eine Versuchsdauer.

Jede Versuchsdauer ist in drei aufeinanderfolgende 20-Minuten-Abschnitte (10 Versuche) unterteilt. Die Anzahl der Reaktionen wird über alle Individien bei gegebener Dosis innerhalb jedes Abschnitts summiert.

Die Anzahl der Versuche, bei denen die Ratten keine Vemeidungsreaktion (Vermeidungs-Blockierung, VB) oder keine Fluchtreaktion (Flucht-Blockierung, FB) zeigten, wird für den Abschnitt bestimmt, der bei jeder Dosis den maximalen Effekt entfaltete. Diese Zahl wird ausgedrückt als Prozentsatz der innerhalb des Abschnitts insgesamt vorgenommenen Versuche. Die für 50% Vermeidungs-Blockierung (VB 50) berechnete Dosis wird aus der Dosis/Wirkung/Regressionslinie erhalten, aufgestellt nach der Methode der kleinsten Quadrate. Die geringste Dosis, die 20% Blockierung der Fluchtreaktion (FB 20) hervorrief, wird aus einem Dosis/Wirkungs-Diagramm abgelesen. Um diese Werte zu erhalten, wird die Wirkung in Prozent gegen den log der Dosis aufgetragen.

Antipsychotische Mittel können von anderen Wirkstoffarten, die das Verhalten von Ratten bei dieser Arbeitsweise beeinträchtigen, durch die größere Trennung zwischen Dosen, die die Vermeidungsreaktion blockieren, und Dosen, die die Fluchtreaktion blockieren, unterscheiden werden. Die klinische Leistungsstärke antipsychotischer Wirkstoffe mit bekannten therapeutischen Anwendungen und Eigenschaften hängt wesentlich und stark mit ihrer Leistungsstärke bei dieser Prozedur zusammen. Folglich können die Verbindungen der Formel A therapeutisch in Dosierungen eingesetzt werden, die mit ihrer Leistungsstärke bei Testverfahren in Übereinstimmung stehen.

Wenn 2-Methyl-1,2,3,4,4a,5,7,8,9,10a-decahydro-4a,10a-trans-6H-cyclopenta[4,5]pyrrolo[2,3-g]isochinolin-10(10H)-on als Testsubstanz verwendet wird, wird eine neuroleptische Aktivität bei einer VB 50 von 0,98 mg/kg p. o. beobachtet.

Verwendet man 2-[4-(4-Fluorphenyl)-4-oxobutyl]-1,2,3,4,4a,5,7,8,9,10a-decahydro-4a,10a-trans-6H-cyclopenta[4,5]pyrrolo[2,3-g]isochinolin-10(10H)-on als Testsubstanz, so beobachtet man eine neuroleptische Aktivität bei einer VB von 0,15 mg/kg p. o.

Verwendet man 2-[4-(4-Fluorphenyl)-4-oxobutyl]-2,3,4,4a,5,7,8,9,10,11a-decahydro-4a,11a-trans-1H,6H-cyclohexa[4,5]pyrrolo[2,3-g]isochinolin-11(11a)-on als Testsubstanz, so beobachtet man eine neuroleptische Aktivität bei einer VB 50 von 0,73 mg/kg p. o.

Verwendet man 2-Methyl-2,3,4,4a,5,7,8,9,10,11a-decahydro-4a,11a-trans-1H,6H-cyclohexa-[4,5]pyrrolo[2,3-g]isochinolin-11(11H)-on-hydrochlorid · 0,75 $H_2O$, das beispielsweise eine $LD_{50}$ von 650 mg/kg p. o. in Mäusen aufweist, als Testsubstanz, so beobachtet man neuroleptische Aktivität bei einer VB 50 von 5,5 mg/kg p. o.

Verwendet man 2-Methyl-2,3,4,4a,5,7,8,9,10,11,12,13a-dodecahydro-4a,13a-trans-1H,6H-cycloocta[4,5]pyrrolo[2,3-g]isochinolin-13(13H)-on als Testsubstanz, so beobachtet man neuroleptische Aktivität bei einer Dosis von 8 mg/kg p. o., wobei die Vermeidungsblockierung 63% beträgt.

Verwendet man 2-Methyl-1,2,3,4,4a,5,7,8,9,10,11,12a-dodecahydro-4a,12a-trans-6H-cyclohepta[4,5]pyrrolo[2,3-g]isochinolin-12(12H)-on als Testsubstanz, so beobachtet man neuroleptische Aktivität bei einer Dosis von 16 mg/kg p. o., wobei die Vermeidungsblockierung 50% beträgt.

Die Verbindungen der Formel A und ihre pharmazeutisch annehmbaren Säureadditionssalze zeigen antipsychotische Wirkungen, die denen von Haloperidol und Trifluorperazin, die durch ihre therapeutische Verwendung und Eigenschaften bekannt sind, qualitativ ähneln. So zeigen die Verbindungen der Formel A ein mit antipsychotischen Wirkstoffen bekannter Wirksamkeit und Sicherheit verbundenes Aktivitätsmuster.

Die Verbindungen der Formel A und ihre pharmazeutisch annehmbaren Säureadditionssalze können in Form herkömmlicher pharmazeutischer Präparate verwendet werden. Beispielsweise enthalten geeignete orale Dosierungseinheiten Mengen im Bereich von 0,05 bis 50 mg, und geeignete orale Dosierungen bei warmblütigen Tieren liegen im Bereich von etwa 0,001 mg/kg/Tag und etwa 10 mg/kg/Tag. Für jedes bestimmte warmblütige Tier jedoch kann die spezifische Dosierung schwanken, und sie sollte nach den individuellen Bedürfnissen und dem fachkundigen Urteil der die Verabreichung einer Verbindung der Formel A oder eines pharmazeutisch annehmbaren Säureadditionssalzes hiervon vornehmenden oder übewachenden Person eingestellt werden.

Ferner schwankt die Häufigkeit, mit der jede solche Dosierungsform verabreicht wird, in Abhängigkeit von der Menge an vorhandenem aktivem Medikament und den Erfordernissen der pharmakologischen Situation.

Für die beschriebene Verwendung werden die Verbindungen der Formel A und ihre pharmazeutisch annehmbaren Säureadditionssalze unter Einsatz herkömmlicher inerter pharmazeutischer Hilfsmaterialien zu Dosierungsformen verarbeitet, die sich für orale oder parenterale Verabreichung eignen. Solche Dosierungsformen umfassen Tabletten, Suspensionen, Lösungen und dergleichen. Ferner können die Verbindungen der Formel A in geeignete harte oder weiche Kapseln eingearbeitet und in Form solcher Kapseln verabreicht werden. Die Art der inerten Hilfsmaterialien, die zur Verarbeitung der Verbindungen der Formel A und ihrer pharmazeutisch annehmbaren Säureadditionssalze zu oralen und parenteralen Dosierungsformen verwendet werden, liegen für den Fachmann sofort auf der Hand. Diese Hilfsmaterialien, entweder anorganischer oder organischer Natur, sind z. B. Wasser, Gelatine, Lactose, Stärke, Magnesiumstearat, Talk, pflanzliche Öle, Harze, Polyalkylenglykole, usw. Ferner können, wenn gewünscht, auch Konservierungsmittel, Stabilisierungsmittel, Netzmittel, Emulgiermittel, Salze zur Veränderung des osmotischen Druckes, Puffer und dergleichen in solche Rezepturen eingearbeitet werden.

Da die Verbindungen der Formel A und ihre pharmazeutisch annehmbaren Säureadditionssalze ein asymmetrisches Kohlenstoffatom besitzen, fallen sie gewöhnlich als Racemate an. Erwünschtenfalls können erhaltene Mischungen von Diastereoisomeren getrennt werden. Die Spaltung von Racematen in die optisch aktiven Formen kann nach an sich bekannten Methoden erfolgen. Optisch aktive Verbindungen können aber auch hergestellt werden, indem man in die weiter oben erwähnten Verfahren optisch aktive Ausgangsstoffe einsetzt. Manche Racemate als Eutektika ausgefällt und danach getrennt werden. Die chemische Spaltung wird jedoch bevorzugt. Danach bilden sich aus dem racemischen Gemisch Diastereoisomere mit einem optisch aktiven Spaltungsmittel, z. B. mit optisch aktiven Säuren, wie (+)-Weinsäure, (+)-Dibenzoyl-D-weinsäure, (+)-d-10-Camphersulfonsäure, (—)3-Pinancarbonsäure und dergleichen, um ein diastereoisomeres Salz zu bilden. Die gebildeten Diastereoisomeren werden durch fraktionierte Kristallisation getrennt und können in die entsprechenden optisch isomeren Basen übergeführt werden. So umfaßt die Erfindung die optisch aktiven Antipoden der Verbindung der Formel A sowie deren Racemate.

Ferner wird aufgrund der möglichen unterschiedlichen räumlichen Anordnungen ihrer Atome verständlich, daß die erfindungsgemäßen Verbindungen in mehr als einer möglichen geometrischen isomeren Form erhalten werden können. Die Verbindungen der Formel A, wie beschrieben und beansprucht, sollen alle solchen isomeren Formen umfassen. So sind die hier gegebenen Beispiele als besondere Gemische geometrischer Isomerer oder einzelner geometrischer Isomerer veranschaulichend und nicht als Begrenzung des Umfangs der Erfindung zu verstehen.

Die folgenden Beispiele veranschaulichen die Erfindung noch eingehender. Alle Temperaturen sind in °C angegeben, sofern nicht anderes erwähnt wird.

## Beispiel 1

Eine Lösung von 185,2 g N-Methyl-(3,5-dimethoxyphenyl)-äthylamin-hydrochlorid in 1600 ml Wasser wird mit 160 ml Ammoniak basisch gestellt. Die Mischung wird dreimal mit je 1000 ml Methylenchlorid ausgeschüttelt. Die vereinigten Auszüge werden mit 1000 ml halb gesättigter Kochsalzlösung gewaschen, über Natriumsulfat getrocknet und bei einer Temperatur von 35—40° eingedampft. Man erhält 156,0 g der freien Base.

In einem 12-l-3-Halskolben (versehen mit Rührer und 2 Trockeneis-Kühlern, einer mit einem Gaseinleitungsrohr versehen und der andere mit einem Natronkalk-Trockenrohr) werden 4,0 l trockenes Ammoniak kondensiert. Dazu gibt man über einen Zeitraum von 15 Minuten eine Lösung von 156,0 g der oben hergestellten freien Base in 400 ml t-Butanol und 400 ml trockenem Äther. Die erhaltene Lösung versetzt man unter Rühren über einen Zeitraum von 50 Minuten mit insgesamt 33,6 g 6,35 cm langen Lithiumdraht-Stücken. Die Zugabegeschwindigkeit wird so eingestellt, daß pro Minute 12,7 cm Draht zugegeben werden. Nach beendeter Zugabe wird die tiefblaue Mischung während 2 Stunden unter Rückfluß gerührt. Anschließend verdünnt man die Mischung mit 2,8 l trockenem Äther und entfernt das Trockenrohr, um den Wasserstoff abzulassen. Über einen Zeitraum von 30 Minuten versetzt man anschließend mit insgesamt 280 g pulverisiertem Ammoniumchlorid, bis die blaue Farbe verschwindet. Man entfernt den Trockeneiskühler und läßt das Ammoniak durch Rühren über Nacht abdampfen. Nach Versetzen des Rückstandes mit 2,8 l Eiswasser gibt man die Mischung unter Nachspülen mit 800 ml Äther in einen Scheidetrichter, und trennt die beiden Phasen. Die wäßrige Phase wird zweimal mit je 1,5 l Methylenchlorid ausgeschüttelt. Die Extrakte werden vereinigt, mit 1 l halbgesättigter Kochsalzlösung gewaschen, über Natriumsulfat getrocknet und bei 40° eingedampft. Man trocknet während 1,5 Stunden bei 40°/1,0 Torr. Man erhält 150,7 g Rohprodukt als gelbes Öl. Das rohe Öl wird über eine 30,5-cm-Goodloe-Kolonne (Bad 150°) destilliert, wobei folgende Fraktionen aufgefangen werden:

| Fraktion | Sdp. | Gewicht | Reinheit (GC) |
|---|---|---|---|
| 1 | 40—80°/0,45 mm | 7,9 g | 4,6% |
| 2 | 80—85°/0,45—0,15 mm | 6,2 g | 50% |
| 3 | 85—86°/0,15 mm | 21,2 g | 92% |
| 4 | 86—87°/0,15 mm | 99,4 g | 100% |

Die Fraktionen 3 und 4 liefern 120,6 g N-Methyl-1,5-dimethoxycyclohexa-1,4-dien-3-äthylamin als farbloses Öl.

Beispiel 2

Eine Mischung aus 15,6 g N-Methyl-1,5-dimethoxycyclohexa-1,4-dien-3-äthylamin (79 mMol), 16,7 g 2-Isonitroso-cyclohexanon (131 mMol) und 19,5 g Zinkstaub (300 mg-Atom) in 300 ml 70%iger Essigsäure wird während 5 Stunden unter Rückfluß zum Sieden erhitzt, abgekühlt und filtriert. Das Filtrat wird im Vakuum eingeengt und mit überschüssigem Dioxan versetzt. Das Dioxan/Essigsäure/Azeotrop wird abdestilliert. Diese Prozedur wird wiederholt bis die Essigsäure vollständig entfernt ist. Der Rückstand wird unter Eluieren mit 10%igem Methanol im Methylenchlorid an Aluminiumoxid III chromatographiert, wobei 10,7 g rohes 1,2,3,5,6,7,8,9-Octahydro-2-[2-(methylamino)äthyl]-4 H-carbazol-4-on anfallen. Das rohe Produkt wird in Methanol aufgenommen und mit methanolischer Salzsäure behandelt. Nach Entfernen des Lösungsmittels erhält man 12,2 g 1,2,3,5,6,7,8,9-Octahydro-2[2-(methylamino)äthyl]-4 H-carbazol-4-on-hydrochlorid.

Beispiel 3

Eine Mischung aus 2,3 g 1,2,3,5,6,7,8,9-Octahydro-2-[2-(methylamino)äthyl]-4 H-carbazol-4-on-hydrochlorid (8,14 mMol) und 2,3 g Paraformaldehyd (76 mMol) in 100 ml n-Butanol wird während 1 Stunde in einer Druckflasche in einem 180° heißen Ölbad erhitzt, wobei sich ein innerer Druck von 5,5 bar einstellt. Man läßt die erhaltene Lösung abkühlen und entfernt das Lösungsmittel unter vermindertem Druck. Der Rückstand wird in Wasser aufgelöst. Die wäßrige Lösung wird mit Methylenchlorid gewaschen, mit Ammoniak alkalisch gestellt und mit Methylenchlorid ausgeschüttelt. Die Auszüge werden über Natriumsulfat getrocknet und auf ein Volumen von 10 ml eingedampft. 10 g Aluminiumoxid werden in der Mischung aufgeschlämmt; man filtriert und dampft zur Trockene ein. Der Rückstand wird unter Eluieren mit 10%igem Methanol in Methylenchlorid an 80 g Aluminiumoxid (Aktivitätsstufe III) chromatographiert und liefert rohes 2-Methyl-2,3,4,4a,5,7,8,9,10,11a-decahydro-4a,11a-trans-1 H,6 H-cyclohexa[4,5]pyrrolo[2,3-g]isochinolin-11(11 H)-on, das wenig von entsprechendem 4a,11a-cis-Isomeren enthält. Nach Umkristallisieren des Rohproduktes aus Methanol/Methylenchlorid/Äther erhält man 2-Methyl-2,3,4,4a,5,7,8,9,10,11a-decahydro-4a,11a-trans-1 H,6 H-cyclohexa[4,5]pyrrolo[2,3-g]isochinolin-11(11 H)-on als weißlichen Festkörper. Durch Behandeln der freien Base mit Salzsäure in Methanol erhält man das entsprechende Hydrochlorid, das zweimal aus Äthanol umkristallisiert wird und 0,46 g reines 2-Methyl-2,3,4,4a,5,7,8,9,10,11a-decahydro-4a,11a-trans-1 H,6 H-cyclohexa[4,5]pyrrolo[2,3-g]isochinolin-11(11 H)-on-hydrochlorid · 0,75 $H_2O$ als Kristalle vom Schmelzpunkt 217–220° liefert.

Beispiel 4

Eine Lösung von 15,0 g (64,7 mMol) N-Methyl-(3,5-dimethoxyphenyl)äthylamin-hydrochlorid in 30 ml Wasser wird mit 35 ml 2 N-Natronlauge behandelt und mit Methylenchlorid ausgeschüttelt. Die vereinigten Extrakte werden eingeengt und mit 65 ml 37%igem wäßrigem Formaldehyd vermischt. Die erhaltene Mischung wird während 2 Stunden unter Rückfluß zum Sieden erhitzt, mit 15 ml 2 N-Natronlauge basisch gestellt und mit Methylenchlorid ausgeschüttelt. Die vereinigten Auszüge werden mit halbgesättigter Kochsalzlösung gewaschen, über Magnesiumsulfat getrocknet und eingedampft, wobei man ein gelbes Öl erhält. Das Öl wird in 100 ml Äthanol aufgenommen und mit äthanolischer Salzsäure behandelt. Nach Zugabe von 75 ml Äther kristallisiert das 3,4-Dihydro-1 H-6,8-dimethoxy-2-methylisochinolin-hydrochlorid aus.

Beispiel 5

150 ml Ammoniak werden in einem Kolben kondensiert, der 9,1 g (123 mMol) tert.-Butanol und 50 ml Diäthyläther enthält. Die erhaltene Lösung versetzt man mit 1,0 g (4,1 mMol) 3,4-Dihydro-1 H-6,8-dimethoxy-2-methylisochinolin-hydrochlorid, rührt während 2–3 Minuten und versetzt über einen Zeitraum von 30 Minuten mit insgesamt 0,57 g (82 mMol) Lithiumdraht in kleinen Stücken. Die blaue Lösung wird während 2,5 Stunden unter Rückfluß gerührt und anschließend bis zur Entfärbung mit 4,5 g Ammoniumchlorid versetzt. Nach Zugabe von 100 ml Äther läßt man das Ammoniak über Nacht abdampfen. Nach Zugabe von 100 ml Eiswasser wird die organische Phase abgetrennt, und die wäßrige Phase mit Essigester und Chloroform ausgeschüttelt. Die vereinigten organischen Auszüge werden mit halbgesättigter Kochsalzlösung gewaschen, über Magnesiumsulfat getrocknet und eingedampft. Man erhält 1,2,3,4,4a,7-Hexahydro-6,8-dimethoxy-2-methylisochinolin als gelbes Öl.

Eine Mischung aus 1,05 g des obigen Rohproduktes und 20 ml 70%iger Essigsäure wird während 5 Stunden unter Rückfluß zum Sieden erhitzt und anschließend eingedampft. Der Rückstand wird in Wasser gelöst. Die wäßrige Phase wird mit Chloroform gewaschen und auf ein Volumen von 10 ml eingedampft. Man chromatographiert unter Eluieren mit 2 M wäßrigem Pyridin an Dowex AG 50 WX 8 und

erhält Octahydro-2-methylisochinolin-6,8-dion als hellgelben Festkörper. Durch Behandeln mit Salzsäure in Methanol erhält man das entsprechende Hydrochlorid vom Schmelzpunkt 193—196°.

Beispiel 6

Eine Mischung aus 72 g (ca. 0,1 Mol) rohem etwa 30%igem Octahydro-2-methylisochinolin-6,8-dion, 21,1 g (ca. 0,1 Mol) rohem etwa 60%igem 2-Isonitrosocyclohexanon, 19,5 g (0,3 g-Atom) Zinkstaub und 500 ml 70%iger wäßriger Essigsäure wird während 1 Stunde unter Rückfluß zum Sieden erhitzt. Anschließend versetzt man mit einer zweiten Portion von 10,5 g 2-Isonitrosocyclohexanon und 6,5 g Zinkstaub und erhitzt weitere 2 Stunden unter Rückfluß zum Sieden. Die Lösung wird abgekühlt, filtriert und im Vakuum eingeengt. Der Rückstand wird in Wasser aufgenommen. Die wäßrige Lösung wird mit Chloroform gewaschen, mit Ammoniak basisch gestellt und mit Chloroform ausgeschüttelt. Die Chloroformauszüge werden mit halbgesättigter Kochsalzlösung gewaschen, über Natriumsulfat getrocknet und eingedampft. Das erhaltene Rohprodukt wird unter Eluieren mit der organischen Phase einer Mischung, erhalten durch Schütteln von 90 Teilen Chloroform, 30 Teilen Methanol, 10 Teilen Wasser und 6 Teilen Essigsäure (Volumenteile), an trockenem Kieselgel chromatographiert; dabei erhält man 2-Methyl-2,3,4,4a,5,7,8,9,10,11a-decahydro-4a,11a-trans-1H,6H-cyclohexa[4,5]pyrrolo[2,3-g]isochinolin-11(11H)-on als weißen amorphen Festkörper. Nach Aufschlämmen in heißem Äthanol erhält man ein Produkt vom Schmelzpunkt 273—275° (Zersetzung).

Beispiel 7

In Analogie zu den Angaben in Beispiel 6 erhält man aus 2-Isonitrosocyclopentanon und Octahydro-2-methylisochinolin-6,8-dion 2-Methyl-1,2,3,4,4a,5,7,8,9,10a-decahydro-4a,10a-trans-6H-cyclopenta[4,5]pyrrolo[2,3-g]isochinolin-10(10H)-on das nach Kristallisieren aus Äthanol/Methanol einem Schmelzpunkt von 296—297° (Zersetzung) aufweist. Das entsprechende Hydrochlorid kristallisiert aus Wasser als Hemihydrat und hat einen Schmelzpunkt von 256—258° (Zersetzung).

Beispiel 8

In Analogie zu den Angaben in Beispiel 6 erhält man aus 2-Isonitrosocycloheptanon und Octahydro-2-methylisochinolin-6,8-dion 2-Methyl-1,2,3,4,4a,5,7,8,9,10,11,12a-dodecahydro-4a,12a-trans-6H-cyclohepta[4,5]pyrrolo[2,3-g]isochinolin-12(12H)-on das nach Umkristallisieren aus Äthanol einen Schmelzpunkt von 293—296° aufweist.

Beispiel 9

In Analogie zu den Angaben in Beispiel 6 erhält man aus 2-Isonitrosocyclooctanon und Octahydro-2-methylisochinolin-6,8-dion das 2-Methyl-2,3,4,4a,5,7,8,9,10,11,12,13a-dodecahydro-4a,13a-trans-1H,6H-cycloocta[4,5]pyrrolo[2,3-g]isochinolin-13(13H)-on, das nach Kristallisieren aus Wasser/Dimethylformamid einen Schmelzpunkt von 298—300° aufweist.

Beispiel 10

Eine Mischung aus 1,9 g 2-Methyl-2,3,4,4a,5,7,8,9,10,11a-decahydro-4a,11a-trans-1H,6H-cyclohexa[4,5]pyrrolo[2,3-g]isochinolin-11(11H)-on, 2,6 g Äthylchloroformat, 3,2 g Kaliumcarbonat und 100 ml Dioxan wird während 6 Stunden unter Rückfluß zum Sieden erhitzt, anschließend abgekühlt und filtriert. Nach Eindampfen des Filtrates unter vermindertem Druck wird der Rückstand in Chloroform aufgenommen. Diese Lösung wird mit 5%iger wäßriger Salzsäure ausgeschüttelt, mit Wasser und halbgesättigter Kochsalzlösung gewaschen und über Natriumsulfat getrocknet. Nach Entfernen des Lösungsmittels erhält man rohes Carbamat, d. h. 2-Äthoxycarbonyl-2,3,4,4a,5,7,8,9,10,11a-decahydro-4a,11a-trans-1H,6H-cyclohexa[4,5]pyrrolo[2,3-g]isochinolin-11(11H)-on. Durch Behandeln der wäßrigen Auszüge mit Ammoniak und Ausschütteln mit Chloroform können noch 0,4 g des Ausgangsmaterials zurückgewonnen werden.

1,4 g des rohen Carbamates werden zusammen mit 15 ml 30%iger wäßriger Natronlauge in einer Mischung aus 15 ml Äthanol und 5 ml Dioxan während 24 Stunden unter Rückfluß zum Sieden erhitzt. Nach Einengen der Mischung im Vakuum wird der Rückstand in 5%iger wäßriger Salzsäure aufgelöst und mit Chloroform gewaschen. Die wäßrige Phase wird mit Ammoniak basisch gestellt und mit Chloroform ausgeschüttelt. Die Auszüge werden mit halbgesättigter Kochsalzlösung gewaschen, getrocknet und eingedampft. Man erhält 2,3,4,4a,5,7,8,9,10,11a-Decahydro-4a,11a-trans-1H-6H-cyclohe-

xa[4,5]pyrrolo[2,3-g]isochinolin-11(11 H)-on.

Beispiel 11

In Analogie zu den Angaben in Beispiel 10 erhält man aus 2-Methyl-1,2,3,4,4a,5,7,8,9,10a-decahydro-4a,10a-trans-6 H-cyclopenta[4,5]pyrrolo[2,3-g]isochinolin-10(10 H)-on über das entsprechende Carbamat 1,2,3,4,4a,5,7,8,9,10a-Decahydro-4a,10a-thrans-6 H-cyclopenta[4,5]pyrrolo[2,3-g]isochinolin-10(10 H)-on das nach Kristallisieren aus Äthanol/Äthylacetat einen Schmelzpunkt von 242—245° (Zersetzung) aufweist.

Beispiel 12

In Analogie zu den Angaben in Beispiel 10 erhält man aus 2-Methyl-2,3,4,4a,5,7,8,9,10,11,12,13a-dodecahydro-4a,13a-trans-1 H,6 H-cycloocta[4,5]pyrrolo[2,3-g]isochinolin-13(13 H)-on über das entsprechende Carbamat 1,2,3,4,4a,5,6,7,8,9,10,11,12,13a-Tetradecahydro-4a,13a-trans-cycloocta[4,5]pyrrolo[2,3-g]isochinolin-4-on, das nach Kristallisieren aus Äthanol einen Schmelzpunkt von 283—285° aufweist.

Beispiel 13

Eine Mischung aus 0,68 g 2,3,4,4a,5,7,8,9,10,11a-Decahydro-4a,11a-trans-1 H,6 H-cyclohexa[4,5]pyrrolo[2,3-g]isochinolin-11(11 H)-on, 1,68 g γ-Chlor-p-fluorbutyrophenon, 1,55 g Kaliumcarbonat und 15 ml Diäthylketon wird während 24 Stunden unter Rückfluß zum Sieden erhitzt. Die Mischung wird abgekühlt, filtriert und eingeengt. Der Rückstand wird an einer trockenen Kolonne unter Eluieren mit der organischen Phase eines Gemisches, hergestellt durch Schütteln von 90 Teilen Chloroform, 30 Teilen Methanol, 10 Teilen Wasser und 6 Teilen Essigsäure (Volumenteile), chromatographiert, wobei rohes 2-[4-(4-Fluorphenyl)-4-oxobutyl]-2,3,4,4a,5,7,8,9,10,11a-decahydro-4a,11a-trans-1 H,6 H-cyclohexa[4,5]pyrrolo[2,3-g]isochinolin-11(11 H)-on anfällt. Nach Umkristallisieren aus Äthanol erhält man reines Material als kristallinen Festkörper vom Schmelzpunkt 220—222°.

Beispiel 14

In Analogie zu den Angaben in Beispiel 13 erhält man durch Alkylieren von 2,3,4,4a,5,7,8,9,10,11a-Decahydro-4a,11a-trans-1 H,6 H-cyclohexa[4,5]pyrrolo[2,3-g]isochinolin-11(11 H)-on mit (2-Bromäthyl)benzol und Umkristallisieren des erhaltenen Stoffes aus Äthanol 2-(2-Phenyläthyl)-2,3,4,4a,5,7,8,9,10,11a-decahydro-4,11a-trans-1 H,6 H-cyclohexa[4,5]pyrrolo[2,3-g]isochinolin-11(11 H)-on als kristallinen Festkörper vom Schmelzpunkt 250—256° (Zersetzung).

Beispiel 15

In Analogie zu den Angaben in Beispiel 13 erhält man durch Alkylieren von 2,3,4,4a,5,7,8,9,10,11a-Decahydro-4a,11a-trans-1 H,6 H-cyclohexa[4,5]pyrrolo[2,3-g]isochinolin-11(11 H)-on mit Benzylchlorid und Umkristallisieren des erhaltenen Stoffes aus Äthanol 2-Benzyl-2,3,4,4a,5,7,8,9,10,11a-decahydro-4a,11a-trans-1 H,6 H-cyclohexa[4,5]pyrrolo[2,3-g]isochinolin-11(11 H)-on als kristallinen Festkörper vom Schmelzpunkt 266—268°.

Beispiel 16

In Analogie zu den Angaben in Beispiel 13 erhält man durch Alkylieren von 1,2,3,4,4a,5,7,8,9,10a-Decahydro-4a,10a-trans-6 H-cyclopenta[4,5]pyrrolo[2,3-g]isochinolin-10(10 H)-on mit Benzylchlorid und Umkristallisieren des erhaltenen Stoffes aus Äthanol 2-Benzyl,1,2,3,4,4a,5,7,8,9,10a-decahydro-4a,10a-trans-6 H-cyclopenta[4,5]pyrrolo[2,3-g]isochinolin-10(10 H)-on als kristallinen Festkörper vom Schmelzpunkt 258—260° (Zersetzung).

Beispiel 17

In Analogie zu den Angaben in Beispiel 13 erhält man durch Alkylieren von 1,2,3,4,4a,5,7,8,9,10a-Decahydro-4a,10a-trans-6 H-cyclopenta[4,5]pyrrolo[2,3-g]isochinolin-10(10 H)-on mit γ-Chlor-p-fluorbutyrophenon und Umkristallisieren des erhaltenen Stoffes aus Äthanol 2-[4-(4-Fluorphenyl)-4-oxobutyl]-1,2,3,4,4a,5,7,8,9,10a-decahydro-4a,10a-trans-6H-cyclopenta[4,5]pyrrolo[2,3-g]isochinolin-10(10H)-on als kristallinen Festkörper vom Schmelzpunkt 225—227°.

## Beispiel 18

In Analogie zu den Angaben in Beispiel 13 erhält man durch Alkylieren von 1,2,3,4,4a,5,7,8,9,10a-Decahydro-4a,10a-trans-6H-cyclopenta[4,5]pyrrolo[2,3-g]isochinolin-10(10H)-on mit (2-Bromäthyl)benzol und Umkristallisieren des erhaltenen Stoffes aus Äthanol 2-(2-Phenyläthyl)-1,2,3,4,4a,5,7,8,9,10a-decahydro-4a,10a-trans-6H-cyclopenta[4,5]pyrrolo[2,3-g]isochinolin-10(10H)-on als kristallinen Festkörper vom Schmelzpunkt 251—254° (Zersetzung).

## Beispiel 19

In Analogie zu den Angaben in Beispiel 13 erhält man durch Alkylieren von 1,2,3,4,4a,5,7,8,9,10a-Decahydro-4a,10a-trans-6H-cyclopenta[4,5]pyrrolo[2,3-g]isochinolin-10(10H)-on mit 4-Methoxybenzylchlorid und Umkristallisieren des erhaltenen Stoffes aus Äthanol 2-(4-Methoxybenzyl)-1,2,3,4,4a,5,7,8,9,10a-decahydro-4a,10a-trans-6H-cyclopenta[4,5]pyrrolo[2,3-g]isochinolin-4-on als kristallinen Festkörper vom Schmelzpunkt 236—238° (Zersetzung).

## Beispiel 20

In Analogie zu den Angaben in Beispiel 13 erhält man durch Alkylieren von 1,2,3,4,4a,5,7,8,9,10a-Decahydro-4a,10a-trans-6H-cyclopenta[4,5]pyrrolo[2,3-g]isochinolin-10(10H)-on mit 4-Chlorbenzylchlorid und Umkristallisieren des erhaltenen Stoffes aus Äthanol 2-(4-Chlorbenzyl)-1,2,3,4,4a,5,7,8,9,10a-decahydro-4a,10a-trans-6H-cyclopenta[4,5]pyrrolo[2,3-g]isochinolin-10(10H)-on als kristallines Monohydrat vom Schmelpunkt 254—256°.

## Beispiel 21

In Analogie zu den Angaben in Beispiel 13 erhält man durch Alkylieren von 1,2,3,4,4a,5,7,8,9,10a-Decahydro-4a,10a-trans-6H-cyclopenta[4,5]pyrrolo[2,3-g]isochinolin-10(10H)-on mit Allylbromid und Umkristallisieren des erhaltenen Stoffes aus Essigester/Äthanol 2-(2-Propenyl)-1,2,3,4,4a,5,7,8,9,10a-decahydro-4a,10a-trans-6H-cyclopenta[4,5]pyrrolo[2,3-g]isochinolin-10(10H)-on vom Schmelzpunkt 257—259° (Zersetzung).

## Beispiel 22

In Analogie zu den Angaben in Beispiel 13 erhält man durch Alkylieren von 1,2,3,4,4a,5,7,8,9,10a-Decahydro-4a,10a-trans-6H-cyclopenta[4,5]pyrrolo[2,3-g]isochinolin-10(10H)-on mit 2-Bromäthyläther und Umkristallisieren des erhaltenen Stoffes aus Äthanol 2-(2-Äthoxyäthyl)-1,2,3,4,4a,5,7,8,9,10a-decahydro-4a,10a-trans-6H-cyclopenta[4,5]pyrrolo[2,3-g]isochinolin-10(10H)-on als kristallinen Festkörper vom Schmelzpunkt 236—238° (Zersetzung).

## Beispiel 23

In Analogie zu den Angaben in Beispiel 13 erhält man durch Alkylieren von 2,3,4,4a,5,7,8,9,10,11,12,13a-Dodecahydro-4a,13a-trans-1H,6H-cycloocta[4,5]pyrrolo[2,3-g]-g]isochinolin-13(13H)-on mit Gamma-chlor-p-fluorbutyrophenonäthylenketal und saure Hydrolyse des Ketals im erhaltenen Stoff 2-[4-(4-Fluorphenyl)-4-oxobutyl]-1,2,3,4,4a,5,7,8,9,10,11,12,13a-tetradecahydro-4a,13a-trans-cycloocta[4,5]pyrrolo[2,3-g]isochinolin-13(13H)-on.

## Beispiel 24

In Analogie zu den Angaben in Beispiel 13 können die in der nachfolgenden Tabelle I aufgeführten Verbindungen ausgehend vom entsprechenden Cycloalka[4,5]pyrrolo[2,3-g]isochinolin und dem entsprechenden Halogenid hergestellt werden.

Tabelle 1

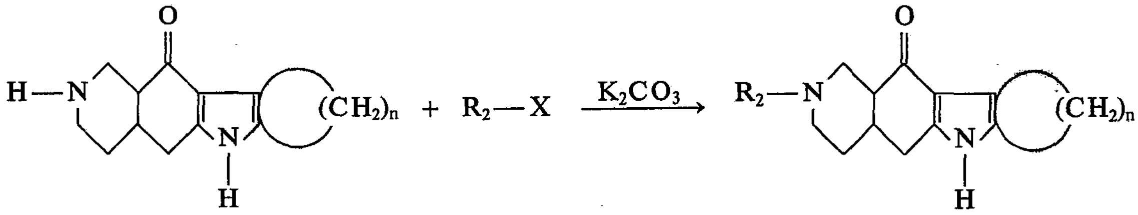


| Bei-spiel | Name | n | $R_2$ | X |
|---|---|---|---|---|
| 24 | 2-Äthyl-1,2,3,4,4a,5,7,8,9,10a,10a-decahydro-4a,10a-trans-6H-cyclopenta[4,5]pyrrolo[2,3-g]isochinolin-10(10H)-on | | $CH_3CH_2—$ | Br |
| 25 | 2-(2-Acetoxyäthyl)-1,2,3,4,4a,5,7,8,9,10a-decahydro-4a,10a-trans-6H-cyclopenta[4,5]pyrrolo-[2,3-g]isochinolin-10(10H)-on | 3 | 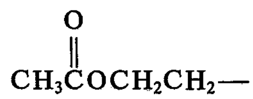 | Br |
| 26 | 2-[3-(4-Fluorphenyl)-3-oxopropyl]-1,2,3,4,4a,5,7,8,9,10a-decahydro-4a,10a-trans-6H-cyclopenta[4,5]pyrrolo[2,3-g]isochinolin-10(10H)-on | 3 | 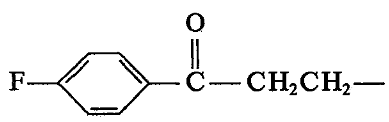 | Cl |
| 27 | 2-Cyclopropylmethyl-1,2,3,4,4a,5,7,8,9,10a-decahydro-4a,10a-trans-6H-cyclopenta[4,5]pyrrolo-[2,3-g]isochinolin-10(10H)-on | 3 | 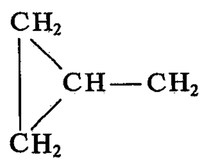 | Cl |
| 28 | 2-Propargyl-1,2,3,4,4a,5,7,8,9,10a-decahydro-4a,10a-trans-6H-cyclopenta[4,5]pyrrolo-[2,3-g]isochinolin-10(10H)-on | 3 | $HC \equiv C—CH_2—$ | Br |
| 29 | 2[2-(2-Thienyl)äthyl]-1,2,3,4,4a,5,7,8,9,10a-decahydro-4a,10a-trans-6H-cyclopenta[4,5]pyrrolo-[2,3-g]isochinolin-10(10H)-on | 3 | 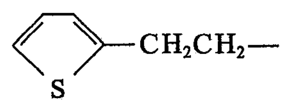 | Br |
| 30 | 2-[2-(2-Furyl)äthyl]-1,2,3,4,4a,5,7,8,9,10a-decahydro-4a,10a-trans-6H-cyclopenta[4,5]pyrrolo-[2,3-g]isochinolin-10(10H)-on | 3 | 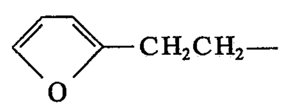 | Br |
| 31 | 2-[2-(2,3-Dihydro-2-oxo-1H-benzimidazol-1-yl)-äthyl-1,2,3,4,4a,5,7,8,9,10a-decahydro-4a,10a-trans-6H-cyclopenta[4,5]pyrrolo[2,3-g]isochinolin chinolin-10(10H)-on | 3 | 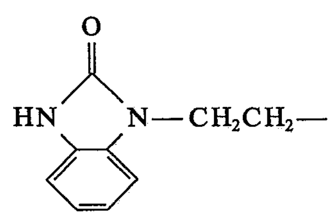 | Br |

Fortsetzung

| Beispiel | Name | n | $R_2$ | X |
|---|---|---|---|---|
| 32 | 2-[2-(Benzyloxy)äthyl]-1,2,3,4,4a,5,7,8,9,10a-decahydro-4a,10a-trans-6H-cyclopenta[4,5]pyrrolo-[2,3-g]isochinolin-10(10H)-on | 3 | 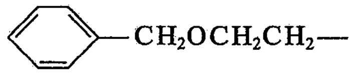 | Br |
| 33 | 2-(3-Phenyl-2-propenyl)-1,2,3,4,4a,5,7,8,9,10a-decahydro-4a,10a-trans-6H-cyclopenta[4,5]-pyrrolo[2,3-g]isochinolin-10(10H)-on | 3 | 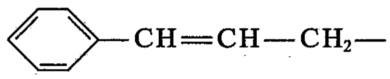 | Br |
| 34 | 2-[2-Äthenyloxy)äthyl]-1,2,3,4,4a,5,7,8,9,10a-decahydro-4a,10a-trans-6H-cyclopenta[4,5]pyrrolo-[2,3-g]isochinolin-10(10H)-on | 3 | $CH_2{=}CH{-}O{-}CH_2CH_2{-}$ | Br |
| 35 | 2-(2-Äthoxyäthyl)-2,3,4,4a,5,7,8,9,10,11a-decahydro-4a,11a-trans-1H,6H-cyclohexa[4,5]-pyrrolo[2,3-g]isochinolin-11(11H)-on | 4 | $CH_3CH_2OCH_2CH_2{-}$ | Br |
| 36 | 2-Cyclobutylmethyl-2,3,4,4a,5,7,8,9,10,11a-decahydro-4a,11a-trans-1H,6H-cyclohexa[4,5]pyrrolo-[2,3-g]isochinolin-11(11H)-on | 4 | 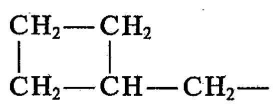 | Br |
| 37 | 2-[2-(2-Thienyl)äthyl]-2,3,4,4a,5,7,8,9,10,11a-decahydro-4a,11a-trans-1H,6H-cyclohexa[4,5]pyrrolo-[2,3-g]isochinolin-11(11H)-on | 4 | 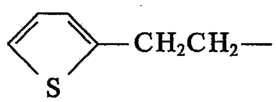 | Br |
| 38 | 2-[2-(2-Furyl)äthyl]-2,3,4,4a,5,7,8,9,10,11-decahydro-4a,11a-trans-1H,6H-cyclohexa[4,5]pyrrolo-[2,3-g]isochinolin-11(11H)-on | 4 | 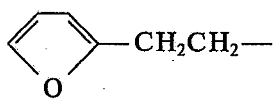 | Br |
| 39 | 2-[3-(4-Fluorphenyl)-3-oxopropyl]-2,3,4,4a,5,7,8,9,10,11a-decahydro-4a,11a-trans-1H,6H-cyclohexa[4,5]pyrrolo[2,3-g]isochinolin-11(11H)-on | 4 | 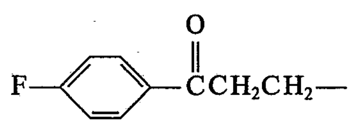 | Cl |
| 40 | 2-(2-Propenyl)-2,3,4,4a,5,7,8,9,10,11a-decahydro-4a,11a-trans-1H,6H-cyclohexa[4,5]pyrrolo-[2,3-g]isochinolin-11(11H)-on | 4 | $CH_2{=}CH{-}CH_2{-}$ | Br |
| 41 | 2-(3-Phenoxypropyl)-2,3,4,4a,5,7,8,9,10,11a-decahydro-4a,11a-trans-1H,6H-cyclohexa[4,5]pyrrolo-[2,3-g]isochinolin-11(11H)-on | 4 | 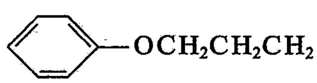 | Br |
| 42 | 2-[3-(2,3-Dihydro-2-oxo-1H-benzimidazol-1-yl)-propyl]-2,3,4,4a,5,7,8,9,10,11a-decahydro-4a,11a-trans-1H,6H-cyclohexa[4,5]pyrrolo[2,3-g]iso-chinolin-11(11H)-on | 4 | 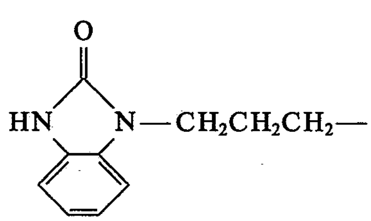 | Br |

Fortsetzung

| Beispiel | Name | n | $R_2$ | X |
|---|---|---|---|---|
| 43 | 2-[4-(4-Fluorphenyl)-4-oxobutyl]-1,2,3,4,4a,5,7,8,9,10,11,12a-dodecahydro-4a,12a-trans-6H-cyclohepta[4,5]pyrrolo[2,3-g]isochinolin-12(12H)-on | 5 | 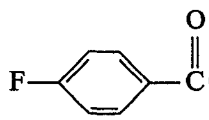 $CH_2CH_2CH_2$— | Cl |
| 44 | 2-(3-Phenoxypropyl)-1,2,3,4,4a,5,7,8,9,10,11,12a-dodecahydro-4a,12a-trans-6H-cyclohepta-[4,5]pyrrolo[2,3-g]isochinolin-12(12H)-on | 5 | 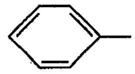 —$OCH_2CH_2CH_2$— | Br |
| 45 | 2-(2-Phenyläthyl)-1,2,3,4,4a,5,7,8,9,10,11,12a-dodecahydro-4a,12a-trans-6H-cyclohepta-[4,5]pyrrolo[2,3-g]isochinolin-12(12H)-on | 5 | 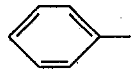 —$CH_2CH_2$— | Br |
| 46 | 2-(2-Phenyläthyl)-2,3,4,4a,5,7,8,9,10,11,12,13a-dodecahydro-4a,13a-trans-1H,6H-cycloocta-[4,5]pyrrolo[2,3-g]isochinolin-13(13H)-on | 6 | 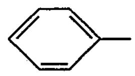 —$CH_2CH_2$— | Br |
| 47 | 2-(2-Äthoxyäthyl)-2,3,4,4a,5,7,8,9,10,11,12,13a-dodecahydro-4a,13a-trans-1H,6H-cycloocta-[4,5]pyrrolo[2,3-g]isochinolin-13(13H)-on | 6 | $CH_3CH_2OCH_2CH_2$— | Br |
| 48 | 2-(3-Phenoxypropyl)-2,3,4,4a,5,7,8,9,10,11,12,13a-dodecahydro-4a,13a-trans-1H,6H-cycloocta-[4,5]pyrrolo[2,3-g]isochinolin-13(13H)-on | 6 | 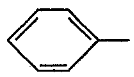 —$OCH_2CH_2CH_2$— | Br |

## Beispiel 49

Eine Mischung aus 244 mg (1,0 mMol) 2-Methyl-1,2,3,4,4a,5,7,8,9,10a-decahydro-4a,10a-trans-6H-cyclopenta[4,5]pyrrolo[2,3-g]isochinolin-10(10H)-on, 222 ml (1,0 mMol) Phosphorpentasulfid und 15 ml Dioxan wird unter Rühren während 17 Stunden unter Rückfluß zum Sieden erhitzt. Nach Abdekantieren der Lösung vom Festkörper wird der Rückstand mit 20 ml Wasser und mit soviel Ammoniak versetzt, daß das pH 8—9 beträgt. Nach Ausschütteln der Mischung mit Chloroform werden die Auszüge mit halbgesättigter Kochsalzlösung gewaschen, getrocknet und eingedampft. Nach Reinigung des rohen Thions durch Chromatographie (wie in Beispiel 13 beschrieben) und Umkristallisieren des erhaltenen reinen Festkörpers aus Acetonitril erhält man 2-Methyl-1,2,3,4,4a,5,7,8,9,10a-decahydro-4a,10a-trans-6H-cyclopenta[4,5]pyrrolo[2,3-g]isochinolin-10(10H)-thion vom Schmelzpunkt 224—227° (Zersetzung).

## Beispiel 50

In Analogie zu den Angaben in Beispiel 49 erhält man 2-Methyl-2,3,4,4a,5,7,8,9,10,11a-decahydro-4a,11a-trans-1H,6H-cyclohexa[4,5]pyrrolo[2,3-g]isochinolin-11(11H)-thion ausgehend von der entsprechenden Oxoverbindung.

## Beispiel 51

In Analogie zu den Angaben in Beispiel 49 erhält man ausgehend von der entsprechenden Oxoverbindung 1,2,3,4,4a,5,7,8,9,10a-decahydro-4a,10a-trans-6H-cyclopenta[4,5]pyrrolo[2,3-g]isochinolin-10(10H)-thion.

Beispiel 52

In Analogie zu den Angaben in Beispiel 13 erhält man aus 1,2,3,4,4a,5,7,8,9,10a-Decahydro-4a,10a-trans-6H-cyclopenta[4,5]pyrrolo[2,3-g]isochinolin-10(10H)-thion und γ-Chlor-p-fluorbutyrophenon 2-[4-(4-Fluorphenyl-4-oxobutyl]-1,2,3,4,4a,5,7,8,9,10a-decahydro-4a,10a-trans-6H-cyclopenta[4,5]pyrrolo[2,3-g]isochinolin-10(10H)-thion.

Beispiel 53

In Analogie zu den Angaben in Beispiel 13 erhält man aus 1,2,3,4,4a,5,7,8,9,10a-Decahydro-4a,10a-trans-6H-cyclopenta[4,5]pyrrolo[2,3-g]isochinolin-10(10H)-thion und (2-Bromäthyl)benzol 2-(2-Pehnyläthyl)-1,2,3,4,4a,5,7,8,9,10a-decahydro-4a,10a-trans-6H-cyclopenta[4,5]pyrrolo-2,3-g]isochinolin-10(10H)-thion.

Beispiel 54

Zu einer Mischung aus 244 mg (1,0 mMol) 2-Methyl-1,2,3,4,4a,5,7,8,9,10a-decahydro-4a,10a-trans-6H-cyclopenta[4,5]pyrrolo[2,3-g]isochinolin-10(10H)-on in 10 ml trockenem Tetrahydrofuran gibt man über einen Zeitraum von 2—3 Minuten bei —30° mit einer Spritze 0,5 ml (1,1 mMol) n-Butyllithium (2,2 M Lösung in Hexan) hinzu. Die Lösung wird während 30 Minuten bei —30° gerührt und anschließend über einen Zeitraum von 2—3 Minuten mit 168 mg (1,2 mMol) Benzoylchlorid versetzt. Die Lösung wird anschließend noch während 1 Stunde bei —30° und 30 Minuten bei Raumtemperatur gerührt. Man gießt auf Eis und extrahiert mit Chloroform. Die Auszüge werden mit halbgesättigter Kochsalzlösung gewaschen, über Natriumsulfat getrocknet und eingedampft. Der Rückstand wird chromatographiert und liefert 6-Benzoyl-2-methyl-1,2,3,4,4a,5,7,8,9,10a-decahydro-4a,10a-trans-6H-cyclopenta[4,5]pyrrolo[2,3-g]isochinolin-10(10H)-on.

Beispiel 55

In Analogie zu den Angaben in Beispiel 54 erhält man, bei Verwendung von Methyliodid anstelle von Benzoylchlorid, 2,6-Dimethyl-1,2,3,4,4a,5,7,8,9,10a-decahydro-4a,10a-trans-6H-cyclopenta[4,5]-pyrrolo[2,3-g]isochinolin-10(10H)-on.

Beispiel 56

Eine Lösung von 488 mg (2,0 mMol) 2,3,4,4a,5,7,8,9,10,11a-Decahydro-4a,11a-trans-1H,6H-cyclohexa[4,5]pyrrolo›2,3-g]isochinolin-11(11H)-on und 300 mg (3,0 mMol) 3,3-Dimethyl-1,2-epoxybutan in 15 ml Methanol wird während 24 Stunden unter Rückfluß zum Sieden erhitzt und anschließend eingedampft. Nach Chromatographieren und Umkristallisieren des erhaltenen Rohproduktes aus Äthanol erhält man 2-(2-Hydroxy-3,3-dimethylbutyl)-2,3,4,4a,5,7,8,9,10,11a-decahydro-4a,11a-1H,6H-cyclohexa[4,5]pyrrolo[2,3-g]isochinolin-11(11H)-on vom Schmelzpunkt 276—278° (Zersetzung).

Beispiel 57

In Analogie zu den Angaben in Beispiel 56 erhält man aus 1,2,3,4,4a,5,7,8,9,10a-Decahydro-4a,10a-trans-6H-cyclopenta[4,5]pyrrolo[2,3-g]isochinolin-10(10H)-on und Styroloxid 2-(2-Hydroxy-2-phenyläthyl)-1,2,3,4,4a,5,7,8,9,10a-decahydro-4a,10a-trans-6H-cyclopenta[4,5]pyrrolo-[2,3-g]isochinolin-10(10H)-on.

Beispiel 58

In Analogie zu den Angaben in Beispiel 56 erhält man durch Alkylieren von 1,2,3,4,4a,5,7,8,9,10a-Decahydro-4a,10a-trans-6H-cyclopenta[4,5]pyrrolo[2,3-g]isochinolin-10(10H)-on mit Äthylenoxid bei Raumtemperatur und Umkristallisieren des erhaltenen Stoffes aus Äthanol 2-(2-Hydroxyäthyl)-1,2,3,4,4a,5,7,8,9,10a-decahydro-4a,10a-trans-6H-cyclopenta[4,5]pyrrolo[2,3-g]isochinolin-10(10H)-on als kristallinen Festkörper vom Schmelzpunkt 237—239° (Zersetzung).

Beispiel 59

In Analogie zu den Angaben in Beispiel 56 erhält man durch Alkylieren von 1,2,3,4,4a,5,7,8,9,10a-Decahydro-4a,10a-trans-6H-cyclopenta[4,5]pyrrolo[2,3-g]isochinolin-10(10H)-on mit 3,3-Dimethyl-1,2-epoxybutan und Umkristallisieren des erhaltenen Stoffes als Äthanol 2-(2-Hydroxy-3,3-dimethylbutyl)-1,2,3,4,4a,5,7,8,9,10a-decahydro-4a,10a-trans-6H-cyclopenta[4,5]pyrrolo[2,3-g]isochinolin-10(10H)-on als kristallinen Festkörper vom Schmelzpunkt 284—286° (Zersetzung).

Beispiel 60

30 mg Natriumhydrid (57%ige Öldispersion) werden vom Öl befreit und zusammen mit 2 ml trockenem Dimethylsulfoxid während 1,5 Stunden auf 65—70° erwärmt. Die erhaltene Lösung wird abgekühlt und portionenweise mit einer Lösung von 244 mg 2-Methyl-1,2,3,4,4a,5,7,8,9,10a-decahydro-4a,10a-trans-6H-cyclopenta[4,5]pyrrolo[2,3-g]isochinolin-10(10H)-on in 1 ml trockenem Dimethylsulfoxid versetzt. Die erhaltene Mischung wird während 2 Stunden bei Raumtemperatur gerührt, mit einer Lösung von 160 mg Benzylchlorid in 1 ml trockenem Dimethylsulfoxid versetzt, während weiteren 2,5 Stunden bei Raumtemperatur gerührt und anschließend auf Eiswasser gegossen. Nach Ausschütteln der Mischung mit Chloroform werden die organischen Auszüge mit gesättigter Kochsalzlösung gewaschen, getrocknet und eingedampft. Der rohe Festkörper wird an Kieselgel unter Eluieren mit dem in Beispiel 62 angegebenen Lösungsmittelgemisch chromatographiert. Nach Umkristallisieren des erhaltenen Stoffes aus Essigester/Äthanol erhält man 6-Benzyl-2-methyl-1,2,3,4,4a,5,7,8,9,10a-decahydro-4a,10a-trans-6H-cyclopenta[4,5]pyrrolo[2,3-g]isochinolin-10(10H)-on als kristallinen Festkörper vom Schmelzpunkt 169—171°.

Beispiel 61

Eine Lösung von 2,35 g rac.-2-Methyl-1,2,3,4,4a,5,7,8,9,10a-decahydro-4a,10a-trans-6H-cyclopenta[4,5]pyrrolo[2,3-g]isochinolin-10(10H)-on in 20 ml Methanol wird mit einer Lösung von 3,62 g (+)-Dibenzoyl-(D)-Weinsäure-monohydrat in 20 ml Methanol versetzt. Nach Eindampfen der erhaltenen Mischung wird der Rückstand dreimal aus Methanol umkristallisiert und mit Ammoniak in die freie Base übergeführt. Das Umkristallisieren der freien Base aus Äthanol erhält man (—)-2-Methyl-1,2,3,4,4a,5,7,8,9,10a-decahydro-4a,10a-trans-6H-cyclopenta[4,5]pyrrolo[2,3-g]isochinolin-10(10H)-on vom Schmelzpunkt 270—272° (Zersetzung). Das entsprechende Hydrochlorid weist in Methanol (1%ig) einen $[\alpha]_D^{25}$ —101,17° auf.

Beispiel 62

Eine Mischung aus 394 mg (1,0 mMol) 2-[4-(4-Fluorphenyl)-4-oxobutyl]-1,2,3,4,4a,5,7,8,9,10a-decahydro-6H-4a,10a-trans-cyclopenta[4,5]pyrrolo[2,3-g]isochinolin-10(10H)-on, 151 mg (4,0 mMol) Natriumborhydrid und 15 ml Äthanol wird während 24 Stunden bei Raumtemperatur gerührt, mit einer zweiten Portion von 150 ml (4,0 mMol) Natriumborhydrid versetzt und während weiteren 24 Stunden bei Raumtemperatur gerührt. Die Mischung wird auf 50 ml Wasser gegossen. Der erhaltene weiße Festkörper, der noch Ausgangsmaterial enthält, wird abfiltriert und an einer trockenen Kieselgelkolonne unter Eluieren mit der unteren Phase einer Mischung, bestehend aus 90 ml Chloroform, 30 ml Methanol, 10 ml Wasser und 6 ml Essigsäure, chromatographiert. Der erhaltene Festkörper wird aus wäßrigem Dimethylformamid umkristallisiert und liefert 2-[4-(4-Fluorphenyl)-4-hydroxybutyl]-1,2,3,4,4a,5,7,8,9,10a-decahydro-6H-4a,10a-trans-cyclopenta[4,5]pyrrolo[2,3-g]isochinolin-10(10H)-on vom Schmelzpunkt 143—245° (Mischung von Diastereoisomeren).

Beispiel 63

In Analogie zu den Angaben in Beispiel 62 erhält man durch Reduktion von 2-[4-(4-Fluorphenyl)-4-oxobutyl]-2,3,4,4a,5,7,8,9,10,11a-decahydro-4a,11a-trans-1H,6H-cyclohexa[4,5]pyrrolo-[2,3-g]isochinolin-11(11H)-on mit Natriumborhydrid und Kristallisieren des erhaltenen Stoffes aus 1,4-Dioxan 2-[4-(4-Fluorphenyl)-4-hydroxybutyl]-2,3,4,4a,5,7,8,9,10,11a-decahydro-4a,11a-trans-1H,6H-cyclohexa[4,5]pyrrolo[2,3-g]isochinolin-11(11H)-on als Mischung von Diastereoisomeren vom Schmelzpunkt 239—241°.

Beispiel 64

In Analogie zu den Angaben in Beispiel 62 erhält man durch Reduktion von 2-[4-(4-Fluorphenyl)-4-oxobutyl]-2,3,4,4a,5,7,8,9,10,11,12,13a-dodecahydro-4a,13a-trans-1H,6H-cycloocta[4,5]pyrrolo[2,3-g]isochinolin-13(13H)-on mit Natriumborhydrid und Kristallisieren des erhaltenen Stoffes aus 1,4-Dioxan 2-[4-(4-Fluorphenyl)-4-hydroxybutyl]-2,3,4,4a,5,7,8,9,10,11,12,13a-dodecahydro-4a,13a-trans-1H,6H-cycloocta[4,5]pyrrolo[2,3-g]isochinolin-13(13H)-on vom Schmelzpunkt 249—251° als Mischung von Diastereoisomeren.

Beispiel 65

Eine Lösung von 5,5 g (27,9 mMol) N-Methyl-1,5-dimethoxycyclohexa-1,4-dien-3-äthylamin in 20 ml Tetrahydrofuran wird unter Rühren in einer Portion mit 10 ml 6-N-Salzsäure versetzt. Die warme Lösung wird während 15 Minuten auf 50° erwärmt und anschließend zu einem hellgelben Öl eingedampft. Das rohe Öl wird in 25 ml Wasser aufgelöst. Die erhaltene Lösung wird in einer Glasfritte mit 50 g Dowex 50 × 8 Harz vermischt (vorher mit 2-N-Salzsäure und deionisiertem Wasser gewaschen). Nach einigen Minuten wird die wäßrige Lösung unter Nachspülen mit vier 50-ml-Portionen Wasser und mit acht 35-ml-Potionen 2 M wäßrigem Pyridin abgesaugt. Die Pyridinfraktionen 3—8 werden vereinigt und eingedampft. Man erhält 5-[(2-Methylamino)äthyl]-cyclohexan-1,3-dion. Eine aus Wasser kristallisierte analytische Probe hat einen Schmelzpunkt 171—174°.

Beispiel 66

In Analogie zu den Angaben in Beispiel 60 erhält man durch Alkylierung von 316 mg 2-Äthoxycarbonyl-2,3,4,4a,5,7,8,9,10,11a-decahydro-4a,11a-trans-1H,6H-cyclohexa[4,5]pyrrolo[2,3-g]isochinolin-11(11H)-on mit 190 mg Benzylchlorid 6-Benzyl-2-äthoxycarbonyl-2,3,4,4a,5,7,8,9,10,11a-decahydro-4a,11a-trans-1H,6H-cyclohexa[4,5]pyrrolo[2,3-g]isochinolin-11(11H)-on vom Schmelzpunkt 54—60°; durch Hydrolyse mittels Natriumhydroxid in Analogie zu den Angaben in Beispiel 10 erhält man 6-Benzyl-2,3,4,4a,5,7,8,9,10,11a-decahydro-4a,11a-trans-1H,6H-cyclohexa[4,5]pyrrolo[2,3-g]isochinolin-11(11H)-on.

Beispiel A

Herstellung von Kapseln

| Bestandteile | mg/Kapsel 0,1 | 0,5 | 5,0 | 10,0 | 25,0 |
|---|---|---|---|---|---|
| 2-[4-(4-Fluorphenyl)-4-oxobutyl]-1,2,3,4,4a,5,7,8,9,10a-decahydro-4a,10a-trans-6H-cyclopenta[4,5]pyrrolo[2,3-g]isochinolin-10(10H)-on | 0,1 | 0,5 | 5,0 | 10,0 | 25,0 |
| Lactose | 183,9 | 183,5 | 179,0 | 218,0 | 257,0 |
| Stärke | 30,0 | 30,0 | 30,0 | 50,0 | 70,0 |
| Talk | 5,0 | 5,0 | 5,0 | 10,0 | 15,0 |
| Magnesiumstearat | 1,0 | 1,0 | 1,0 | 2,0 | 3,0 |
| Kapselfüllgewicht | 220,0 | 220,0 | 220,0 | 290,0 | 370,0 |

Verfahren:

2-[4-(4-Fluorphenyl)-4-oxobutyl]-1,2,3,4,4a,5,7,8,9,10a-decahydro-4a,10a-trans-6H-cyclopenta-[4,5]pyrrolo[2,3-g]isochinolin-10(10H)-on, Lactose und Stärke werden in einem geeigneten Mixer vermischt, in einer geeigneten Mühle vermahlen, mit dem Talk und dem Magnesiumstearat vermischt und in Kapseln abgefüllt.

Beispiel B

Herstellung von Tabletten (direkte Verpressung)

| Bestandteile | mg/Tablette | | | | |
|---|---|---|---|---|---|
| | 0,1 | 0,5 | 5,0 | 10,0 | 25,0 |
| 2-[4-(4-Fluorphenyl)-4-oxobutyl]-1,2,3,4,4a,5,7,8,9,10a-decahydro-4a,10a-trans-6H-cyclopenta[4,5]pyrrolo[2,3-g]isochinolin-10(10H)-on | 0,1 | 0,5 | 5,0 | 10,0 | 25,0 |
| Lactose | 85,4 | 85,5 | 81,0 | 103,0 | 112,5 |
| Avicel | 30,0 | 30,0 | 30,0 | 45,0 | 60,0 |
| Modifizierte Stärke | 8 | 7,5 | 7,5 | 10,0 | 15,0 |
| Magnesiumstearat | 1,5 | 1,5 | 1,5 | 2,0 | 2,5 |
| Tablettengewicht | 125,0 | 125,0 | 125,0 | 170,0 | 215,0 |

Verfahren:

2-[4-(4-Fluorphenyl)-4-oxobutyl]-1,2,3,4,4a,5,7,8,9,10a-decahydro-4a,10a-trans-6H-cyclopenta-[4,5]pyrrolo[2,3-g]isochinolin-10(10H)-on, Lactose, Avicel und modifizierte Stärke werden während 10 bis 15 Minuten in einem geeigneten Mixer vermischt, mit vorgemischtem Magnesiumstearat während 4 Minuten vermischt und auf einer geeigneten Presse verpreßt.

Beispiel C

Herstellung von Tabletten (Naßgranulierung)

| Bestandteile | mg/Tablette | | | | |
|---|---|---|---|---|---|
| | 0,1 | 0,5 | 5,0 | 10,0 | 25,0 |
| 2-[4-(4-Fluorphenyl)-4-oxobutyl]-1,2,3,4,4a,5,7,8,9,10a-decahydro-4a,10a-trans-6H-cyclopenta[4,5]pyrrolo[2,3-g]isochinolin-10(10H)-on | 0,1 | 0,5 | 5,0 | 10,0 | 25,0 |
| Lactose | 103,9 | 103,5 | 99,5 | 148,0 | 197,0 |
| Modifizierte Stärke | 10,0 | 10,0 | 10,0 | 20,0 | 30,0 |
| Vorgelatinisierte Stärke | 10,0 | 10,0 | 10,0 | 20,0 | 30,0 |
| Magnesiumstearat | 1,0 | 1,0 | 1,0 | 2,0 | 3,0 |
| Tablettengewicht | 125,0 | 125,0 | 125,0 | 200,0 | 285,0 |

Verfahren:

2-[4-(4-Fluorphenyl)-4-oxobutyl]-1,2,3,4,4a,5,7,8,9,10a-decahydro-4a,10a-trans-6H-cyclopenta-[4,5]pyrrolo[2,3-g]isochinolin-10(10H)-on, Lactose, modifizierte Stärke und vorgelatinisierte Stärke werden in einem geeigneten Mixer vermischt, mit Wasser granuliert, getrocknet, gemahlen, mit dem Magnesiumstearat vermischt und auf einer geeigneten Presse verpreßt.

**Patentansprüche für die Vertragsstaaten: BE, CH, DE, FR, GB, IT, LI, LU, NL, SE**

1. Cycloalka[4,5]pyrrolo[2,3-g]isochinoline der allgemeinen Formel

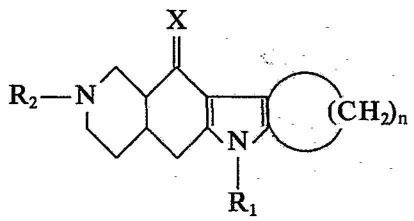

(A)

worin

$R_1$ Wasserstoff, niederes Alkyl, niederes Alkanoyl, Arylcarbonyl oder Aryl-niederes Alkyl,
$R_2$ Wasserstoff, niederes Alkyl, niederes Hydroxyalkyl, Aryl-niederes Hydroxyalkyl, niederes Alkoxy-niederes Alkyl, niederes Alkanoyloxy-niederes Alkyl, Arylcarbonyloxy-niederes Alkyl, niederes Alkanoyl-niederes Alkyl, Arylcarbonyl-niederes Alkyl, Aryl-niederes Alkyl, niederes Alkenyl, $(C_{3-6})$-Cycloalkyl-niederes Alkyl, niederes Alkinyl, Thienyl-niederes Alkyl, Furyl-niederes Alkyl, Arylcarboxamido-niederes Alkyl, Aryl-niederes Alkenyl, niederes Alkenyloxy-niederes Alkyl, Aryloxy-niederes Alkyl, Aryl-niederes Alkyloxy-niederes Alkyl oder Aryl-N-imidazolonyl-niederes Alkyl,
X ein Sauerstoff- oder Schwefelatom und
n die Zahl 3, 4, 5 oder 6 bedeuten, wobei die mit nieder bezeichneten Reste bis zu 7 Kohlenstoffatome enthalten und Aryl Phenyl oder durch Halogen, Trifluormethyl, niederes Alkyl, niederes Alkoxy, Nitro, Amino, niederes Alkylamino oder di(niederes Alkyl)amino substituiertes Phenyl bedeutet,

optische und geometrische Isomeren dieser Verbindungen und pharmazeutisch annehmbare Säureadditionssalze davon.

2. Verbindungen gemäß Anspruch 1, dadurch gekennzeichnet, daß $R_1$ Wasserstoff und $R_2$ niederes Alkyl, niederes Hydroxyalkyl, Aryl-niederes Hydroxyalkyl, niederes Alkoxy-niederes Alkyl, Aryloxy-niederes Alkyl, niederes Alkanoyl-niederes Alkyl, Arylcarbonyl-niederes Alkyl oder Aryl-niederes Alkyl bedeuten.

3. Verbindungen gemäß Anspruch 2, dadurch gekennzeichnet, daß n die Zahl 3 oder 4 und X ein Sauerstoffatom bedeuten.

4. Verbindungen gemäß einem der Ansprüche 1—3, dadurch gekennzeichnet, daß sie die tran-Konfiguration aufweisen.

5. 2 - Methyl - 1,2,3,4,4a,5,7,8,9,10a - decahydro - 4a,10a - trans - 6H - cyclopenta[4,5]pyrrolo-[2,3-g]isochinolin-10(10H)-on.

6. 2 - Methyl - 1,2,3,4,4a,5,7,8,9,10a - decahydro - 4a,10a - trans - 6H - cyclopenta[4,5]pyrrolo-[2,3-g]isochinolin-10(10H)-on-hydrochlorid · 0,5 $H_2O$.

7. 2-[4-(4-Fluorphenyl)-4-oxobutyl]-1,2,3,4,4a,5,7,8,9,10a-decahydro-4a,10a-trans-6H-cyclopenta[4,5]pyrrolo[2,3-g]isochinolin-10(10H)-on.

8. 2 - (2 - Phenyläthyl) - 1,2,3,4,4a,5,7,8,9,10a - decahydro - 4a,10a - trans - 6H - cyclopenta[4,5]-pyrrolo[2,3-g]isochinolin-10(10H)-on.

9. (—) - 2 - Methyl - 1,2,3,4,4a,5,7,8,9,10a - decahydro - 4a,10a - trans - 6H - cyclopenta[4,5]pyrrolo-[2,3-g]isochinolin-10(10H)-on.

10. 2-Methyl-2,3,4,4a,5,7,8,9,10,11a-decahydro-4a,11a-trans-1H,6H-cyclohexa[4,5]pyrrolo-[2,3-g]isochinolin-11(11H)-on.

11. 2-Methyl-2,3,4,4a,5,7,8,9,10,11a-decahydro-4a,11a-trans-1H,6H-cyclohexa[4,5]pyrrolo-[2,3-g]isochinolin-11(11H)-on · 0,75 $H_2O$.

12. 2-[4-(4-Fluorphenyl)-4-oxobutyl]-2,3,4,4a,5,7,8,9,10,11a-decahydro-4a,11a-trans-1H,6H-cyclohexa[4,5]pyrrolo[2,3-g]isochinolin-11(11H)-on.

13. 2 - (2 - Phenyläthyl) - 2,3,4,4a,5,7,8,9,10,11a - decahydro - 4a,11a - trans - 1H,6H - cyclohexa-[4,5]pyrrolo[2,3-g]isochinolin-11(11H)-on.

14. 2 - Methyl - 1,2,3,4,4a,5,7,8,9,10a - decahydro - 4a,10a - trans - 6H - cyclopenta[4,5]pyrrolo-[2,3-g]isochinolin-10(10H)-thion.

15. Verbindungen der allgemeinen Formel

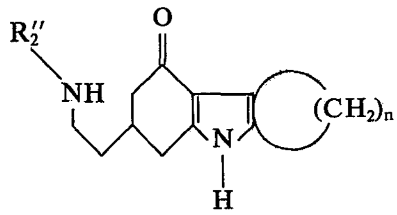

(IX)

worin

$R_2''$ niederes Alkyl, niederes Alkoxy-niederes Alkyl oder ($C_{3-6}$)-Cycloalkyl-niederes Alkyl und
n die Zahl 3, 4, 5 oder 6 bedeuten, wobei die mit nieder bezeichneten Reste bis zu 7 Kohlenstoffatome enthalten.

16. Verbindungen gemäß Anspruch 15, dadurch gekennzeichnet, daß $R_2''$ Methyl bedeutet.
17. Verbindungen der allgemeinen Formel

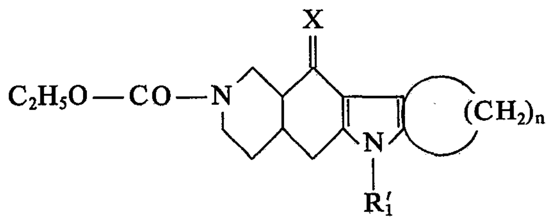

(XIII)

worin

X ein Sauerstoff- oder Schwefelatom,
n die Zahl 3,4,5 oder 6, und
$R_1$ Wasserstoff, niederes Alkyl, niederes Alkanoyl, Arylcarbonyl oder Aryl-niederes Alkyl bedeuten, wobei die mit nieder bezeichneten Reste bis zu 7 Kohlenstoffatome enthalten und Aryl Phenyl oder durch Halogen, Trifluormethyl, niederes Alkyl, niederes Alkoxy, Nitro, Amino, niederes Alkylamino oder di(niederes Alkyl)amino substituiertes Phenyl bedeutet.

18. Verbindungen gemäß einem der Ansprüche 1—14 als pharmazeutische Wirkstoffe.
19. Verbindungen gemäß einem der Ansprüche 1—14 als antipsychotische Wirkstoffe.
20. Verfahren zur Herstellung von Verbindungen gemäß einem der Ansprüche 1—14, dadurch gekennzeichnet, daß man

a) zur Herstellung einer Verbindung der allgemeinen Formel

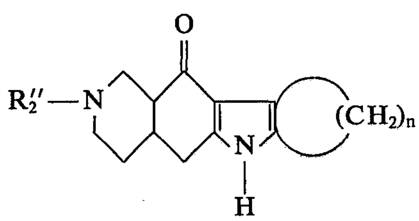

(Ia)

worin

$R_2''$ niederes Alkyl, niederes Alkoxy-niederes Alkyl oder ($C_{3-6}$)-Cycloalkyl-niederes Alkyl bedeutet und
n obige Bedeutung besitzt,

eine Verbindung der allgemeinen Formel

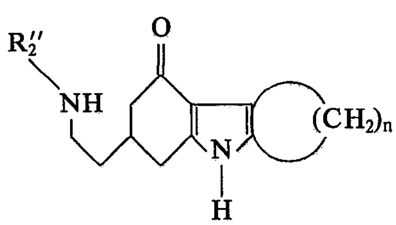

(IX)

worin $R_2''$ und n obige Bedeutung besitzen,

mit Formaldehyd behandelt, oder

b) zur Herstellung einer Verbindung der obigen allgemeinen Formel Ia, eine Verbindung der allgemeinen Formel

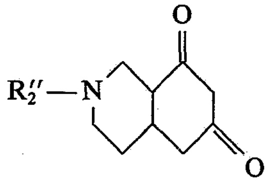

(XII)

worin $R_2''$ obige Bedeutung besitzt,

mit einer Verbindung der allgemeinen Formel

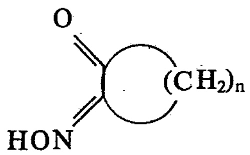

(VII)

in Gegenwart eines Reduktionsmittels oder mit einer Verbindung der allgemeinen Formel

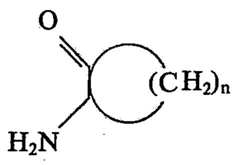

(VIII)

worin n obige Bedeutung besitzt,

oder einer Vorstufe von dieser, behandelt, oder

c) zur Herstellung einer Verbindung der allgemeinen Formel

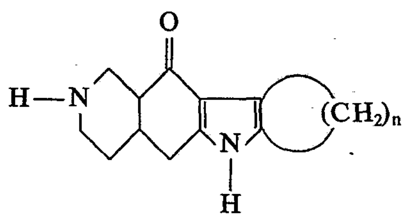

(Ib)

worin n obige Bedeutung besitzt,

eine Verbindung der obigen allgemeinen Formel Ia, worin $R_2''$ bedeutet, N-demethyliert, oder

d) zur Herstellung einer Verbindung der allgemeinen Formel

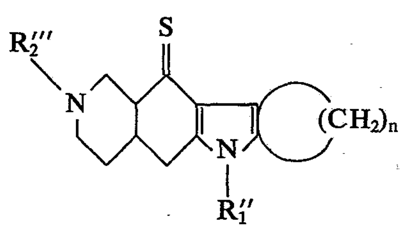

(IIe)

worin

$R_1''$ Wasserstoff, niederes Alkyl oder Aryl-niederes Alkyl und
$R_2'''$ Wasserstoff, niederes Alkyl, niederes Alkoxy-niederes Alkyl, Aryl-niederes Alkyl, niederes Alkenyl, $(C_{3-6})$-Cycloalkyl-niederes Alkyl, niederes Alkinyl, Thienyl-niederes Alkyl, Furyl-niederes Alkyl, niederes Alkenyloxy-niederes Alkyl, Aryl-niederes Alkenyl, Aryloxy-niederes Alkyl

oder Aryl-niederes Alkyloxy-niederes Alkyl bedeuten und
n obige Bedeutung besitzt,

eine Verbindung der allgemeinen Formel

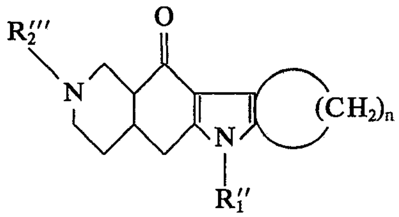

(Ie)

worin $R_1''$, $R_2'''$ und n obige Bedeutung besitzen,

mit Phosphorpentasulfid behandelt, oder

e) zur Herstellung einer Verbindung der allgemeinen Formel

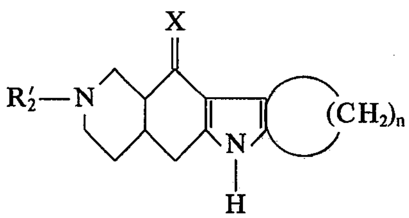

(Ad)

worin

$R_2'$ niederes Alkyl, niederes Hydroxyalkyl, Aryl-niederes Hydroxyalkyl, niederes Alkoxy-niederes Alkyl, niederes Alkanoyloxy-niederes Alkyl, Arylcarbonyloxy-niederes Alkyl, niederes Alkanoyl-niederes Alkyl, Arylcarbonyl-niederes Alkyl, Aryl-niederes Alkyl, niederes Alkenyl, ($C_{3-6}$)-Cycloalkyl-niederes Alkyl, niederes Alkinyl, Thienyl-niederes Alkyl, Furyl-niederes Alkyl, Arylcarboxamido-niederes Alkyl, Aryl-niederes Alkenyl, niederes Alkenyloxy-niederes Alkyl, Aryloxy-niederes Alkyl, Aryl-niederes Alkyloxy-niederes Alkyl oder Aryl-N-imidazolonyl-niederes Alkyl

bedeutet und

X und n obige Bedeutung besitzen,

eine Verbindung der allgemeinen Formel

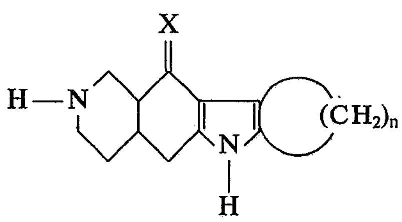

(Ab)

worin X und n obige Bedeutung besitzen,

am Isochinolin-Stickstoffatom substituiert, oder

f) zur Herstellung einer Verbindung der allgemeinen Formel

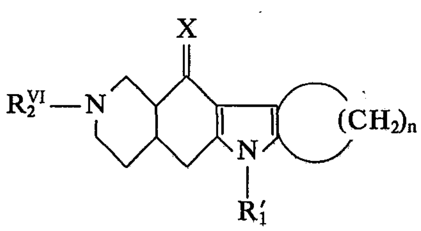

(A'c)

worin

$R_1'$ niederes Alkyl, niederes Alkanoyl, Arylcarbonyl oder Aryl-niederes Alkyl und
$R_2^{VI}$ niederes Alkyl, niederes Alkoxy-niederes Alkyl, niederes Alkanoyloxy-niederes Alkyl, Arylcarbonyloxy-niederes Alkyl, niederes Alkanoyl-niederes Alkyl, Arylcarbonyl-niederes Alkyl, Aryl-niederes Alkyl, niederes Alkenyl, $(C_{3-6})$-Cycloalkyl-niederes Alkyl, Thienyl-niederes Alkyl, niederes Alkinyl, Furyl-niederes Alkyl, Arylcarboxamido-niederes Alkyl, Aryl-niederes Alkenyl, niederes Alkenyloxy-niederes Alkyl, Aryloxy-niederes Alkyl, Aryl-niederes Alkyloxy-niederes Alkyl oder Aryl-N-imidazolonyl-niederes Alkyl bedeuten und
X und n obige Bedeutung besitzen,

eine Verbindung der allgemeinen Formel

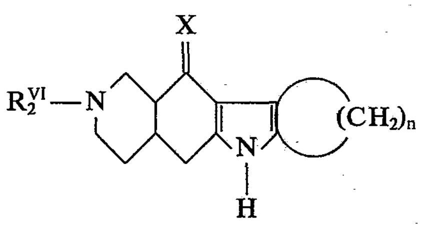

(Ad')

worin $R_2^{VI}$, X und n obige Bedeutung besitzen,

am Pyrrol-Stickstoffatom substituiert, oder

g) zur Herstellung einer Verbindung der allgemeinen Formel

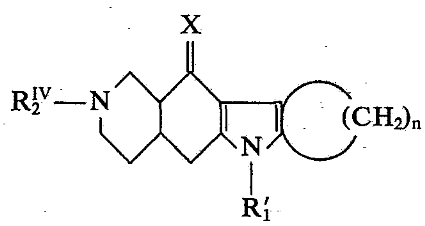

(Ac")

worin

$R_2^{VI}$ niederes Hydroxyalkyl oder Aryl-niederes Hydroxyalkyl bedeuten und
$R_1'$, X und n obige Bedeutung besitzen,

in einer Verbindung der allgemeinen Formel

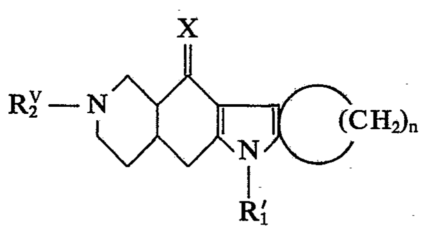

(XIV)

worin

$R_2^{V}$ eine an der Hydroxygruppe geschützte niedere Hydroxyalkyl- oder Aryl-niedere Hydroxyalkyl-Gruppe bedeutet und
$R_1'$, X und n obige Bedeutung besitzen,

die Schutzgruppe abspaltet, oder

h) zur Herstellung einer Verbindung der allgemeinen Formel

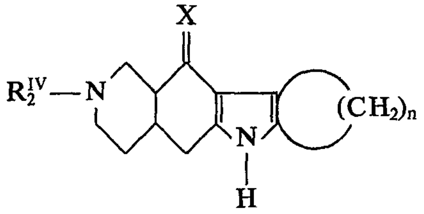

(Ad'')

worin $R_2^{IV}$, X und n obige Bedeutung besitzen,

in einer Verbindung der allgemeinen Formel

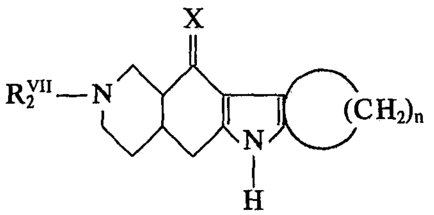

(Ad''')

worin

$R_2^{VII}$ niederes Alkanoyl-niederes Alkyl oder Arylcarbonyl-niederes Alkyl bedeutet und
X und n obige Bedeutung besitzen,

die niedere Alkanoyl-niedere Alkyl- oder Arylcarbonyl-niedere Alkyl-Gruppe reduziert, oder

i) zur Herstellung einer Verbindung der allgemeinen Formel

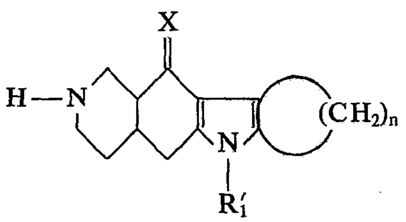

(Ah)

worin X, n und $R_1'$ obige Bedeutung besitzen,

in einer Verbindung der allgemeinen Formel

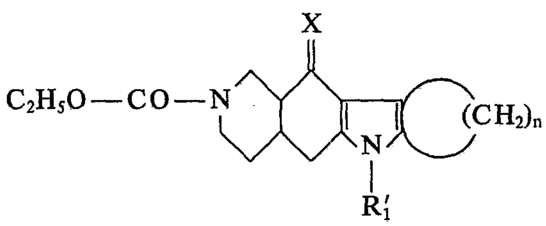

(XIII)

worin X, n und $R_1'$ obige Bedeutung besitzen,

die Äthoxycarbonylgruppe abspaltet, und

j) erwünschtenfalls ein erhaltenes Gemisch der cis- und trans-Isomeren zu einem Endverhältnis, das überwiegend das trans-Isomere aufweist, isomerisiert, und/oder
k) erwünschtenfalls das trans-Isomere aus dem erhaltenen Gemisch abtrennt, und/oder
l) erwünschtenfalls ein erhaltenes Racemat in die optischen Antipoden spaltet, und/oder
m) erwünschtenfalls eine erhaltene Verbindung oder ein pharmazeutisch nicht annehmbares Säureadditionssalz in ein pharmazeutisch annehmbares Säureadditionssalz überführt.

21. Verfahren gemäß Anspruch 20, dadurch gekennzeichnet, daß man nach den Verfahrensvarianten a), b), c), d), e), f), g), j), k), l) und m) verfährt.

22. Verfahren gemäß Anspruch 20, dadurch gekennzeichnet, daß man nach Verfahrensvariante h) verfährt.

23. Arzneimittel, enthaltend eine Verbindung gemäß einem der Ansprüche 1–14.

24. Neuroleptische/antipsychotische Mittel, enthaltend eine Verbindung gemäß einem der Ansprüche 1–14.

**Patentansprüche für den Vertragsstaat: AT**

1. Verfahren zur Herstellung von Cycloalka[4,5]pyrrolo[2,3-g]isochinolinen der allgemeinen Formel

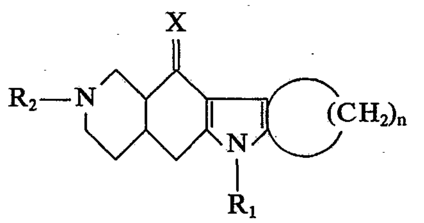

(A)

worin

$R_1$ Wasserstoff, niederes Alkyl, niederes Alkanoyl, Arylcarbonyl oder Aryl-niederes Alkyl,
$R_2$ Wasserstoff, niederes Alkyl, niederes Hydroxyalkyl, Aryl-niederes Hydroxyalkyl, niederes Alkoxy-niederes Alkyl, niederes Alkanoyloxy-niederes Alkyl, Arylcarbonyloxy-niederes Alkyl, niederes Alkanoyl-niederes Alkyl, Arylcarbonyl-niederes Alkyl, Aryl-niederes Alkyl, niederes Alkenyl, $(C_{3-6})$-Cycloalkyl-niederes Alkyl, niederes Alkinyl, Thienyl-niederes Alkyl, Furyl-niederes Alkyl, Arylcarboxamido-niederes Alkyl, Aryl-niederes Alkenyl, niederes Alkenyloxy-niederes Alkyl, Aryloxy-niederes Alkyl, Aryl-niederes Alkyloxy-niederes Alkyl oder Aryl-N-imidazolonyl-niederes Alkyl,
X ein Sauerstoff- oder Schwefelatom und
n die Zahl 3, 4, 5 oder 6 bedeuten, wobei die mit nieder bezeichneten Reste bis zu 7 Kohlenstoffatome enthalten und Aryl Phenyl oder durch Halogen, Trifluormethyl, niederes Alkyl, niederes Alkoxy, Nitro, Amino, niederes Alkylamino oder di(niederes Alkyl)amino substituiertes Phenyl bedeutet,

ihren optischen und geometrischen Isomeren, sowie ihrer pharmazeutisch annehmbaren Säureadditionssalze, dadurch gekennzeichnet, daß man

a) zur Herstellung einer Verbindung der allgemeinen Formel

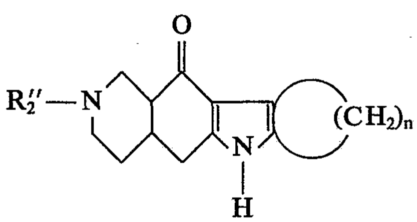

(Ia)

worin

$R_2''$ niederes Alkyl, niederes Alkoxy-niederes Alkyl oder $(C_{3-6})$-Cycloalkyl-niederes Alkyl bedeutet und
n obige Bedeutung besitzt,

eine Verbindung der allgemeinen Formel

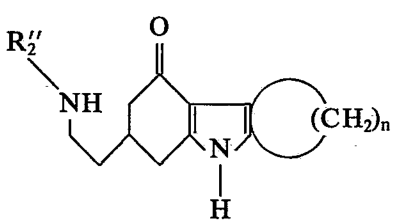

(IX)

worin $R_2''$ und n obige Bedeutung besitzen,

mit Formaldehyd behandelt, oder

b) zur Herstellung einer Verbindung der obigen allgemeinen Formel Ia, eine Verbindung der allgemeinen Formel

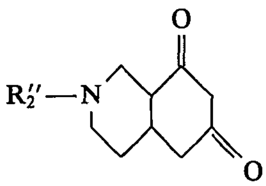

(XII)

worin $R_2''$ obige Bedeutung besitzt,

mit einer Verbindung der allgemeinen Formel

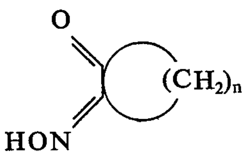

(VII)

in Gegenwart eines Reduktionsmittels oder mit einer Verbindung der allgemeinen Formel

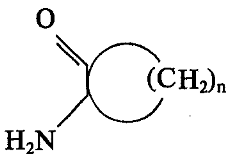

(VIII)

worin n obige Bedeutung besitzt,

oder einer Vorstufe von dieser, behandelt, oder

c) zur Herstellung einer Verbindung der allgemeinen Formel

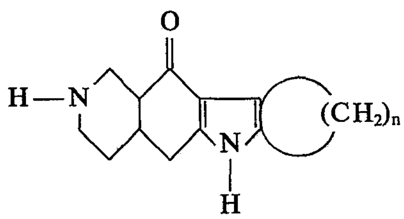

(Ib)

worin n obige Bedeutung besitzt,

eine Verbindung der obigen allgemeinen Formel Ia, worin $R_2''$ Methyl bedeutet, N-demethyliert, oder

d) zur Herstellung einer Verbindung der allgemeinen Formel

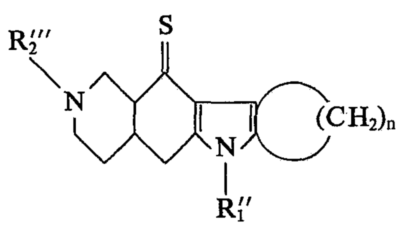

(IIe)

worin

$R_1''$ Wasserstoff, niederes Alkyl oder Aryl-niederes Alkyl und
$R_2'''$ Wasserstoff, niederes Alkyl, niederes Alkoxy-niederes Alkyl, Aryl-niederes Alkyl, niederes Alkenyl, $(C_{3-6})$-Cycloalkyl-niederes Alkyl, niederes Alkinyl, Thienyl-niederes Alkyl, Furyl-niederes Alkyl, niederes Alkenyloxy-niederes Alkyl, Aryl-niederes Alkenyl, Aryloxy-niederes Alkyl oder Aryl-niederes Alkyloxy-niederes Alkyl bedeuten und
n obige Bedeutung besitzt,

eine Verbindung der allgemeinen Formel

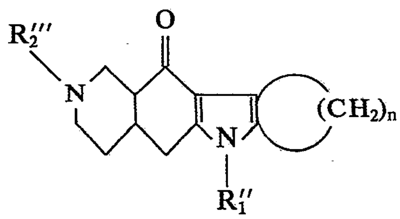

(Ie)

worin $R_1''$, $R_2'''$ und n obige Bedeutung besitzen,

mit Phosphorpentasulfid behandelt, oder

e) zur Herstellung einer Verbindung der allgemeinen Formel

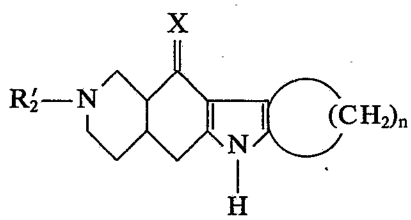

(Ad)

worin

$R_2'$ niederes Alkyl, niederes Hydroxyalkyl, Aryl-niederes Hydroxyalkyl, niederes Alkoxy-niederes Alkyl, niederes Alkanoyloxy-niederes Alkyl, Arylcarbonyloxy-niederes Alkyl, niederes Alkanoyl-niederes Alkyl, Arylcarbonyl-niederes Alkyl, Aryl-niederes Alkyl, niederes Alkenyl, $(C_{3-6})$-Cycloalkyl-niederes Alkyl, niederes Alkinyl, Thienyl-niederes Alkyl, Furyl-niederes Alkyl, Arylcarboxamido-niederes Alkyl, Aryl-niederes Alkenyl, niederes Alkenyloxy-niederes Alkyl, Aryloxy-niederes Alkyl, Aryl-niederes Alkyloxy-niederes Alkyl oder Aryl-N-imidazolonyl-niederes Alkyl

bedeutet und

X und n obige Bedeutung besitzen,

eine Verbindung der allgemeinen Formel

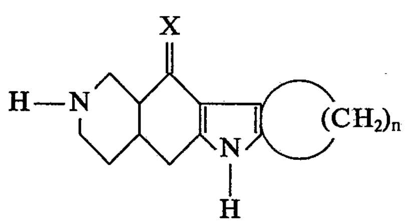

(Ab)

worin X und n obige Bedeutung besitzen,

am Isochinolin-Stickstoffatom substituiert, oder

f) zur Herstellung einer Verbindung der allgemeinen Formel

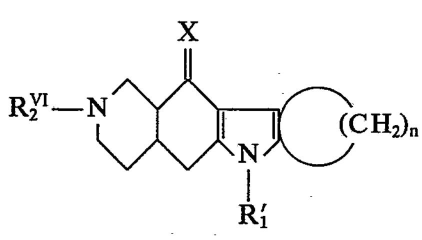

(A'c)

worin

$R_1'$ niederes Alkyl, niederes Alkanoyl, Arylcarbonyl oder Aryl-niederes Alkyl und
$R_2^{VI}$ niederes Alkyl, niederes Alkoxy-niederes Alkyl, niederes Alkanoyloxy-niederes Alkyl, Arylcarbonyloxy-niederes Alkyl, niederes Alkanoyl-niederes Alkyl, Arylcarbonyl-niederes

Alkyl, Aryl-niederes Alkyl, niederes Alkenyl, $(C_{3-6})$-Cycloalkyl-niederes Alkyl, Thienyl-niederes Alkyl, niederes Alkinyl, Furyl-niederes Alkyl, Arylcarboxamido-niederes Alkyl, Aryl-niederes Alkenyl, niederes Alkenyloxy-niederes Alkyl, Aryloxy-niederes Alkyl, Aryl-niederes Alkyloxy-niederes Alkyl oder Aryl-N-imidazolonyl-niederes Alkyl bedeuten und
X und n obige Bedeutung besitzen,

eine Verbindung der allgemeinen Formel

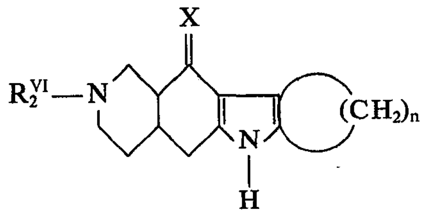

(Ad')

worin $R_2^{VI}$, X und n obige Bedeutung besitzen,

am Pyrrol-Stickstoffatom substituiert, oder

g) zur Herstellung einer Verbindung der allgemeinen Formel

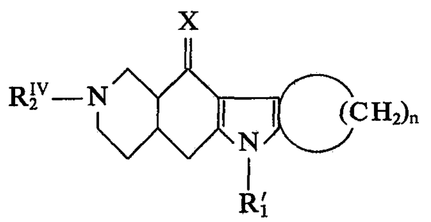

(Ac")

worin

$R_2^{VI}$ niederes Hydroxyalkyl oder Aryl-niederes Hydroxyalkyl bedeuten und
$R_1'$, X und n obige Bedeutung besitzen,

in einer Verbindung der allgemeinen Formel

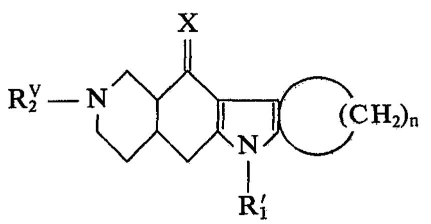

(XIV)

worin

$R_2^{V}$ eine an der Hydroxygruppe geschützte niedere Hydroxyalkyl- oder Aryl-niedere Hydroxyalkyl-Gruppe bedeutet und
$R_1'$, X und n obige Bedeutung besitzen,

die Schutzgruppe abspaltet, oder

h) zur Herstellung einer Verbindung der allgemeinen Formel

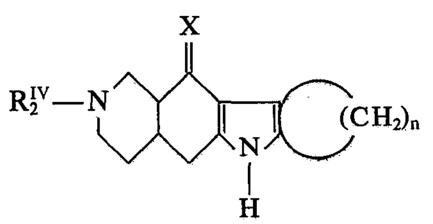

(Ad")

worin $R_2^{IV}$, X und n obige Bedeutung besitzen,

in einer Verbindung der allgemeinen Formel

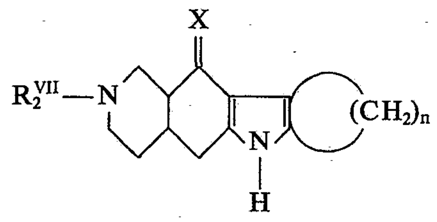

(Ad''')

worin

$R_2^{VII}$ niederes Alkanoyl-niederes Alkyl oder Arylcarbonyl-niederes Alkyl bedeutet und X und n obige Bedeutung besitzen,

die niedere Alkanoyl-niedere Alkyl- oder Arylcarbonyl-niedere Alkyl-Gruppe reduziert, oder

i) zur Herstellung einer Verbindung der allgemeinen Formel

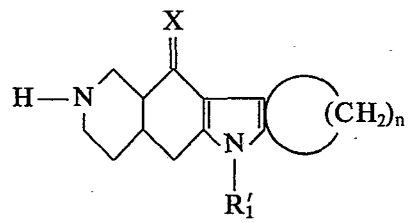

(Ah)

worin X, n und $R_1'$ obige Bedeutung besitzen,

in einer Verbindung der allgemeinen Formel

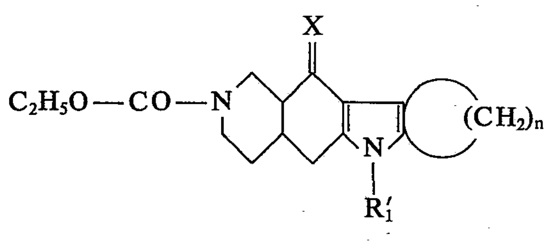

(XIII)

worin X, n und $R_1'$ obige Bedeutung besitzen,

die Äthoxycarbonylgruppe abspaltet, und

j) erwünschtenfalls ein erhaltenes Gemisch der cis- und trans-Isomeren zu einem Endverhältnis, das überwiegend das trans-Isomere aufweist, isomerisiert, und/oder
k) erwünschtenfalls das trans-Isomere aus dem erhaltenen Gemisch abtrennt, und/oder
l) erwünschtenfalls ein erhaltenes Racemat in die optischen Antipoden spaltet, und/oder
m) erwünschtenfalls eine erhaltene Verbindung oder ein pharmazeutisch nicht annehmbares Säureadditionssalz in ein pharmazeutisch annehmbares Säureadditionssalz überführt.

2. Verfahren gemäß Anspruch 1, dadurch gekennzeichnet, daß man nach den Verfahrensvarianten a), b), c), d), e), f), g), j), k), l) und m) verfährt.
3. Verfahren nach Anspruch 1, dadurch gekennzeichnet, daß man nach Verfahrensvariante h) verfährt.
4. Verfahren gemäß einem der Ansprüche 1 bis 3, dadurch gekennzeichnet, daß man eine Verbindung der in Anspruch 1 definierten allgemeinen Formel A, worin $R_1$ Wasserstoff und $R_2$ niederes Alkyl, niederes Hydroxyalkyl, Aryl-niederes Hydroxyalkyl, niederes Alkoxy-niederes Alkyl, Aryloxy-niederes Alkyl, niederes Alkanoyl-niederes Alkyl, Arylcarbonyl-niederes Alkyl oder Aryl-niederes Alkyl bedeuten, herstellt.
5. Verfahren gemäß Anspruch 4, dadurch gekennzeichnet, daß man eine Verbindung der in Anspruch 1 definierten allgemeinen Formel A, worin n die Zahl 3 oder 4 und X ein Sauerstoffatom bedeuten, herstellt.
6. Verfahren gemäß einem der Ansprüche 1–5, dadurch gekennzeichnet, daß man eine Verbindung der in Anspruch 1 definierten allgemeinen Formel A, welche die trans-Konfiguration aufweist, herstellt.
7. Verfahren nach Anspruch 2, dadurch gekennzeichnet, daß man 2-Methyl-1,2,3,4,4a,5,7,8,9,10a-decahydro-4a,10a-trans-6H-cyclopenta[4,5]pyrrolo[2,3-g]isochinolin-10(10H)-on herstellt.

8. Verfahren nach Anspruch 2, dadurch gekennzeichnet, daß man 2-Methyl-1,2,3,4,4a,5,7,8,9,10a-decahydro-4a,10a-trans-6H-cyclopenta[4,5]pyrrolo[2,3-g]isochinolin-10(10H)-on-hydrochlorid · 0,5 $H_2O$ herstellt.

9. Verfahren nach Anspruch 2, dadurch gekennzeichnet, daß man 2-[4-(4-Fluorphenyl)-4-oxobutyl]-1,2,3,4,4a,5,7,8,9,10a-decahydro-4a,10a-trans-6H-cyclopenta[4,5]pyrrolo[2,3-g]isochinolin-10(10H)-on herstellt.

10. Verfahren nach Anspruch 2, dadurch gekennzeichnet, daß man 2-(2-Phenyläthyl)-1,2,3,4,4a,5,7,8,9,10a-decahydro-4a,10a-trans-6H-cyclopenta[4,5]pyrrolo[2,3-g]isochinolin-10(10H)-on herstellt.

11. Verfahren nach Anspruch 2, dadurch gekennzeichnet, daß man (–)-2-Methyl-1,2,3,4,4a,5,7,8,9,10a-decahydro-4a,10a-trans-6H-cyclopenta[4,5]pyrrolo[2,3-g]isochinolin-10(10H)-on herstellt.

12. Verfahren nach Anspruch 2, dadurch gekennzeichnet, daß man 2-Methyl-2,3,4,4a,5,7,8,9,10,11a-decahydro-4a,11a-trans-1H,6H-cyclohexa[4,5]pyrrolo[2,3-g]isochinolin-11(11H)-on herstellt.

13. Verfahren nach Anspruch 2, dadurch gekennzeichnet, daß man 2-Methyl-2,3,4,4a,5,7,8,9,10,11a-decahydro-4a,11a-trans-1H,6H-cyclohexa[4,5]pyrrolo[2,3-g]isochinolin-11(11H)-on · 0,75 $H_2O$ herstellt.

14. Verfahren nach Anspruch 2, dadurch gekennzeichnet, daß man 2-[4-(4-Fluorphenyl)-4-oxobutyl]-2,3,4,4a,5,7,8,9,10,11a-decahydro-4a,11a-trans-1H,6H-cyclohexa[4,5]pyrrolo[2,3-g]isochinolin-11(11H)-on herstellt.

15. Verfahren nach Anspruch 2, dadurch gekennzeichnet, daß man 2-(2-Phenyläthyl)-2,3,4,4a,5,7,8,9,10,11a-decahydro-4a,11a-trans-1H,6H-cyclohexa[4,5]pyrrolo[2,3-g]isochinolin-11(11H)-on herstellt.

16. Verfahren nach Anspruch 2, dadurch gekennzeichnet, daß man 2-Methyl-1,2,3,4,4a,5,7,8,9,10a-decahydro-4a,10a-trans-6H-cyclopenta[4,5]pyrrolo[2,3-g]isochinolin-10(10H)-thion herstellt.

**Claims for the Contracting States: BE, CH, DE, FR, GB, IT, LI, LU, NL, SE**

1. Cycloalka[4,5]pyrrolo[2,3-g]isoquinolines of the general formula

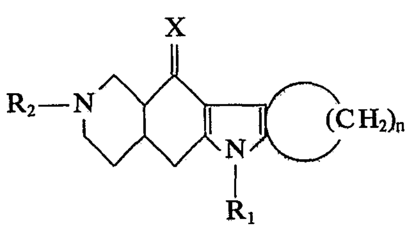

(A)

wherein

$R_1$ signifies hydrogen, lower alkyl, lower alkanoyl, arylcarbonyl or aryl-lower alkyl,

$R_2$ signifies hydrogen, lower alkyl, lower hydroxyalkyl, aryl-lower hydroxyalkyl, lower alkoxy-lower alkyl, lower alkanoyloxy-lower alkyl, arylcarbonyloxy-lower alkyl, lower alkanoyl-lower alkyl, arylcarbonyl-lower alkyl, aryl-lower alkyl, lower alkenyl, ($C_{3-6}$)-cycloalkyl-lower alkyl, lower alkynyl, thienyl-lower alkyl, furyl-lower alkyl, arylcarboxamido-lower alkyl, aryl-lower alkenyl, lower alkenyloxy-lower alkyl, aryloxy-lower alkyl, aryl-lower alkyloxy-lower alkyl or aryl-N-imidazolonyl-lower alkyl,

X signifies an oxygen or sulphur atom and

n signifies the number 3, 4, 5 or 6, wherby the residues denoted as lower contain up to 7 carbon atoms and aryl signifies phenyl or phenyl substituted by halogen, trifluoromethyl, lower alkyl, lower alkoxy, nitro, amino, lower alkylamino or di(lower alkyl)amino,

optical and geometric isomers of these compounds and pharmaceutically acceptable acid addition salts thereof.

2. Compounds in accordance with claim 1, characterized in that $R_1$ signifies hydrogen and $R_2$ signifies lower alkyl, lower hydroxyalkyl, aryl-lower hydroxyalkyl, lower alkoxy-lower alkyl, aryloxy-lower alkyl, lower alkanoyl-lower alkyl, arylcarbonyl-lower alkyl or aryl-lower alkyl.

3. Compounds in accordance with claim 2, characterized in that n signifies the number 3 or 4 and X signifies an oxygen atom.

4. Compounds in accordance with any one of claims 1–3, characterized in that they have the trans configuration.

5. 2-Methyl-1,2,3,4,4a,5,7,8,9,10a-decahydro-4a,10a-trans-6H-cyclopenta[4,5]pyrrolo[2,3-g]-isoquinolin-10(10H)-one.

6. 2-Methyl-1,2,3,4,4a,5,7,8,9,10a-decahydro-4a,10a-trans-6H-cyclopenta[4,5]pyrrolo[2,3-g]-isoquinolin-10(10H)-one hydrochloride 0,5 $H_2O$.

7. 2-[4-(4-Fluorophenyl)-4-oxobutyl]-1,2,3,4,4a,5,7,8,9,10a-decahydro-4a,10a-trans-6H-cyclopenta[4,5]pyrrolo[2,3-g]isoquinolin-10(10H)-one.

8. 2-(2-Phenylethyl)-1,2,3,4,4a,5,7,8,9,10a-decahydro-4a,10a-trans-6H-cyclopenta[4,5]pyrrolo[2,3-g]isoquinolin-10(10H)-one.

9. (−)-2-Methyl-1,2,3,4,4a,5,7,8,9,10a-decahydro-4a,10a-trans-6H-cyclopenta[4,5]pyrrolo-[2,3-g]isoquinolin-10(10H)-one.

10. 2-Methyl-2,3,4,4a,5,7,8,9,10,11a-decahydro-4a,11a-trans-1H,6H-cyclohexa[4,5]pyrrolo-[2,3-g]isoquinolin-11(11H)-one.

11. 2-Methyl-2,3,4,4a,5,7,8,9,10,11a-decahydro-4a,11a-trans-1H,6H-cyclohexa[4,5]pyrrolo-[2,3-g]isoquinolin-11(11H)-one 0,75 $H_2O$.

12. 2-[4-(4-Fluorophenyl)-4-oxobutyl]-2,3,4,4a,5,7,8,9,10,11a-decahydro-4a,11a-trans-1H,6H-cyclohexa[4,5]pyrrolo[2,3-g]isoquinolin-11(11H)-one.

13. 2-(2-Phenylethyl)-2,3,4,4a,5,7,8,9,10,11a-decahydro-4a,11a-trans-1H,6H-cyclohexa[4,5]-pyrrolo[2,3-g]isoquinolin-11(11H)-one.

14. 2-Methyl-1,2,3,4,4a,5,7,8,9,10a-decahydro-4a,10a-trans-6H-cyclopenta[4,5]pyrrolo[2,3-g]-isoquinoline-10(10H)-thione.

15. Compounds of the general formula

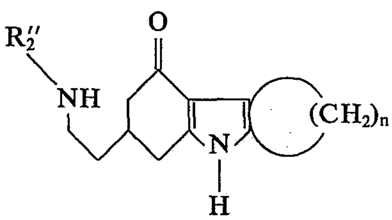

(IX)

wherein

$R_2''$ signifies lower alkyl, lower alkoxy-lower alkyl or ($C_{3-6}$)-cycloalkyl-lower alkyl and
n signifies the number 3,4,5 or 6, whereby the residues denoted as lower contain up to 7 carbon atoms.

16. Compounds in accordance with claim 15, characterized in that $R_2''$ signifies methyl.

17. Compounds of the general formula

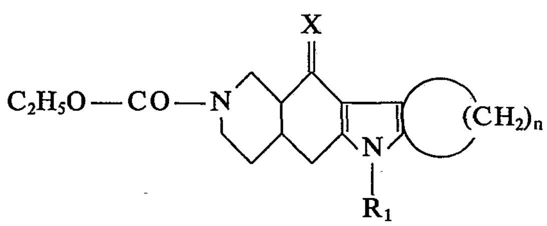

(XIII)

wherein

X signifies an oxygen or sulphur atom,
n signifies the nuber 3, 4, 5 or 6, and
$R_1$ signifies hydrogen, lower alkyl, lower alkanoyl, arylcarbonyl or aryl-lower alkyl, whereby the residues denoted as lower contain up to 7 carbon atoms and aryl signifies phenyl or phenyl substituted by halogen, trifluoromethyl, lower alkyl, lower alkoxy, nitro, amino, lower akylamino or di(lower alkyl)amino.

18. Compounds in accordance with any one of claims 1—14 as pharmaceutically active substances.

19. Compounds in accordance with any one of claims 1—14 as antipsychotic active substances.

20. A process for the manufacture of compounds in accordance with any one of claims 1—14, characterized by

a) for the manufacture of a compound of the general formula

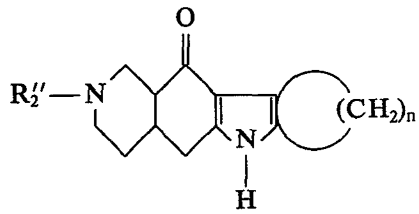

(Ia)

wherein

$R_2''$ signifies lower alkyl, lower alkoxy-lower alkyl or $(C_{3-6})$-cycloalkyl-lower alkyl and
n has the above significance,

treating a compound of the general formula

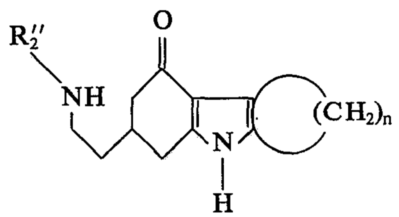

(IX)

wherein $R_2''$ and n have the above significance,

with formaldehyde, or

b) for the manufacture of a compound of the general formula Ia above, treating a compound of the general formula

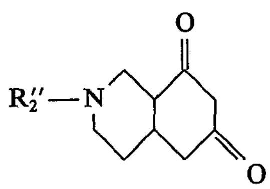

(XII)

wherein $R_2''$ has the above significance,

with a compound of the general formula

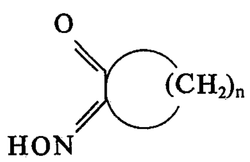

(VII)

in the presence of a reduction agent or with a compound of the general formula

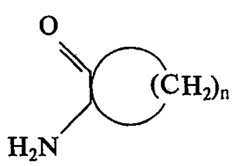

(VIII)

wherein n has the above significance,

or a precursor of this, or

c) for the manufacture of a compound of the general formula

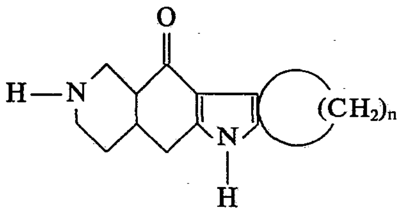

(Ib)

wherein n has the above significance,

N-demethylating a compound of general formula Ia above wherein $R_2''$ signifies, or

d) for the manufacture of a compound of the general formula

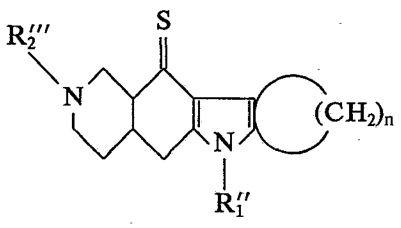

(IIe)

wherein

$R_1''$ signifies hydrogen, lower alkyl or aryl-lower alkyl and
$R_2'''$ signifies hydrogen, lower alkyl, lower alkoxy-lower alkyl, aryl-lower alkyl, lower alkenyl, $(C_{3-6})$-cycloalkyl-lower alkyl, lower alkynyl, thienyl-lower alkyl, furyl-lower alkyl, lower alkenyloxy-lower alkyl, aryl-lower alkenyl, aryloxy-lower alkyl or aryl-lower alkyloxy-lower alkyl and
n has the above significance,

treating a compound of the general formula

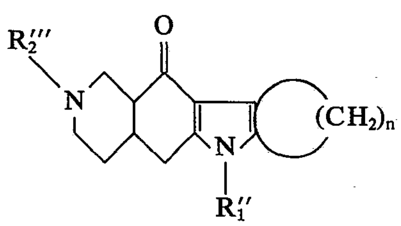

(Ie)

wherein $R_1''$, $R_2'''$ and n have the above significance.

with phosphorus pentasulphide, or

e) for the manufacture of a compound of the general formula

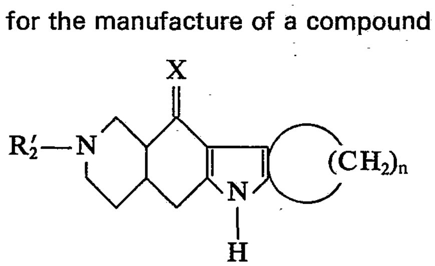

(Ad)

wherein

$R_2'$ signifies lower alkyl, lower hydroxyalkyl, aryl-lower hydroxyalkyl, lower alkoxy-lower alkyl, lower alkanoyloxy-lower alkyl, arylcarbonyloxy-lower alkyl, lower alkanoyl-lower alkyl, arylcarbonyl-lower alkyl, aryl-lower alkyl, lower alkenyl, $(C_{3-6})$-cycloalkyl-lower alkyl, lower alkynyl, thienyl-lower alkyl, furyl-lower alkyl, arylcarboxamido-lower alkyl, aryl-lower alkenyl, lower alkenyloxy-lower alkyl, aryloxy-lower alkyl, aryl-lower alkyloxy-lower alkyl or aryl-N-imidazolonyl-lower alkyl and
X and n have the above significance,

substituting a compound of the general formula

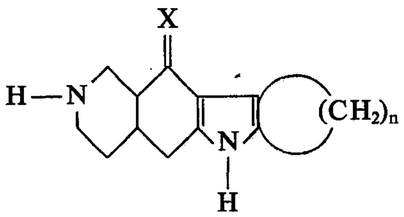

(Ab)

wherein X and n have the above significance,

at the isoquinoline nitrogen atom, or

f) for the manufacture of a compound of the general formula

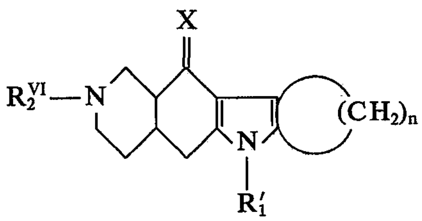

(A'c)

wherein

$R_1'$ signifies lower alkyl, lower alkanoyl, arylcarbonyl or aryl-lower alkyl and
$R_2^{VI}$ signifies lower alkyl, lower alkoxy-lower alkyl, lower alkanoyloxy-lower alkyl, arylcarbonyloxy-lower alkyl, lower alkanoyl-lower alkyl, arylcarbonyl-lower alkyl, aryl-lower alkyl, lower alkenyl, $(C_{3-6})$-cycloalkyl-lower alkyl, thienyl-lower alkyl, lower alkynyl, furyl-lower alkyl, arylcarboxamido-lower alkyl, aryl-lower alkenyl, lower alkenyloxy-lower alkyl, aryloxy-lower alkyl, aryl-lower alkyloxy-lower alkyl or aryl-N-imidazolonyl-lower alkyl and
X and n have the above significance,

substituting a compound of the general formula

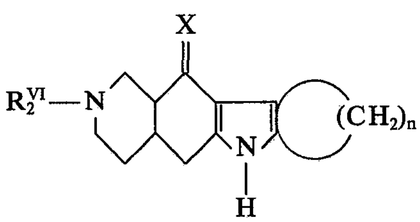

(Ad')

wherein $R_2^{VI}$, X and n have the above significance,

at the pyrrole nitrogen atom, or

g) for the manufacture of a compound of the general formula

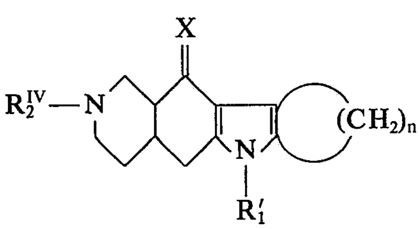

(Ac")

wherein

$R_2^{VI}$ signifies lower hydroxyalkyl or aryl-lower hydroxyalkyl and
$R_1'$, X and n have the above significance,

cleaving off the protecting group in a compound of the general formula

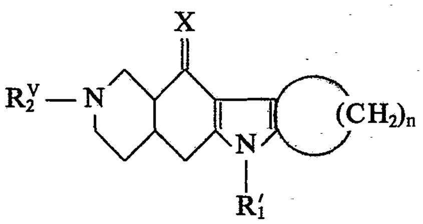

(XIV)

wherein

$R_2^V$ signifies a lower hydroxyalkyl or aryl-lower hydroxyalkyl group protected at the hydroxy group and

$R_1'$, X and n have the above significance, or

h) for the manufacture of a compound of the general formula

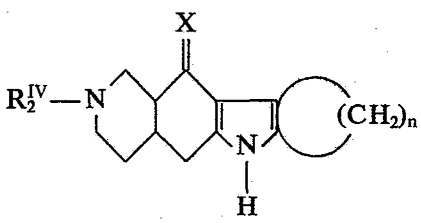

(Ad″)

wherein $R_2^{IV}$, X and n have the above significance,

reducing the lower alkanoyl-lower alkyl or arylcarbonyl-lower alkyl group in a compound of the general formula

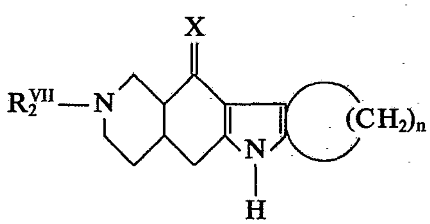

(Ad‴)

wherein

$R_2^{VII}$ signifies lower alkanoyl-lower alkyl or arylcarbonyl-lower alkyl and

X and n have the above significance, or

i) for the manufacture of a compound of the general formula

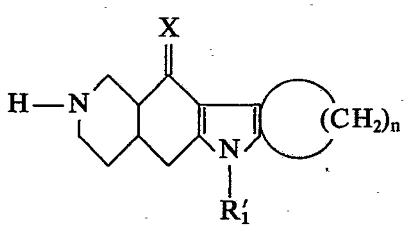

(Ah)

wherein X, n and $R_1'$ have the above significance,

cleaving off the ethoxycarbonyl group in a compound of the general formula

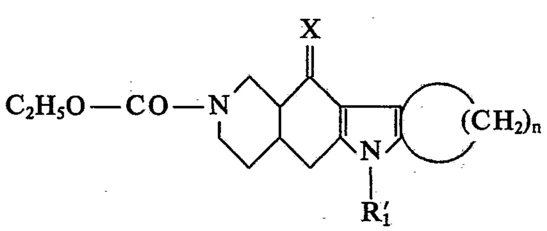

(XIII)

wherein X, n and $R_1'$ have the above significance, and

j) if desired, isomerizing a mixture of the cis and trans isomers obtained to a final ratio which comprises predominantly the trans isomer, and/or
k) if desired, separating the trans isomer from the mixture obtained, and/or
l) if desired, resolving a racemate in to the optical antipodes, and/or
m) if desired, converting a compound obtained or a nonpharmaceutically acceptable acid addition salt into a pharmaceutically acceptable acid addition salt.

21. A process in accordance with claim 20, characterized in that process variants a), b), c), d), e), f), g), j), k), l) and m) are carried out.
22. A process in accordance with claim 20, characterized in that process variant h) is carried out.
23. A medicament containing a compound in accordance with any one of claims 1—14.
24. A neuroleptic/antipsychotic medicament containing a compound in accordance with any one of claims 1—14.

**Claims for the Contracting State: AT**

1. A process for the manufacture of cycloalka[4,5]pyrrolo[2,3-g]isoquinolines of the general formula

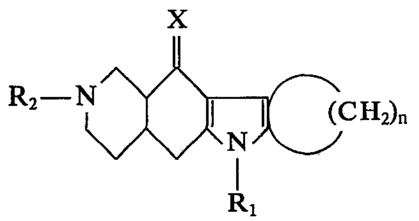

(A)

wherein

$R_1$ signifies hydrogen, lower alkyl, lower alkanoyl, arylcarbonyl or aryl-lower alkyl,
$R_2$ signifies hydrogen, lower alkyl, lower hydroxyalkyl, aryl-lower hydroxyalkyl, lower alkoxy-lower alkyl, lower alkanoyloxy-lower alkyl, arylcarbonyloxy-lower alkyl, lower alkanoyl-lower alkyl, arylcarbonyl-lower alkyl, aryl-lower alkyl, lower alkenyl, $(C_{3-6})$-cycloalkyl-lower alkyl, lower alkynyl, thienyl-lower alkyl, furyl-lower alkyl, arylcarboxamido-lower alkyl, aryl-lower alkenyl, lower alkenyloxy-lower alkyl, aryloxy-lower alkyl, aryl-lower alkyloxy-lower alkyl or aryl-N-imidazolonyl-lower alkyl,
X signifies an oxygen or sulphur atom and
n signifies the number 3, 4, 5 or 6, whereby the residues denoted as lower contain up to 7 carbon atoms and aryl signifies phenyl or phenyl substituted by halogen, trifluoromethyl, lower alkyl, lower alkoxy, nitro, amino, lower alkylamino or di(lower alkyl)amino,

their optical and geometric isomers, as well as their pharmaceutically acceptable acid addition salts, characterized by

a) for the manufacture of a compound of the general formula

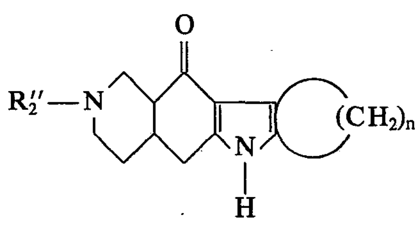

(Ia)

wherein

$R_2''$ signifies lower alkyl, lower alkoxy-lower alkyl or $(C_{3-6})$-cycloalkyl-lower alkyl and
n has the above significance,

treating a compound of the general formula

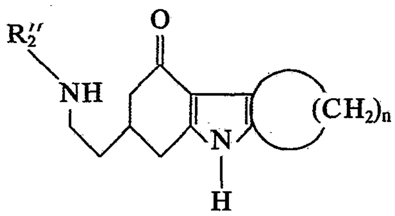

(IX)

wherein $R_2''$ and n have the above significance,

with formaldehyde, or

b) for the manufacture of a compound of the general formula Ia above, treating a compound of the general formula

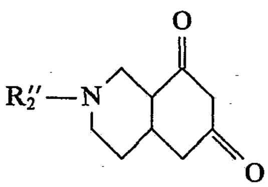

(XII)

wherein $R_2''$ has the above significance,

with a compound of the general formula

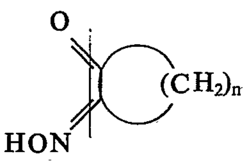

(VII)

in the presence of a reduction agent or with a compound of the general formula

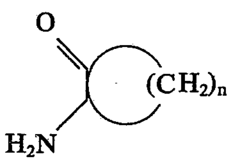

(VIII)

wherein n has the above significance,

or a precursor of this, or

c) for the manufacture of a compound of the general formula

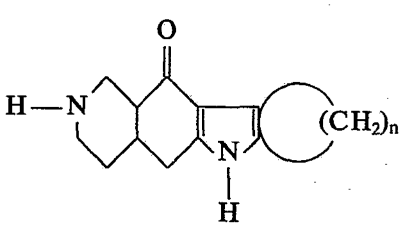

(Ib)

wherein n has the above significance,

N-demethylating a compound of general formula Ia above wherein $R_2''$ signifies, methyl, or

d) for the manufacture of a compound of the general formula

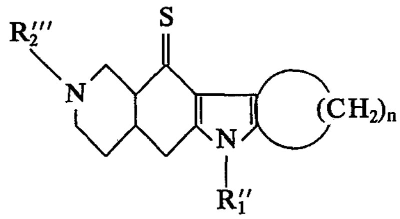

(IIe)

wherein

$R_1''$ signifies hydrogen, lower alkyl or aryl-lower alkyl and
$R_2'''$ signifies hydrogen, lower alkyl, lower alkoxy-lower alkyl, aryl-lower alkyl, lower alkenyl, ($C_{3-6}$)-cycloalkyl-lower alkyl, lower alkynyl, thienyl-lower alkyl, furyl-lower alkyl, lower alkenyloxy-lower alkyl, aryl-lower alkenyl, aryloxy-lower alkyl or aryl-lower alkyloxy-lower alkyl and
n has the above significance,

treating a compound of the general formula

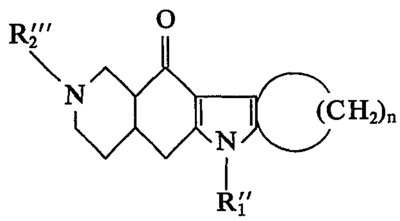

(Ie)

wherein $R_1''$, $R_2'''$ and n have the above significance,

with phosphorus pentasulphide, or

e) for the manufacture of a compound of the general formula

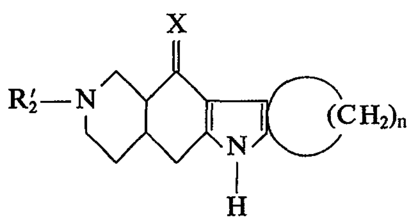

(Ad)

wherein

$R_2'$ signifies lower alkyl, lower hydroxyalkyl, aryl-lower hydroxyalkyl, lower alkoxy-lower alkyl, lower alkanoyloxy-lower alkyl, arylcarbonyloxy-lower alkyl, lower alkanoyl-lower alkyl, arylcarbonyl-lower alkyl, aryl-lower alkyl, lower alkenyl, ($C_{3-6}$)-cycloalkyl-lower alkyl, lower alkynyl, thienyl-lower alkyl, furyl-lower alkyl, arylcarboxamido-lower alkyl, aryl-lower alkenyl, lower alkenyloxy-lower alkyl, aryloxy-lower alkyl, aryl-lower alkyloxy-lower alkyl or aryl-N-imidazolonyl-lower alkyl and
X and n have the above significance,

substituting a compound of the general formula

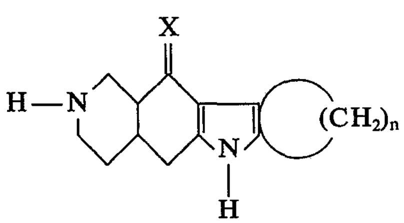

(Ab)

wherein X and n have the above significance,

at the isoquinoline nitrogen atom, or

f) for the manufacture of a compound of the general formula

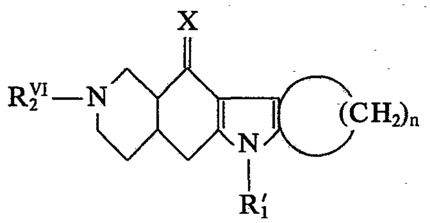

(A'c)

wherein

| | |
|---|---|
| $R_1'$ | signifies lower alkyl, lower alkanoyl, arylcarbonyl or aryl-lower alkyl and |
| $R_2^{VI}$ | signifies lower alkyl, lower alkoxy-lower alkyl, lower alkanoyloxy-lower alkyl, arylcarbonyloxy-lower alkyl, lower alkanoyl-lower alkyl, arylcarbonyl-lower alkyl, aryl-lower alkyl, lower alkenyl, $(C_{3-6})$-cycloalkyl-lower alkyl, thienyl-lower alkyl, lower alkynyl, furyl-lower alkyl, arylcarboxamido-lower alkyl, aryl-lower alkenyl, lower alkenyloxy-lower alkyl, aryloxy-lower alkyl, aryl-lower alkyloxy-lower alkyl or aryl-N-imidazolonyl-lower alkyl and |
| X and n | have the above significance, |

substituting a compound of the general formula

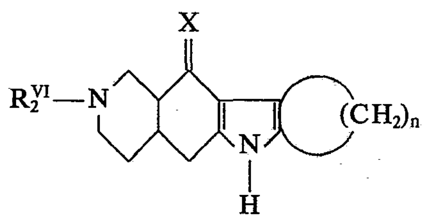

(Ad')

wherein $R_2^{VI}$, X and n have the above significance,

at the pyrrole nitrogen atom, or

g) for the manufacture of a compound of the general formula

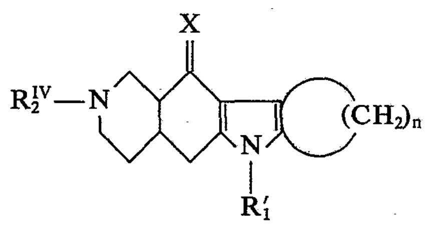

(Ac'')

wherein

| | |
|---|---|
| $R_2^{VI}$ | signifies lower hydroxyalkyl or aryl-lower hydroxyalkyl and |
| $R_1'$, X and n | have the above significance, |

cleaving off the protecting group in a compound of the general formula

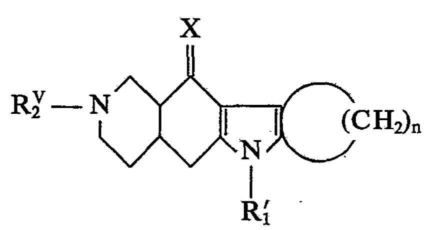

(XIV)

wherein

| | |
|---|---|
| $R_2^{V}$ | signifies a lower hydroxyalkyl or aryl-lower hydroxyalkyl group protected at the hydroxy group and |
| $R_1'$, X and n | have the above significance, or |

h) for the manufacture of a compound of the general formula

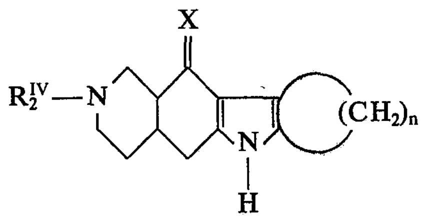

(Ad'')

wherein $R_2^{IV}$, X and n have the above significance,

reducing the lower alkanoyl-lower alkyl or arylcarbonyl-lower alkyl group in a compound of the general formula

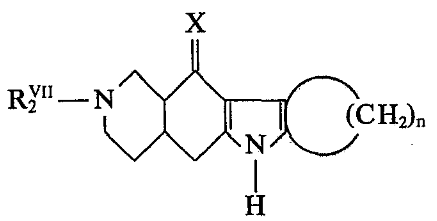

(Ad''')

wherein

$R_2^{VII}$ signifies lower alkanoyl-lower alkyl or arylcarbonyl-lower alkyl and
X and n have the above significance, or

i) for the manufacture of a compound of the general formula

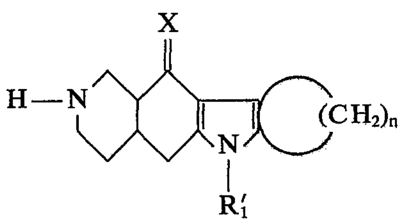

(Ah)

wherein X, n and $R_1'$ have the above significance,

cleaving off the ethoxycarbonyl group in a compound of the general formula

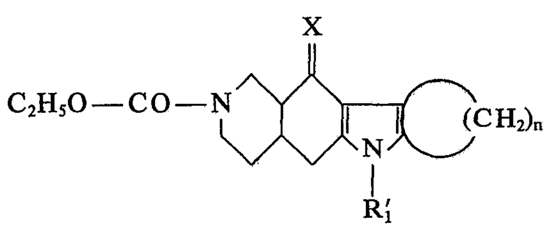

(XIII)

wherein X, n and $R_1'$ have the above significance, and

j) if desired, isomerizing a mixture of the cis- and trans isomers obtained to a final ratio which comprises predominantly the trans isomer, and/or

k) if desired, separating the trans isomer from the mixture obtained, and/or

l) if desired, resolving a racemate in to the optical antipodes, and/or

m) if desired, converting a compound obtained or a non-pharmaceutically acceptable acid addition salt into a pharmaceutically acceptable acid addition salt.

2. A process in accordance with claim 1, characterized in that process variants a), b), c), d), e), f), g), j), k), l) and m) are carried out.

3. A process according to claim 1, characterized in that process variant h) is carried out.

4. A process in accordance with any one of claims 1 to 3, characterized in that a compound of the general formula A defined in claim 1 in which $R_1$ signifies hydrogen and $R_2$ signifies lower alkyl, lower hydroxyalkyl, aryl-lower hydroxyalkyl, lower alkoxy-lower alkyl, aryloxy-lower alkyl, lower alkanoyl-lower alkyl, arylcarbonyl-lower alkyl or aryl-lower alkyl is manufactured.

5. A process in accordance with claim 4, characterized in that a compound of the general formula defined in claim 1 in which n signifies the number 3 or 4 and X signifies an oxygen atom is manufactured.

6. A process in accordance with any one of claims 1—5, characterized in that a compound of the general formula A defined in claim 1 which has the trans configuration is manufactured.

7. A process according to claim 2, characterized in that 2-methyl-1,2,3,4,4a,5,7,8,9,10a-decahydro-4a,10a-trans-6H-cyclopenta[4,5]pyrrolo[2,3-g]isoquinolin-10(10H)-one is manufactured.

8. A process according to claim 2, characterized in that 2-methyl-1,2,3,4,4a,5,7,8,9,10a-decahydro-4a,10a-trans-6H-cyclopenta[4,5]pyrrolo[2,3-g]isoquinolin-10(10H)-one hydrochloride 0,5 $H_2O$ is manufactured.

9. A process according to claim 2, characterized in that 2-[4-(4-fluorphenyl)-4-oxobutyl]-1,2,3,4,4a,5,7,8,9,10a-decahydro-4a,10a-trans-6H-cyclopenta[4,5]pyrrolo[2,3-g]isoquinolin-10(10H)-one is manufactured.

10. A process according to claim 2, characterized in that 2-(2-phenylethyl)-1,2,3,4,4a,5,7,8,9,10a-decahydro-4a,10a-trans-6H-cyclopenta[4,5]pyrrolo[2,3-g]isoquinolin-10(10H)-one is manufactured.

11. A process according to claim 2, characterized in that (—)-2-methyl-1,2,3,4,4a,5,7,8,9,10a-decahydro-4a,10a-trans-6H-cyclopenta[4,5]pyrrolo[2,3-g]isoquinolin-10(10H)-one is manufactured.

12. A process according to claim 2, characterized in that 2-methyl-2,3,4,4a,5,7,8,9,10,11a-decahydro-4a,11a-trans-1H,6H-cyclohexa[4,5]pyrrolo[2,3-g]isoquinolin-11(11H)-one is manufactured.

13. A process according to claim 2, characterized in that 2-methyl-2,3,4,4a,5,7,8,9,10,11a-decahydro-4a,11a-trans-1H,6H-cyclohexa[4,5]pyrrolo[2,3-g]isoquinolin-11(11H)-one 0,75 $H_2O$ is manufactured.

14. A process according to claim 2, characterized in that 2-[4-(4-fluorophenyl)-4-oxobutyl]-2,3,4,4a,5,7,8,9,10,11a-decahydro-4a,11a-trans-1H,6H-cyclohexa[4,5]pyrrolo[2,3-g]isoquinolin-11(11H)-one is manufactured.

15. A process according to claim 2, characterized in that 2-(2-phenylethyl)-2,3,4,4a,5,7,8,9,10,11a-decahydro-4a,11a-trans-1H,6H-cyclohexa[4,5]pyrrolo[2,3-g]isoquinolin-11(11H)-one is manufactured.

16. A process according to claim 2, characterized in that 2-methyl-1,2,3,4,4a,5,7,8,9,10a-decahydro-4a,10a-trans-6H-cyclopenta[4,5]pyrrolo[2,3-g]isoquinolin-10(10H)-thione is manufactured.

**Revendications pour les Etats contractants: BE, CH, DE, FR, GB, IT, LI, LU, NL, SE**

1. Cycloalca[4,5]pyrrolo[2,3-g]isoquinoléines de formule générale

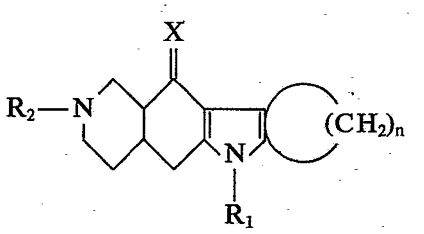

(A)

où

- $R_1$ représente un hydrogène, un alcoyle inférieur, un alcanoyle inférieur, un arylcarbonyle ou un arylalcoyle inférieur,
- $R_2$ représente un hydrogène, un alcoyle inférieur, un hydroxyalcoyle inférieur, un aryl-hydroxyalcoyle inférieur, un alcoxy inférieur-alcoyle inférieur, un alcanoyloxy inférieur-alcoyle inférieur, un arylcarbonyloxy-alcoyle inférieur, un alcanoyle inférieur-alcoyle inférieur, un arylcarbonyl-alcoyle inférieur, un aryl-alcoyle inférieur, un alcényle inférieur, un cycloalcoyle en $C_3$ à $C_6$-alcoyle inférieur, un alcynyle inférieur, un thiényl-alcoyle inférieur, un furyl-alcoyle inférieur, un arylcarboxamido-alcoyle inférieur, un aryl-alcényle inférieur, un alcényloxy inférieur-alcoyle inférieur, un aryloxy-alcoyle inférieur, un aryl-alcoyloxy inférieur-alcoyle inférieur ou un aryl-N-imidazolonyl-alcoyle inférieur,
- X représente un atome d'oxygène ou de soufre et
- n le nombre 3, 4, 5 ou 6, où les radicaux désignés comme inférieurs contiennent jusqu'à 7 atomes de carbone et aryle représente un phényle ou un phényle substitué par un halogène, un trifluorométhyle, un alcoyle inférieur, un alcoxy inférieur, un nitro, un amino, un alcoyle inférieur-amino ou un di(alcoyle inférieur)amino,

les isomères optiques et géométriques de ces composés et leurs sels d'addition d'acides pharmaceutiquement acceptables.

2. Composés selon la revendication 1, caractérisés en ce que $R_1$ représente un hydrogène et $R_2$ un alcoyle inférieur, un hydroxyalcoyle inférieur, un aryl-hydroxyalcoyle inférieur, un alcoxy inférieur-alcoyle inférieur, un aryloxy-alcoyle inférieur, un alcanoyle inférieur-alcoyle inférieur, un arylcarbonyl-alcoyle inférieur ou un aryl-alcoyle inférieur.

3. Composés selon la revendication 2, caractérisés en ce que n représente le nombre 3 ou 4 et X un atome d'oxygène.

4. Composés selon l'une des revendications 1—3, caractérisés en ce qu'ils présentent la configuration trans.

5. 2 - Méthyl - 1,2,3,4,4a,5,7,8,9,10a - décahydro - 4a,10a - trans - 6 H - cyclopenta[4,5]pyrrolo-[2,3-g]isoquinolin-10(10 H)-one.

6. Chlorhydrate de 2-méthyl-1,2,3,4,4a,5,7,8,9,10a-décahydro-4a,10a-trans-6 H-cyclopenta-[4,5]pyrrolo[2,3-g]isoquinolin-10(10 H)-one · 0,5 $H_2O$.

7. 2-[4-(4-Fluorophényl)-4-oxobutyl]-1,2,3,4,4a,5,7,8,9,10a-décahydro-4a,10a-trans-6 H-cyclopenta[4,5]pyrrolo[2,3-g]isoquinolin-10(10 H)-one.

8. 2 - (2 - Phényléthyl) - 1,2,3,4,4a,5,7,8,9,10a - décahydro - 4a,10a - trans - 6 H - cyclopenta[4,5]-pyrrolo[2,3-g]isoquinolin-10(10 H)-one.

9. (—) - 2 - Méthyl - 1,2,3,4,4a,5,7,8,9,10a - décahydro - 4a,10a - trans - 6 H - cyclopenta[4,5]pyrrolo-[2,3-g]isoquinolin-10(10 H)-one.

10. 2-Méthyl-2,3,4,4a,5,7,8,9,10,11a-décahydro-4a,11a-trans-1 H,6 H-cyclohexa[4,5]pyrrolo-[2,3-g]isoquinolin-11(11 H)-one.

11. 2-Méthyl-2,3,4,4a,5,7,8,9,10,11a-décahydro-4a,11a-trans-1 H,6 H-cyclohexa[4,5]pyrrolo-[2,3-g]isoquinolin-11(11 H)-one · 0,75 $H_2O$.

12. 2 - [4 - (4 - Fluorophényl) - 4 - oxobutyl] - 2,3,4,4a,5,7,8,9,10,11a - décahydro - 4a,11a - trans-1 H,6 H-cyclohexa[4,5]pyrrolo[2,3-g]isoquinolin-11(11 H)-one.

13. 2-(2-Phényléthyl)-2,3,4,4a,5,7,8,9,10,11a-décahydro-4a,11a-trans-1 H,6 H-cyclohexa[4,5]-pyrrolo[2,3-g]isoquinolin-11(11 H)-one.

14. 2 - Méthyl - 1,2,3,4,4a,5,7,8,9,10a - décahydro - 4a,10a - trans - 6 H - cyclopenta[4,5]pyrrolo-[2,3-g]isoquinolin-10(10 H)-thione.

15. Composés de formule générale

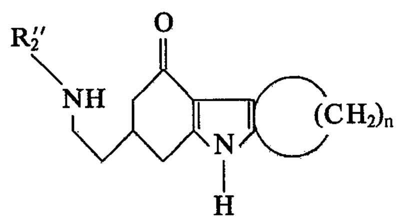

(IX)

où

$R_2''$ représente un alcoyle inférieur, un alcoxy inférieur-alcoyle inférieur ou un cycloalcoyle en $C_3$ à $C_6$-alcoyle inférieur et

n le nombre 3, 4, 5 ou 6, où les radicaux désignés comme inférieurs contiennent jusqu'à 7 atomes de carbone.

16. Composés selon la revendication 15, caractérisés en ce que $R_2''$ représente un méthyle.

17. Composés de formule générale

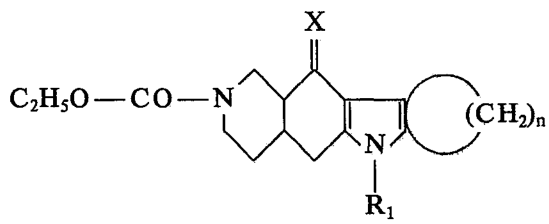

(XIII)

où

X représente un atome d'oxygène ou de soufre,

n représente le nombre 3, 4, 5 ou 6, et

$R_1$ représente un hydrogène, un alcoyle inférieur, un alcanoyle inférieur, un arylcarbonyle ou un aryl-alcoyle inférieur, où les radicaux désignés comme inférieurs contiennent jusqu'à 7 atomes de carbone et aryle représente un phényle ou un phényle substitué par un halogène, un trifluorométhyle, un alcoyle inférieur, un alcoxy inférieur, un nitro, un amino, un alcoyle inférieur-amino ou un di-(alcoyle inférieur)amino.

18. Composés selon l'une des revendications 1—14 comme substances actives pharmaceutiques.

19. Composés selon l'une des revendications 1—14 comme substances actives anti-psychotiques.

20. Procédé de préparation de composés selon l'une des revendications 1—14, caractérisé en ce que

a) pour préparer un composé de formule générale

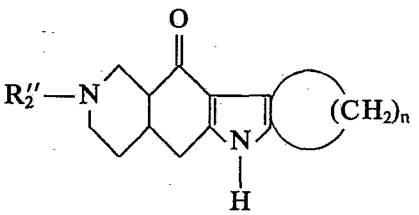

(Ia)

où

$R_2''$ représente un alcoyle inférieur, un alcoxy inférieur-alcoyle inférieur ou un cycloalcoyle en $C_3$ à $C_6$-alcoyle inférieur et

n a la signification donnée ci-dessus,

on traite un composé de formule générale

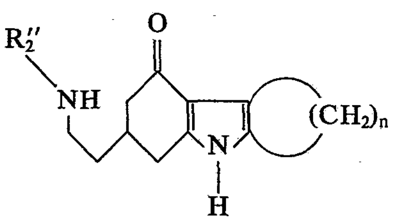

(IX)

où $R_2''$ et n ont la signification donnée ci-dessus,

avec du formaldéhyde, ou

b) pour préparer un composé de formule générale I a ci-dessus, on traite un composé de formule générale

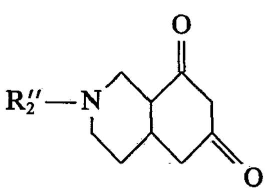

(XII)

où $R_2''$ a la signification donnée ci-dessus,

avec un composé de formule générale

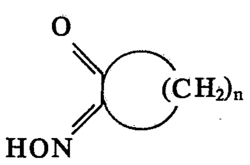

(VII)

en présence d'un réducteur ou avec un composé de formule générale

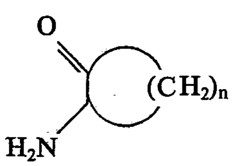

(VIII)

où n a la signification donnée ci-dessus,

ou un précurseur de celui-ci, ou

c) pour préparer un composé de formule générale

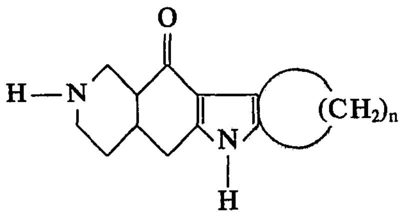

(Ib)

où n a la signification donnée ci-dessus,

on N-déméthyle un composé de formule générale Ia ci-dessus, où $R_2''$ représente un méthyle, ou

d) pour préparer un composé de formule générale

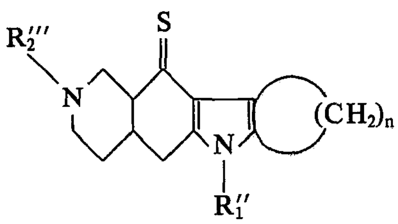

(IIe)

où

$R_1''$ représente un hydrogène, un alcoyle inférieur ou un aryl-alcoyle inférieur et

$R_2'''$ représente un hydrogène, un alcoyle inférieur, un alcoxy inférieur-alcoyle inférieur, un aryl-alcoyle inférieur, un alcényle inférieur, un cyclo-alcoyle en $C_3$ à $C_6$-alcoyle inférieur, un alcynyle inférieur, un thiényl-alcoyle inférieur, un furyl-alcoyle inférieur, un alcényloxy inférieur-alcoyle inférieur, un aryl-alcényle inférieur, un aryloxy-alcoyle inférieur ou un aryl-alcoyloxy-inférieur-alcoyle inférieur et

n a la signification donnée ci-dessus,

on traite un composé de formule générale

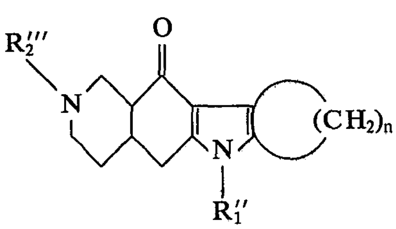

(Ie)

où $R_1''$, $R_2'''$ et n ont la signification donnée ci-dessus,

avec du pentasulfure de phosphore, ou

e) pour préparer un composé de formule générale

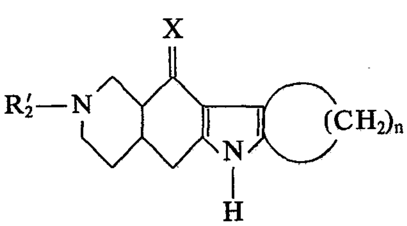

(Ad)

où

$R_2'$ représente un alcoyle inférieur, un hydroxyalcoyle inférieur, un aryl-hydroxyalcoyle inférieur, un alcoxy inférieur-alcoyle inférieur, un alcanoyloxy inférieur-alcoyle inférieur, un arylcarbonyl-alcoyle inférieur, un alcanoyle inférieur-alcoyle inférieur, un arylcarbonyl-alcoyle inférieur, un aryl-alcoyle inférieur, un alcényle inférieur, un cycloalcoyle en $C_3$ à $C_6$-alcoyle inférieur, un alcynyle inférieur, un thiényl-alcoyle inférieur, un furyl-alcoyle inférieur, un arylcarboxamido-alcoyle inférieur, un aryl-alcényle inférieur, un alcényloxy inférieur-alcoyle inférieur, un aryloxy-alcoyle inférieur, un aryl-alcoyloxy inférieur-alcoyle inférieur ou un aryl-N-imidazolonyl-alcoyle inférieur et

X et n ont la signification donnée ci-dessus,

on substitue sur l'atome d'azote de l'isoquinoléine un composé de formule générale

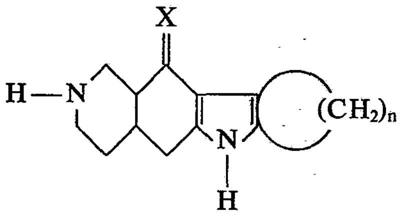

(Ab)

où X et n ont la signification donnée ci-dessus, ou

f) pour préparer un composé de formule générale

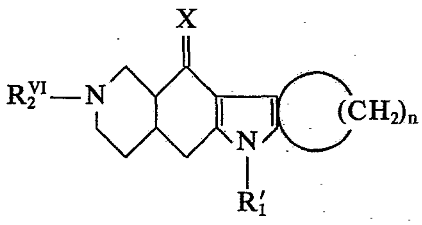

(A'c)

où

$R'_1$ représente un alcoyle inférieur, un alcanoyle inférieur, un arylcarbonyle ou un aryl-alcoyle inférieur et

$R_2^{VI}$ représente un alcoyle inférieur, un alcoxy inférieur-alcoyle inférieur, un alcanoyloxy inférieur-alcoyle inférieur, un arylcarbonyloxy-alcoyle inférieur, un alcanoyle inférieur-alcoyle inférieur, un arylcarbonyl-alcoyle inférieur, un aryl-alcoyle inférieur, un alcényle inférieur, un cyclo-alcoyle en $C_3$ à $C_6$-alcoyle inférieur, un thiényl-alcoyle inférieur, un alcynyle inférieur, un furyl-alcoyle inférieur, un arylcarboxamido-alcoyle inférieur, un aryl-alcényle inférieur, un alcényloxy inférieur-alcoyle inférieur, un aryloxy-alcoyle inférieur, un aryl-alcoyloxy inférieur-alcoyle inférieur ou un aryl-N-imidazolonyl-alcoyle inférieur et

X et n ont la signification donnée ci-dessus,

on substitue sur l'atome d'azote du pyrrole un composé de formule générale

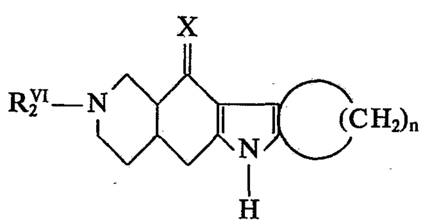

(Ad')

où $R_2^{VI}$, X et n ont la signification donnée ci-dessus, ou

g) pour préparer un composé de formule générale

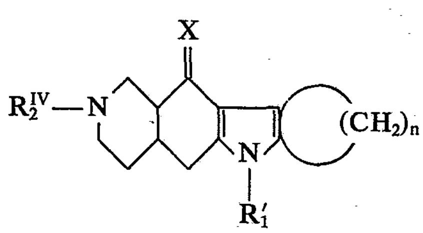

(Ac")

où

$R_2^{VI}$ représente un hydroxyalcoyle inférieur ou un arylhydroxyalcoyle inférieur et
$R_1'$, X et n ont la signification donnée ci-dessus,

on sépare le groupe protecteur dans un composé de formule générale

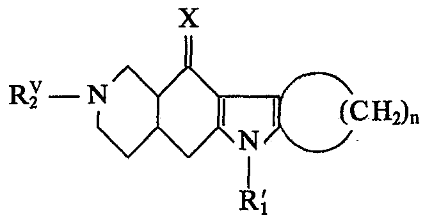

(XIV)

où

$R_2^{V}$ représente un groupe hydroxyalcoyle inférieur ou aryl-hydroxyalcoyle inférieur protégé sur le groupe hydroxy et
$R_1'$, X et n ont la signification donnée ci-dessus, ou

h) pour préparer un composé de formule générale

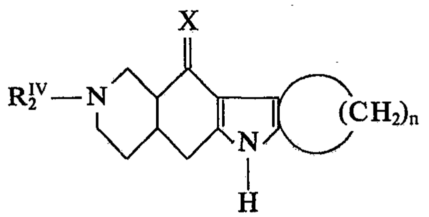

(Ad")

où $R_2^{IV}$, X et n ont la signification donnée ci-dessus,

on réduit le groupe alcanoyle inférieur-alcoyle inférieur ou arylcarbonyl-alcoyle inférieur dans un composé de formule générale

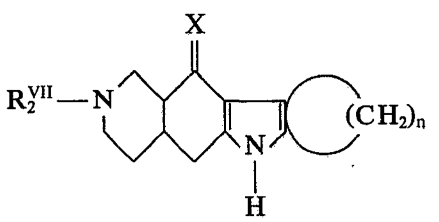

(Ad"')

où

$R_2^{VII}$ représente un alcanoyle inférieur-alcoyle inférieur ou un arylcarbonyl-alcoyle inférieur et
X et n ont la signification donnée ci-dessus, ou

i) pour préparer un composé de formule générale

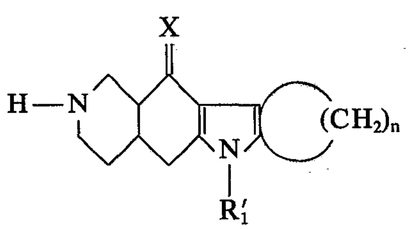

(Ah)

où X, n et $R_1'$ ont la signification donnée ci-dessus,

on sépare le groupe éthoxycarbonyle dans un composé de formule générale

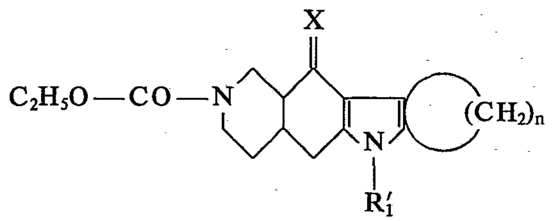

(XIII)

où X, n et $R_1'$ ont la signification donnée ci-dessus, et

j) si on le désire, on isomérise un mélange obtenu des isomères cis et trans pour donner un rapport final présentant surtout l'isomère trans, et/ou
k) si on le désire, on sépare l'isomère trans du mélange obtenu, et/ou
l) si on le désire, on dédouble un racémate obtenu en les antipodes optiques, et/ou
m) si on le désire, on transforme un composé obtenu ou un sel d'addition d'acide non pharmaceutiquement acceptable en un sel d'addition d'acide pharmaceutiquement acceptable.

21. Procédé selon la revendication 20, caractérisé en ce qu'on procède selon les variantes a), b), c), d), e), f), g), j), k), l), et m) du procédé.

22. Procédé selon la revendication 20, caractérisé en ce qu'on procède selon la variante h) du procédé.

23. Médicament contenant un composé selon l'une des revendications 1—14.

24. Agent neuroleptique/antipsychotique contenant un composé selon l'une des revendications 1—14.

**Revendications pour l'Etat contractant: AT**

1. Procédé de préparation de cycloalca[4,5]pyrrolo[2,3-g]isoquinoléines de formule générale

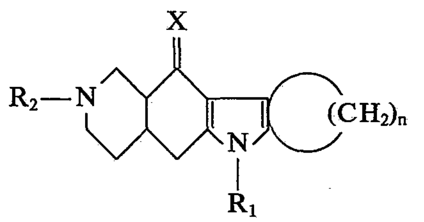

(A)

où

$R_1$ représente un hydrogène, un alcoyle inférieur, un alcanoyle inférieur, un arylcarbonyle ou un aryl-alcoyle inférieur,
$R_2$ représente un hydrogène, un alcoyle inférieur, un hydroxyalcoyle inférieur, un aryl-hydroxyalcoyle inférieur, un alcoxy inférieur-alcoyle inférieur, un alcanoyloxy inférieur-alcoyle inférieur, un arylcarbonyloxy-alcoyle inférieur, un alcanoyle inférieur-alcoyle inférieur, un arylcarbonyl-alcoyle inférieur, un aryl-alcoyle inférieur, un alcényle inférieur, un cycloalcoyle en $C_3$ à $C_6$-alcoyle inférieur, un alcynyle inférieur, un thiényl-alcoyle inférieur, un furyl-alcoyle inférieur, un arylcarboxamido-alcoyle inférieur, un aryl-alcényle inférieur, un alcényloxy inférieur-alcoyle inférieur, un aryloxy-alcoyle inférieur, un aryl-alcoyloxy inférieur-alcoyle inférieur ou un aryl-N-imidazolonyl-alcoyle inférieur,
X représente un atome d'oxygène ou de soufre et
n le nombre 3, 4, 5 ou 6, où les radicaux désignés comme inférieurs contiennent jusqu'à 7 atomes de carbone et aryle représente un phényle ou un phényle substitué par un halogène, un trifluorométhyle, un alcoyle inférieur, un alcoxy inférieur, un nitro, un amino, un alcoyle inférieur-amino ou un di(alcoyle inférieur)amino,

de leurs isomères optiques et géométriques, ainsi que de leurs sels d'addition d'acides pharmaceutiquement acceptables, caractérisé en ce que

a) pour préparer un composé de formule générale

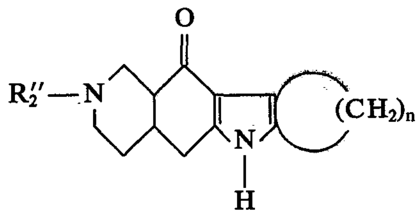

(Ia)

où

$R_2''$ représente un alcoyle inférieur, un alcoxy inférieur-alcoyle inférieur ou un cycloalcoyle en $C_3$ à $C_6$-alcoyle inférieur et

n a la signification donnée ci-dessus,

on traite un composé de formule générale

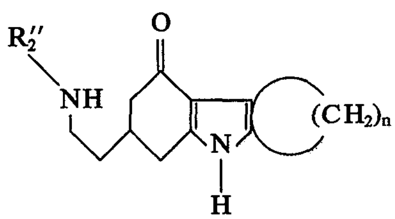

(IX)

où $R_2''$ et n ont la signification donnée ci-dessus,

avec du formaldéhyde, ou

b) pour préparer un composé de formule générale I a ci-dessus, on traite un composé de formule générale

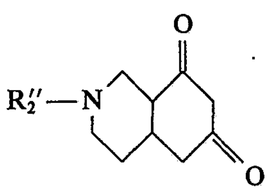

(XII)

où $R_2''$ a la signification donnée ci-dessus,

avec un composé de formule générale

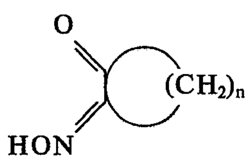

(VII)

en présence d'un réducteur ou avec un composé de formule générale

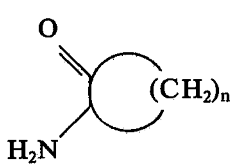

(VIII)

où n a la signification donnée ci-dessus,

ou un précurseur de celui-ci, ou

c) pour préparer un composé de formule générale

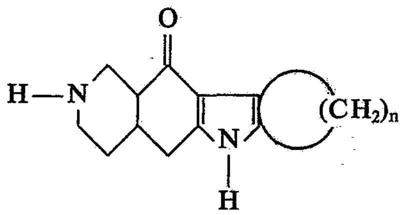

(Ib)

où n a la signification donnée ci-dessus,

on N-déméthyle un composé de formule générale Ia ci-dessus, où $R_2''$ représente un méthyle, ou

d) pour préparer un composé de formule générale

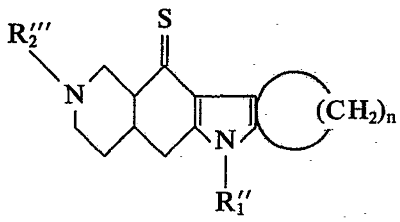

(IIe)

où

$R_1''$ représente un hydrogène, un alcoyle inférieur ou un aryl-alcoyle inférieur et
$R_2'''$ représente un hydrogène, un alcoyle inférieur, un alcoxy inférieur-alcoyle inférieur, un aryl-alcoyle inférieur, un alcényle inférieur, un cycloalcoyle en $C_3$ à $C_6$-alcoyle inférieur, un alcynyle inférieur, un thiényl-alcoyle inférieur, un furyl-alcoyle inférieur, un alcényloxy inférieur-alcoyle inférieur, un aryl-alcényle inférieur, un aryloxy-alcoyle inférieur ou un aryl-alcoyloxy-inférieur-alcoyle inférieur et
n a la signification donnée ci-dessus,

on traite un composé de formule générale

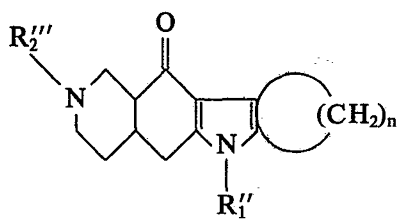

(Ie)

où $R_1''$, $R_2'''$ et n ont la signification donnée ci-dessus,

avec du pentasulfure de phosphore, ou

e) pour préparer un composé de formule générale

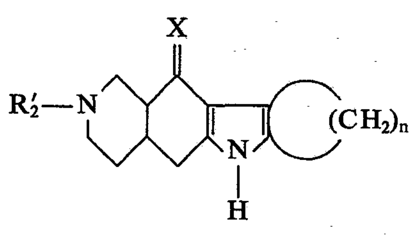

(Ad)

où

$R_2'$ représente un alcoyle inférieur, un hydroxyalcoyle inférieur, un aryl-hydroxyalcoyle inférieur, un alcoxy inférieur-alcoyle inférieur, un alcanoyloxy inférieur-alcoyle inférieur, un arylcarbonyloxy-alcoyle inférieur, un alcanoyle inférieur-alcoyle inférieur, un arylcarbonyl-alcoyle inférieur, un aryl-alcoyle inférieur, un alcényle inférieur, un cycloalcoyle en $C_3$ à $C_6$-alcoyle inférieur, un alcynyle inférieur, un thiényl-alcoyle inférieur, un furyl-alcoyle inférieur, un arylcarboxamido-alcoyle inférieur, un aryl-alcényle inférieur, un alcényloxy inférieur-alcoyle inférieur, un aryloxy-alcoyle inférieur, un aryl-alcoyloxy inférieur-alcoyle

inférieur ou un aryl-N-imidazolonyl-alcoyle inférieur et

X et n ont la signification donnée ci-dessus,

on substitue sur l'atome d'azote de l'isoquinoléine un composé de formule générale

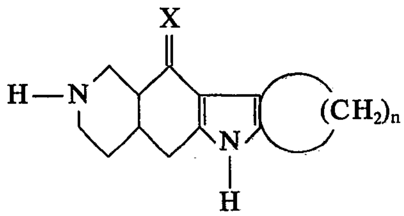

(Ab)

où X et n ont la signification donnée ci-dessus, ou

f) pour préparer un composé de formule générale

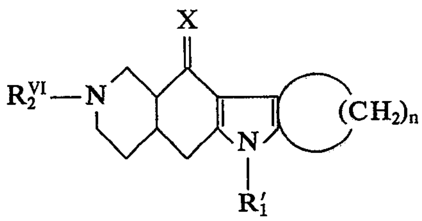

(A'c)

où

$R_1'$ représente un alcoyle inférieur, un alcanoyle inférieur, un arylcarbonyle ou un aryl-alcoyle inférieur et

$R_2^{VI}$ représente un alcoyle inférieur, un alcoxy inférieur-alcoyle inférieur, un alcanoyloxy inférieur-alcoyle inférieur, un arylcarbonyloxy-alcoyle inférieur, un alcanoyle inférieur-alcoyle inférieur, un arylcarbonyl-alcoyle inférieur, un aryl-alcoyle inférieur, un alcényle inférieur, un cycloalcoyle en $C_3$ à $C_6$-alcoyle inférieur, un thiényl-alcoyle inférieur, un alcynyle inférieur, un furyl-alcoyle inférieur, un arylcarboxamido-alcoyle inférieur, un aryl-alcényle inférieur, un alcényloxy inférieur-alcoyle inférieur, un aryloxy-alcoyle inférieur, un aryl-alcoyloxy inférieur-alcoyle inférieur ou un aryl-N-imidazolonyl-alcoyle inférieur et

X et n ont la signification donnée ci-dessus,

on substitue sur l'atome d'azote du pyrrole un composé de formule générale

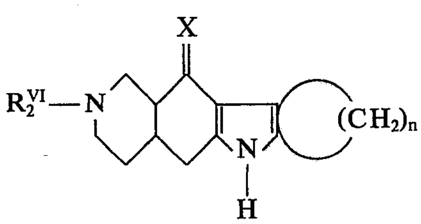

(Ad')

où $R_2^{VI}$, X et n ont la signification donnée ci-dessus, ou

g) pour préparer un composé de formule générale

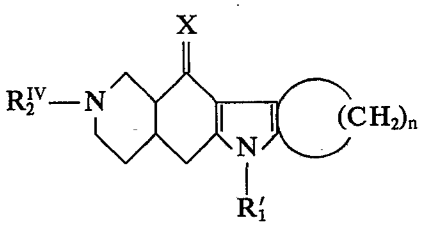

(Ac'')

où

$R_2^{VI}$ représente un hydroxyalcoyle inférieur ou un arylhydroxyalcoyle inférieur et

$R_1'$, X et n ont la signification donnée ci-dessus,

on sépare le groupe protecteur dans un composé de formule générale

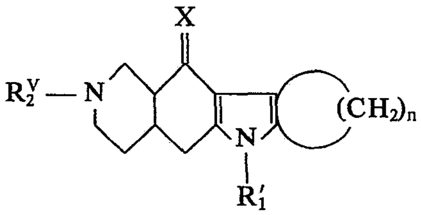

(XIV)

où

$R_2^V$ représente un groupe hydroxyalcoyle inférieur ou aryl-hydroxyalcoyle inférieur protégé sur le groupe hydroxy et
$R_1'$, X et n ont la signification donnée ci-dessus, ou

h) pour préparer un composé de formule générale

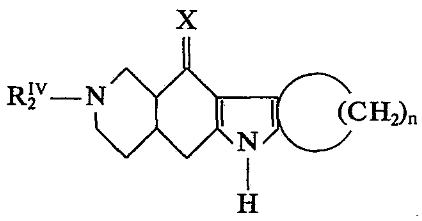

(Ad″)

où $R_2^{IV}$, X et n ont la signification donnée ci-dessus,

on réduit le groupe alcanoyle inférieur-alcoyle inférieur ou arylcarbonyl-alcoyle inférieur dans un composé de formule générale

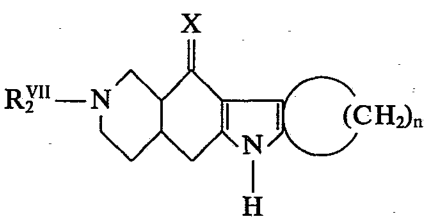

(Ad‴)

où

$R_2^{VII}$ représente un alcanoyle inférieur-alcoyle inférieur ou un arylcarbonyl-alcoyle inférieur et
X et n ont la signification donnée ci-dessus, ou

i) pour préparer un composé de formule générale

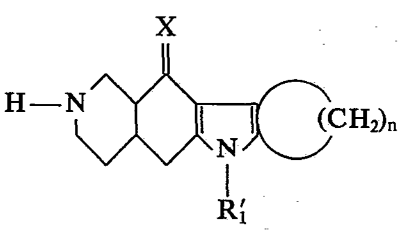

(Ah)

où X, n et $R_1'$ ont la signification donnée ci-dessus,

on sépare le groupe éthoxycarbonyle dans un composé de formule générale

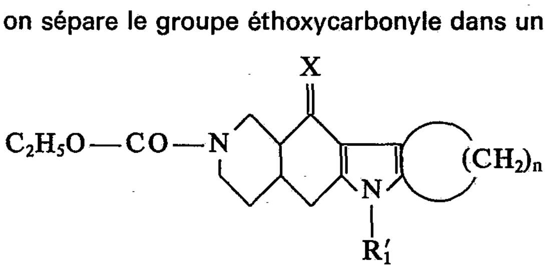

(XIII)

où X, n et $R'_1$ ont la signification donnée ci-dessus, et

j) si on le désire, on isomérise un mélange obtenu des isomères cis et trans pour donner un rapport final qui présente de façon prédominante l'isomère trans, et/ou

k) si on le désire, on sépare l'isomère trans du mélange obtenu, et/ou

l) si on le désire, on dédouble un racémate obtenu en les antipodes optiques, et/ou

m) si on le désire, on transforme un composé obtenu ou un sel d'addition d'acide non pharmaceutiquement acceptable en un sel d'addition d'acide pharmaceutiquement acceptable.

2. Procédé selon la revendication 1, caractérisé en ce qu'on procède selon les variantes a), b), c), d), e), f), g), j), k), l) et m) du procédé.

3. Procédé selon la revendication 1, caractérisé en ce qu'on procède selon la variante h) du procédé.

4. Procédé selon l'une des revendications 1 à 3, caractérisé en ce qu'on prépare un composé de formule générale A définie dans la revendication 1, où $R_1$ représente un hydrogène et $R_2$ représente un alcoyle inférieur, un hydroxyalcoyle inférieur, un aryl-hydroxyalcoyle inférieur, un alcoxy inférieur-alcoyle inférieur, un aryloxy-alcoyle inférieur, un alcanoyle inférieur-alcoyle inférieur, un arylcarbonyl-alcoyle inférieur ou un aryl-alcoyle inférieur.

5. Procédé selon la revendication 4, caractérisé en ce qu'on prépare un composé de formule générale A définie dans la revendication 1, où n représente le nombre 3 ou 4 et X représente un atome d'oxygène.

6. Procédé selon l'une des revendications 1—5, caractérisé en ce qu'on prépare un composé de formule générale A définie dans la revendication 1 qui présente la configuration trans.

7. Procédé selon la revendication 2, caractérisé en ce qu'on prépare la 2-méthyl-1,2,3,4,4a,5,7,8,9,10a-décahydro-4a,10a-trans-6H-cyclopenta[4,5]pyrrolo[2,3-g]isoquinolin-10(10H)-one.

8. Procédé selon la revendication 2, caractérisé en ce qu'on prépare le chlorhydrate de 2-méthyl-1,2,3,4,4a,5,7,8,9,10a-décahydro-4a,10a-trans-6H-cyclopenta[4,5]pyrrolo[2,3-g]isoquinolin-10(10H)-one · 0,5 $H_2O$.

9. Procédé selon la revendication 2, caractérisé en ce qu'on prépare la 2-[4-(4-fluorophényl)-4-oxobutyl]-1,2,3,4,4a,5,7,8,9,10a-décahydro-4a,10a-trans-6H-cyclopenta[4,5]pyrrolo[2,3-g]isoquinolin-10(10H)-one.

10. Procédé selon la revendication 2, caractérisé en ce qu'on prépare la 2-(2-phényléthyl)-1,2,3,4,4a,5,7,8,9,10a-décahydro-4a,10a-trans-6H-cyclopenta[4,5]pyrrolo[2,3-g]isoquinolin-10(10H)-one.

11. Procédé selon la revendication 2, caractérisé en ce qu'on prépare la (—)-2-méthyl-1,2,3,4,4a,5,7,8,9,10a-décahydro-4a,10a-trans-6H-cyclopenta[4,5]pyrrolo[2,3-g]isoquinolin-10(10H)-one.

12. Procédé selon la revendication 2, caractérisé en ce qu'on prépare la 2-méthyl-2,3,4,4a,5,7,8,9,10,11a-décahydro-4a,11a-trans-1H,6H-cyclohexa[4,5]pyrrolo[2,3-g]isoquinolin-11(11H)-one.

13. Procédé selon la revendication 2, caractérisé en ce qu'on prépare la 2-méthyl-2,3,4,4a,5,7,8,9,10,11a-décahydro-4a,11a-trans-1H,6H-cyclohexa[4,5]pyrrolo[2,3-g]isoquinolin-11-(11H)-one · 0,75 $H_2O$.

14. Procédé selon la revendication 2, caractérisé en ce qu'on prépare la 2-[4-(4-fluorophényl)-4-oxobutyl]-2,3,4,4a,5,7,8,9,10,11a-décahydro-4a,11a-trans-1H,6H-cyclohexa[4,5]pyrrolo-[2,3-g]isoquinolin-11(11H)-one.

15. Procédé selon la revendication 2, caractérisé en ce qu'on prépare la 2-(2-phényléthyl)-2,3,4,4a,5,7,8,9,10,11a-décahydro-4a,11a-trans-1H,6H-cyclohexa[4,5]pyrrolo[2,3-g]isoquinolin-11(11H)-one.

16. Procédé selon la revendication 2, caractérisé en ce qu'on prépare la 2-méthyl-1,2,3,4,4a,5,7,8,9,10a-décahydro-4a,10a-trans-6H-cyclopenta[4,5]pyrrolo[2,3-g]isoquinolin-10(10H)-thione.